# EUROPEAN PATENT APPLICATION

(11) **EP 2 845 588 A1**
(43) Date of publication of application: **11.03.2015**
(21) Application number: 13306234.9
(22) Date of filing: 09.09.2013
(51) Int. Cl.: A61K 31/122, A61K 31/166, A61K 31/343, A61K 31/381, A61K 31/403, A61K 31/435, A61K 31/4409, A61K 31/517, A61K 31/661, A61P 33/00

(54) **PIN1 inhibitors for use in the prevention and/or treatment of theileriosis, and related applications**

(71) Applicant: Centre National de la Recherche Scientifique (CNRS), 75016 Paris (FR); Université Paris Diderot - Paris 7, 75013 Paris (FR)
(72) Inventor: Marsolier, Justine, 93330 NEUILLY-SUR-MARNE (FR); Weitzman, Jonathan, 92170 VANVES (FR); Medjkane, Souhila, 75012 PARIS (FR); Perichon, Martine, 77500 CHELLES (FR)
(74) Representative: Regimbeau

(57) **Abstract**

The present invention relates to the field of lymphoproliferative Theileriosis, and more particularly to peptidyl-prolyl *cis*/*trans* isomerase 1 (PIN1) inhibitors for their use in the prevention and/or treatment of lymphoproliferative Theileriosis. The invention further encompasses screening, diagnostic and therapeutic methods, as well as to kits useful for carrying out said methods.

SEQUENCE LISTING

<110>  CENTRE NATIONAL DE LA RECHERCHE SCIENTIFIQUE (CNRS)
       UNIVERSITE PARIS DIDEROT PARIS 7

<120>  PIN1 INHIBITORS FOR USE IN THE PREVENTION AND/OR TREATMENT OF
       THEILERIOSIS, AND RELATED APPLICATIONS

<130>  70678D32471

<160>  69

<170>  PatentIn version 3.5

<210>  1
<211>  110
<212>  PRT
<213>  Theileria annulata

<220>
<221>  misc_feature
<223>  wt TaPIN amino-acid sequence (without the signal peptide)

<400>  1

Ala His Leu Leu Leu Lys His Thr Gly Ser Arg Asn Pro Val Asn Arg
1               5                   10                  15

Asn Thr Gly Met Ala Val Thr Arg Thr Lys Glu Glu Ala Val Ser Glu
            20                  25                  30

Met Lys Gly Tyr Leu Glu Met Leu Arg Lys Ser Asp Asn Leu Asp Gln
        35                  40                  45

Glu Phe Arg Arg Leu Ala Thr Ala Lys Ser Glu Cys Ser Ser Ala Arg
    50                  55                  60

Lys Gly Gly Asp Leu Gly Phe Phe Asp Arg Asn Thr Met Gln Lys Pro
65                  70                  75                  80

Phe Thr Glu Ala Ser Phe Lys Leu Glu Val Asn Glu Ile Ser Asp Leu
                85                  90                  95

Val Glu Thr Asp Ser Gly Val His Leu Ile Tyr Arg Ile Ala
            100                 105                 110

<210>  2
<211>  333
<212>  DNA
<213>  Theileria annulata

<220>
<221>  misc_feature
<223>  wt TaPIN nucleotide sequence (without the signal peptide)

<400>  2
gcccacttgc tactaaagca cactggatct aggaatccag tgaataggaa tactggaatg      60

gcagtaacaa gaacgaaaga agaggctgta agtgagatga aaggttattt ggaaatgctg     120

aggaaatctg ataatctgga ccaagagttt aggagacttg caactgccaa atctgagtgt     180

tcaagcgcaa gaaaaggggg agatttaggc tttttcgata gaaacaccat gcaaaaaccc     240

tttacggaag cctcatttaa acttaaggtt aatgagatta gtgacctggt tgagactgat     300

tctggagtac atcttatata tagaatcgca taa                                  333


<210>  3
<211>  32
<212>  PRT
<213>  Theileria annulata


<220>
<221>  misc_feature
<223>  wt TaPIN signal peptide amino-acid sequence

<400>  3

Met Ile Arg Asn Phe Leu Asn Phe Leu Trp Asn Asn Thr Ser Leu Arg
1               5                   10                  15


Phe Leu Ile Ile Asn Thr Ile Ile Phe Ala Met Asp Lys Val Arg Cys
            20                  25                  30


<210>  4
<211>  96
<212>  DNA
<213>  Theileria annulata


<220>
<221>  misc_feature
<223>  wt TaPIN signal peptide nucleotide sequence

<400>  4
atgattcgga attttctaaa ttttttgtgg aataatacta gcttaagatt tttaataatt      60

aatacaatta tatttgcaat ggacaaggtt agatgt                               96


<210>  5
<211>  32
<212>  PRT
<213>  Theileria annulata


<220>
<221>  misc_feature
<223>  T22I/A26L mutated TaPIN signal peptide amino-acid sequence

<400>  5

Met Ile Arg Asn Phe Leu Asn Phe Leu Trp Asn Asn Thr Ser Leu Arg
1               5                   10                  15

Phe Leu Ile Ile Asn Ile Ile Ile Phe Leu Met Asp Lys Val Arg Cys
            20                  25                  30

<210>  6
<211>  96
<212>  DNA
<213>  Theileria annulata

<220>
<221>  misc_feature
<223>  T22I/A26L mutated TaPIN signal peptide nucleotide sequence

<400>  6
atgattcgga attttctaaa ttttttgtgg aataatacta gcttaagatt tttaataatt      60

aatataatta tatttctaat ggacaaggtg agatgt                                96

<210>  7
<211>  142
<212>  PRT
<213>  Theileria annulata

<220>
<221>  misc_feature
<223>  wt TaPIN amino-acid sequence (with the signal peptide)

<400>  7

Met Ile Arg Asn Phe Leu Asn Phe Leu Trp Asn Asn Thr Ser Leu Arg
1               5                   10                  15

Phe Leu Ile Ile Asn Thr Ile Ile Phe Ala Met Asp Lys Val Arg Cys
            20                  25                  30

Ala His Leu Leu Leu Lys His Thr Gly Ser Arg Asn Pro Val Asn Arg
        35                  40                  45

Asn Thr Gly Met Ala Val Thr Arg Thr Lys Glu Glu Ala Val Ser Glu
    50                  55                  60

Met Lys Gly Tyr Leu Glu Met Leu Arg Lys Ser Asp Asn Leu Asp Gln
65                  70                  75                  80

Glu Phe Arg Arg Leu Ala Thr Ala Lys Ser Glu Cys Ser Ser Ala Arg
                85                  90                  95

Lys Gly Gly Asp Leu Gly Phe Phe Asp Arg Asn Thr Met Gln Lys Pro
            100                 105                 110

Phe Thr Glu Ala Ser Phe Lys Leu Glu Val Asn Glu Ile Ser Asp Leu
        115                 120                 125

Val Glu Thr Asp Ser Gly Val His Leu Ile Tyr Arg Ile Ala
    130                 135                 140

<210>  8
<211>  429
<212>  DNA
<213>  Theileria annulata

<220>
<221>  misc_feature
<223>  wt TaPIN nucleotide sequence (with the signal peptide)

<400>  8
atgattcgga attttctaaa ttttttgtgg aataatacta gcttaagatt tttaataatt      60

aatacaatta tatttgcaat ggacaaggtt agatgtgccc acttgctact aaagcacact     120

ggatctagga atccagtgaa taggaatact ggaatggcag taacaagaac gaaagaagag     180

gctgtaagtg agatgaaagg ttatttggaa atgctgagga aatctgataa tctggaccaa     240

gaatttagga gacttgcaac tgccaaatct gagtgttcaa gcgcaagaaa agggggagat     300

ttaggctttt tcgatagaaa caccatgcaa aaacccttta cggaagcctc atttaaactt     360

gaggttaatg agattagtga cctggttgaa actgactctg gagtacatct tatatataga     420

atcgcataa                                                             429

<210>  9
<211>  110
<212>  PRT
<213>  Theileria annulata

<220>
<221>  misc_feature
<223>  A53P mutated TaPIN amino-acid sequence (without the signal
       peptide)

<400>  9

Ala His Leu Leu Leu Lys His Thr Gly Ser Arg Asn Pro Val Asn Arg
1               5                   10                  15

Asn Thr Gly Met Pro Val Thr Arg Thr Lys Glu Glu Ala Val Ser Glu
            20                  25                  30

Met Lys Gly Tyr Leu Glu Met Leu Arg Lys Ser Asp Asn Leu Asp Gln
        35                  40                  45

Glu Phe Arg Arg Leu Ala Thr Ala Lys Ser Glu Cys Ser Ser Ala Arg

50                  55                  60

Lys Gly Gly Asp Leu Gly Phe Phe Asp Arg Asn Thr Met Gln Lys Pro
65                  70                  75                  80

Phe Thr Glu Ala Ser Phe Lys Leu Glu Val Asn Glu Ile Ser Asp Leu
                85                  90                  95

Val Glu Thr Asp Ser Gly Val His Leu Ile Tyr Arg Ile Ala
            100                 105                 110

<210>  10
<211>  333
<212>  DNA
<213>  Theileria annulata

<220>
<221>  misc_feature
<223>  A53P mutated TaPIN nucleotide sequence (without the signal
       peptide)

<400>  10
gcccatttgc tactaaagca cactggatct aggaatccag tgaacaggaa tactggaatg      60

ccagtaacaa gaacgaaaga agaggctgta agtgagatga aaggttattt ggaaatgctg     120

aggaaatctg ataatctgga ccaagagttt aggagacttg caactgccaa atctgagtgt     180

tcaagcgcaa gaaaaggagg agatttaggc tttttcgata gaaacaccat gcaaaaaccc     240

tttacggaag cctcatttaa acttaaggtt aatgagatta gtgacctggt tgagactgat     300

tctggagtac atcttatata tagaatcgca taa                                  333

<210>  11
<211>  142
<212>  PRT
<213>  Theileria annulata

<220>
<221>  misc_feature
<223>  A53P mutated TaPIN amino-acid sequence (with the signal peptide)

<400>  11

Met Ile Arg Asn Phe Leu Asn Phe Leu Trp Asn Asn Thr Ser Leu Arg
1               5                   10                  15

Phe Leu Ile Ile Asn Thr Ile Ile Phe Ala Met Asp Lys Val Arg Cys
            20                  25                  30

Ala His Leu Leu Leu Lys His Thr Gly Ser Arg Asn Pro Val Asn Arg
        35                  40                  45

Asn Thr Gly Met Pro Val Thr Arg Thr Lys Glu Glu Ala Val Ser Glu
    50                  55                  60

Met Lys Gly Tyr Leu Glu Met Leu Arg Lys Ser Asp Asn Leu Asp Gln
65                  70                  75                  80

Glu Phe Arg Arg Leu Ala Thr Ala Lys Ser Glu Cys Ser Ser Ala Arg
                85                  90                  95

Lys Gly Gly Asp Leu Gly Phe Phe Asp Arg Asn Thr Met Gln Lys Pro
            100                 105                 110

Phe Thr Glu Ala Ser Phe Lys Leu Glu Val Asn Glu Ile Ser Asp Leu
        115                 120                 125

Val Glu Thr Asp Ser Gly Val His Leu Ile Tyr Arg Ile Ala
    130                 135                 140

<210>  12
<211>  429
<212>  DNA
<213>  Theileria annulata

<220>
<221>  misc_feature
<223>  A53P mutated TaPIN nucleotide sequence (with the  signal peptide)

<400>  12
atgattcgga attttctaaa ttttttgtgg aataatacta gcttaagatt tttaataatt      60

aatacaatta tatttgcaat ggacaaggtg agatgtgccc atttgctact aaagcacact     120

ggatctagga atccagtgaa caggaatact ggaatgccag taacaagaac gaaagaagag     180

gctgtaagtg agatgaaagg ttatttggaa atgctgagga aatctgataa tctggaccaa     240

gagtttagga gacttgcaac tgccaaatct gagtgttcaa gcgcaagaaa aggaggagat     300

ttaggctttt tcgatagaaa caccatgcaa aaacccttta cggaagcctc atttaaactt     360

aaggttaatg agattagtga cctggttgag actgattctg gagtacatct tatatataga     420

atcgcataa                                                             429

<210>  13
<211>  142
<212>  PRT
<213>  Theileria annulata

<220>
<221>  misc_feature
<223>  T22I/A26L/A53P mutated TaPIN amino-acid sequence (with the signal
       peptide)

<400>  13

Met Ile Arg Asn Phe Leu Asn Phe Leu Trp Asn Asn Thr Ser Leu Arg
1               5                   10                  15

Phe Leu Ile Ile Asn Ile Ile Ile Phe Leu Met Asp Lys Val Arg Cys
            20                  25                  30

Ala His Leu Leu Leu Lys His Thr Gly Ser Arg Asn Pro Val Asn Arg
        35                  40                  45

Asn Thr Gly Met Pro Val Thr Arg Thr Lys Glu Glu Ala Val Ser Glu
    50                  55                  60

Met Lys Gly Tyr Leu Glu Met Leu Arg Lys Ser Asp Asn Leu Asp Gln
65                  70                  75                  80

Glu Phe Arg Arg Leu Ala Thr Ala Lys Ser Glu Cys Ser Ser Ala Arg
                85                  90                  95

Lys Gly Gly Asp Leu Gly Phe Phe Asp Arg Asn Thr Met Gln Lys Pro
            100                 105                 110

Phe Thr Glu Ala Ser Phe Lys Leu Glu Val Asn Glu Ile Ser Asp Leu
        115                 120                 125

Val Glu Thr Asp Ser Gly Val His Leu Ile Tyr Arg Ile Ala
    130                 135                 140

<210>  14
<211>  429
<212>  DNA
<213>  Theileria annulata

<220>
<221>  misc_feature
<223>  T22I/A26L/A53P mutated TaPIN nucleotide sequence (with the signal
       peptide)

<400>  14
atgattcgga attttctaaa ttttttgtgg aataatacta gcttaagatt tttaataatt      60

aatataatta tatttctaat ggacaaggtg agatgtgccc atttgctact aaagcacact     120

ggatctagga atccagtgaa caggaatact ggaatgccag taacaagaac gaaagaagag     180

gctgtaagtg agatgaaagg ttatttggaa atgctgagga aatctgataa tctggaccaa     240

gagtttagga gacttgcaac tgccaaatct gagtgttcaa gcgcaagaaa aggaggagat     300

ttaggctttt tcgatagaaa caccatgcaa aaacccttta cggaagcctc atttaaactt     360

aaggttaatg agattagtga cctggttgag actgattctg gagtacatct tatatataga 420

atcgcataa 429

<210> 15
<211> 110
<212> PRT
<213> Theileria parva

<220>
<221> misc_feature
<223> wt TpPIN amino-acid sequence (without the signal peptide)

<400> 15

Ala His Leu Leu Leu Lys His Thr Gly Ser Arg Asn Pro Val Asn Arg
1               5                   10                  15

Asn Thr Gly Met Ala Val Thr Arg Thr Lys Glu Glu Ala Val Asn Glu
            20                  25                  30

Met Lys Gly Tyr Leu Glu Met Leu Arg Lys Ser Glu Asn Leu Asp Gln
        35                  40                  45

Glu Phe Arg Arg Leu Ala Thr Ala Lys Ser Glu Cys Ser Ser Ala Arg
    50                  55                  60

Lys Gly Gly Asp Leu Gly Phe Phe Asp Arg Asn Thr Met Gln Lys Pro
65                  70                  75                  80

Phe Thr Glu Ala Ser Phe Asn Leu Lys Val Asn Glu Ile Ser Asp Leu
                85                  90                  95

Val Glu Thr Asp Ser Gly Ile His Leu Ile Tyr Arg Ile Ala
            100                 105                 110

<210> 16
<211> 333
<212> DNA
<213> Theileria parva

<220>
<221> misc_feature
<223> wt TpPIN nucleotide sequence (without the signal peptide)

<400> 16
gctcacctgt tactaaagca cactggatct aggaatccag tgaatagaaa cactggaatg 60

gccgtaacga gaacaaaaga agaagcggtg aatgaaatga aaggttattt ggaaatgctg 120

aggaaatctg aaaacctgga tcaagaattc aggagacttg caacggctaa atccgagtgt 180

tcaagtgcaa ggaaaggagg tgatttaggc ttctttgatc gaaacacaat gcaaaaacct 240

tttactgaag cttcatttaa cctcaaggtt aatgagatta gtgacctggt tgaaactgat 300

tccgggatac atcttatata cagaatcgca taa 333

<210> 17
<211> 33
<212> PRT
<213> Theileria parva

<220>
<221> misc_feature
<223> wt TpPIN signal peptide amino acid sequence

<400> 17

Met Ile Arg Cys Phe Leu Asn Phe Leu Trp Asn Lys Thr Ser Phe Arg
1               5                   10                  15

Phe Leu Ile Ile Asn Thr Ile Ile Lys Phe Thr Met Asp Lys Val Arg
            20                  25                  30

Cys

<210> 18
<211> 99
<212> DNA
<213> Theileria parva

<220>
<221> misc_feature
<223> wt TpPIN signal peptide nucleotide sequence

<400> 18
atgattcgtt gctttctaaa ttttttgtgg aataaaacta gttttagatt tctaataatt 60

aatacaataa tcaagtttac catggataag gtgcgatgt 99

<210> 19
<211> 33
<212> PRT
<213> Theileria parva

<220>
<221> misc_feature
<223> T22I/T27L mutated TpPIN signal peptide amino-acid sequence

<400> 19

Met Ile Arg Cys Phe Leu Asn Phe Leu Trp Asn Lys Thr Ser Phe Arg
1               5                   10                  15

Phe Leu Ile Ile Asn Ile Ile Ile Lys Phe Leu Met Asp Lys Val Arg

            20                  25                  30

Cys

<210> 20
<211> 99
<212> DNA
<213> Theileria parva

<220>
<221> misc_feature
<223> T22I/T27L mutated TpPIN signal peptide nucleotide sequence

<400> 20
atgattcgtt gctttctaaa ttttttgtgg aataaaacta gttttagatt tctaataatt 60

aatataataa tcaagtttct catggataag gtgcgatgt 99

<210> 21
<211> 143
<212> PRT
<213> Theileria parva

<220>
<221> misc_feature
<223> wt TpPIN amino-acid sequence (with the signal peptide)

<400> 21

Met Ile Arg Cys Phe Leu Asn Phe Leu Trp Asn Lys Thr Ser Phe Arg
1               5                   10                  15

Phe Leu Ile Ile Asn Thr Ile Ile Lys Phe Thr Met Asp Lys Val Arg
            20                  25                  30

Cys Ala His Leu Leu Leu Lys His Thr Gly Ser Arg Asn Pro Val Asn
        35                  40                  45

Arg Asn Thr Gly Met Ala Val Thr Arg Thr Lys Glu Glu Ala Val Asn
    50                  55                  60

Glu Met Lys Gly Tyr Leu Glu Met Leu Arg Lys Ser Glu Asn Leu Asp
65                  70                  75                  80

Gln Glu Phe Arg Arg Leu Ala Thr Ala Lys Ser Glu Cys Ser Ser Ala
                85                  90                  95

Arg Lys Gly Gly Asp Leu Gly Phe Phe Asp Arg Asn Thr Met Gln Lys
            100                 105                 110

Pro Phe Thr Glu Ala Ser Phe Asn Leu Lys Val Asn Glu Ile Ser Asp
        115                 120                 125

Leu Val Glu Thr Asp Ser Gly Ile His Leu Ile Tyr Arg Ile Ala
    130                 135                 140

<210>  22
<211>  432
<212>  DNA
<213>  Theileria parva

<220>
<221>  misc_feature
<223>  wt TpPIN nucleotide sequence (with the signal peptide)

<400>  22
atgattcgtt gctttctaaa ttttttgtgg aataaaacta gttttagatt tctaataatt      60

aatacaataa tcaagtttac catggataag gtgcgatgtg ctcacctgtt actaaagcac     120

actggatcta ggaatccagt gaatagaaac actggaatgg ccgtaacgag aacaaaagaa     180

gaagcggtga atgaaatgaa aggttatttg gaaatgctga ggaaatctga aaacctggat     240

caagaattca ggagacttgc aacggctaaa tccgagtgtt caagtgcaag gaaaggaggt     300

gatttaggct tctttgatcg aaacacaatg caaaaacctt ttactgaagc ttcatttaac     360

ctcaaggtta atgagattag tgacctggtt gaaactgatt ccgggataca tcttatatac     420

agaatcgcat aa                                                         432

<210>  23
<211>  110
<212>  PRT
<213>  Theileria parva

<220>
<221>  misc_feature
<223>  A54P mutated TpPIN amino-acid sequence (without the signal
       peptide)

<400>  23

Ala His Leu Leu Leu Lys His Thr Gly Ser Arg Asn Pro Val Asn Arg
1               5                   10                  15

Asn Thr Gly Met Pro Val Thr Arg Thr Lys Glu Glu Ala Val Asn Glu
            20                  25                  30

Met Lys Gly Tyr Leu Glu Met Leu Arg Lys Ser Glu Asn Leu Asp Gln
        35                  40                  45

Glu Phe Arg Arg Leu Ala Thr Ala Lys Ser Glu Cys Ser Ser Ala Arg
    50                  55                  60

Lys Gly Gly Asp Leu Gly Phe Phe Asp Arg Asn Thr Met Gln Lys Pro
65                  70                  75                  80

Phe Thr Glu Ala Ser Phe Asn Leu Lys Val Asn Glu Ile Ser Asp Leu
                85                  90                  95

Val Glu Thr Asp Ser Gly Ile His Leu Ile Tyr Arg Ile Ala
            100                 105                 110

<210>  24
<211>  333
<212>  DNA
<213>  Theileria parva

<220>
<221>  misc_feature
<223>  A54P mutated TpPIN nucleotide sequence (without the signal
       peptide)

<400>  24
gctcacctgt tactaaagca cactggatct aggaatccag tgaatagaaa cactggaatg      60

cccgtaacga gaacaaaaga agaagcggtg aatgaaatga aaggttattt ggaaatgctg     120

aggaaatctg aaaacctgga tcaagaattc aggagacttg caacggctaa atccgagtgt     180

tcaagtgcaa ggaaaggagg tgatttaggc ttctttgatc gaaacacaat gcaaaaacct     240

tttactgaag cttcatttaa cctcaaggtt aatgagatta gtgacctggt tgaaactgat     300

tccgggatac atcttatata cagaatcgca taa                                  333

<210>  25
<211>  143
<212>  PRT
<213>  Theileria parva

<220>
<221>  misc_feature
<223>  A54P mutated TpPIN amino-acid sequence (with the signal peptide)

<400>  25

Met Ile Arg Cys Phe Leu Asn Phe Leu Trp Asn Lys Thr Ser Phe Arg
1               5                   10                  15

Phe Leu Ile Ile Asn Thr Ile Ile Lys Phe Thr Met Asp Lys Val Arg
            20                  25                  30

Cys Ala His Leu Leu Leu Lys His Thr Gly Ser Arg Asn Pro Val Asn
        35                  40                  45

Arg Asn Thr Gly Met Pro Val Thr Arg Thr Lys Glu Glu Ala Val Asn
    50                  55                  60

Glu Met Lys Gly Tyr Leu Glu Met Leu Arg Lys Ser Glu Asn Leu Asp
65                  70                  75                  80

Gln Glu Phe Arg Arg Leu Ala Thr Ala Lys Ser Glu Cys Ser Ser Ala
                85                  90                  95

Arg Lys Gly Gly Asp Leu Gly Phe Phe Asp Arg Asn Thr Met Gln Lys
            100                 105                 110

Pro Phe Thr Glu Ala Ser Phe Asn Leu Lys Val Asn Glu Ile Ser Asp
        115                 120                 125

Leu Val Glu Thr Asp Ser Gly Ile His Leu Ile Tyr Arg Ile Ala
    130                 135                 140

<210>  26
<211>  432
<212>  DNA
<213>  Theileria parva

<220>
<221>  misc_feature
<223>  A54P mutated TpPIN nucleotide sequence (with the signal peptide)

<400>  26
atgattcgtt gctttctaaa ttttttgtgg aataaaacta gttttagatt tctaataatt      60

aatacaataa tcaagtttac catggataag gtgcgatgtg ctcacctgtt actaaagcac     120

actggatcta ggaatccagt gaatagaaac actggaatgc ccgtaacgag aacaaaagaa     180

gaagcggtga atgaaatgaa aggttatttg gaaatgctga ggaaatctga aaacctggat     240

caagaattca ggagacttgc aacggctaaa tccgagtgtt caagtgcaag gaaaggaggt     300

gatttaggct tctttgatcg aaacacaatg caaaaacctt ttactgaagc ttcatttaac     360

ctcaaggtta atgagattag tgacctggtt gaaactgatt ccgggataca tcttatatac     420

agaatcgcat aa                                                         432

<210>  27
<211>  143
<212>  PRT
<213>  Theileria parva

<220>
<221>  misc_feature
<223>  T22I/A27L/A54P mutated TpPIN amino-acid sequence (with the signal
       peptide)

<400>  27

Met Ile Arg Cys Phe Leu Asn Phe Leu Trp Asn Lys Thr Ser Phe Arg
1               5                   10                  15

Phe Leu Ile Ile Asn Ile Ile Ile Lys Phe Leu Met Asp Lys Val Arg
            20                  25                  30

Cys Ala His Leu Leu Leu Lys His Thr Gly Ser Arg Asn Pro Val Asn
        35                  40                  45

Arg Asn Thr Gly Met Pro Val Thr Arg Thr Lys Glu Glu Ala Val Asn
    50                  55                  60

Glu Met Lys Gly Tyr Leu Glu Met Leu Arg Lys Ser Glu Asn Leu Asp
65                  70                  75                  80

Gln Glu Phe Arg Arg Leu Ala Thr Ala Lys Ser Glu Cys Ser Ser Ala
                85                  90                  95

Arg Lys Gly Gly Asp Leu Gly Phe Phe Asp Arg Asn Thr Met Gln Lys
            100                 105                 110

Pro Phe Thr Glu Ala Ser Phe Asn Leu Lys Val Asn Glu Ile Ser Asp
        115                 120                 125

Leu Val Glu Thr Asp Ser Gly Ile His Leu Ile Tyr Arg Ile Ala
    130                 135                 140

<210>  28
<211>  432
<212>  DNA
<213>  Theileria parva

<220>
<221>  misc_feature
<223>  T22I/A27L/A54P mutated TpPIN nucleotide sequence (with the signal
       peptide)

<400>  28
atgattcgtt gctttctaaa ttttttgtgg aataaaacta gttttagatt tctaataatt       60

aatataataa tcaagtttct catggataag gtgcgatgtg ctcacctgtt actaaagcac      120

actggatcta ggaatccagt gaatagaaac actggaatgc ccgtaacgag aacaaaagaa      180

gaagcggtga atgaaatgaa aggttatttg gaaatgctga ggaaatctga aaacctggat      240

caagaattca ggagacttgc aacggctaaa tccgagtgtt caagtgcaag gaaaggaggt      300

gatttaggct tctttgatcg aaacacaatg caaaaacctt ttactgaagc ttcatttaac      360

ctcaaggtta atgagattag tgacctggtt gaaactgatt ccgggataca tcttatatac      420

agaatcgcat aa                                                          432

<210>  29
<211>  30
<212>  DNA
<213>  artificial

<220>
<223>  TaCycophilin F primer

<400>  29
ccgctcgagt tctacaatca acccaagcat                                      30

<210>  30
<211>  40
<212>  DNA
<213>  artificial

<220>
<223>  TaCycophilin R primer

<400>  30
aaggaaaaaa gcggccgctc acaataattc tccacagtcc                           40

<210>  31
<211>  27
<212>  DNA
<213>  artificial

<220>
<223>  human PIN1 F primer

<400>  31
ccgctcgagg cggacgagga gaagctg                                         27

<210>  32
<211>  38
<212>  DNA
<213>  artificial

<220>
<223>  human PIN1 R primer

<400>  32
aaggaaaaaa gcggccgctc actcagtgcg gaggatga                             38

<210>  33
<211>  27
<212>  DNA
<213>  artificial

<220>
<223>  wt TaPIN F primer

<400>  33
ccgctcgagg cccacttgct actaaag                                         27

<210>  34
<211>  40
<212>  DNA
<213>  artificial

<220>
<223>  wt TaPIN R primer

<400>  34
ataagaatgc ggccgcttat gcgattctat atataagatg                              40


<210>  35
<211>  27
<212>  DNA
<213>  artificial

<220>
<223>  TaPIN sequencing primer

<400>  35
ccgctcgagg cccacttgct actaaag                                            27


<210>  36
<211>  40
<212>  DNA
<213>  artificial

<220>
<223>  TaPIN sequencing primer

<400>  36
ataagaatgc ggccgcttat gcgattctat atataagatg                              40


<210>  37
<211>  22
<212>  DNA
<213>  artificial

<220>
<223>  TaPIN sequencing primer

<400>  37
gtctgtcaaa taggtagaaa tc                                                 22


<210>  38
<211>  22
<212>  DNA
<213>  artificial

<220>
<223>  TaPIN sequencing primer

<400>  38
gagaggaagt tgaatcaaac at                                                 22


<210>  39
<211>  33

<212>  DNA
<213>  artificial

<220>
<223>  K38ATaPIN F primer

<400>  39
gcccacttgc tactagcgca cactggatct agg                                    33


<210>  40
<211>  32
<212>  DNA
<213>  artificial

<220>
<223>  K38ATaPIN R primer

<400>  40
cctagatcca gtgtgcgtag tagcaagtgg gc                                     32


<210>  41
<211>  28
<212>  DNA
<213>  artificial

<220>
<223>  A53PTaPIN F primer

<400>  41
ggaatactgg aatgccagta acaagaac                                          28


<210>  42
<211>  28
<212>  DNA
<213>  artificial

<220>
<223>  A53PTaPIN R primer

<400>  42
gttcttgtta ctggcattcc agtattcc                                          28


<210>  43
<211>  38
<212>  DNA
<213>  artificial

<220>
<223>  43 C92ATaPIN F primer

<400>  43
gcaactgcca aatctgaggc ttcaagcgca agaaaagg                               38


<210>  44
<211>  38
<212>  DNA
<213>  artificial

<220>
<223>  43 C92ATaPIN R primer

<400>  44
ccttttcttg cgcttgaagc ctcagatttg gcagttgc                              38


<210>  45
<211>  20
<212>  DNA
<213>  artificial

<220>
<223>  HKII F primer

<400>  45
cgaccaagtg cagaaggttg                                                   20


<210>  46
<211>  19
<212>  DNA
<213>  artificial

<220>
<223>  HKII R primer

<400>  46
cgtctggagt agacctcac                                                    19


<210>  47
<211>  19
<212>  DNA
<213>  artificial

<220>
<223>  GLUT1 F primer

<400>  47
catggagccc accagcaag                                                    19


<210>  48
<211>  20
<212>  DNA
<213>  artificial

<220>
<223>  GLUT1 R primer

<400>  48
cgttgatgac tccagtgttg                                                   20


<210>  49
<211>  18
<212>  DNA
<213>  artificial

<220>
<223>  LDHA F primer

<400>  49
ggctacacat cctgggcc                                                    18


<210>  50
<211>  21
<212>  DNA
<213>  artificial

<220>
<223>  LDHA R primer

<400>  50
ggaacactaa ggaagacatc c                                                21


<210>  51
<211>  20
<212>  DNA
<213>  artificial

<220>
<223>  PDK1 F primer

<400>  51
ctaggcgtct gtgtgatttg                                                  20


<210>  52
<211>  19
<212>  DNA
<213>  artificial

<220>
<223>  PDK1 R primer

<400>  52
gatagaggtg ggatggtac                                                   19


<210>  53
<211>  20
<212>  DNA
<213>  artificial

<220>
<223>  HIF1a F primer

<400>  53
catgtgacca cgaggaaatg                                                  20


<210>  54
<211>  20
<212>  DNA
<213>  artificial

<220>
<223>  HIF1a R primer

<400>  54
tagttctccc ccggctagtt                                                  20

<210>  55
<211>  19
<212>  DNA
<213>  artificial

<220>
<223>  PKM2 F primer

<400>  55
ccattaccag cgaccccac                                                    19


<210>  56
<211>  20
<212>  DNA
<213>  artificial

<220>
<223>  PKM2 R primer

<400>  56
gtatctggcc acctgatgtg                                                   20


<210>  57
<211>  20
<212>  DNA
<213>  artificial

<220>
<223>  cJUN F primer

<400>  57
acgttttgag gcgagactgt                                                   20


<210>  58
<211>  20
<212>  DNA
<213>  artificial

<220>
<223>  cJUN R primer

<400>  58
tctgtttccc tctcgcaact                                                   20


<210>  59
<211>  24
<212>  DNA
<213>  artificial

<220>
<223>  FBW7 F primer

<400>  59
agctggagtg gaccagagaa attg                                              24


<210>  60
<211>  21

<212>  DNA
<213>  artificial

<220>
<223>  FBW7 R primer

<400>  60
gaatgagagc acgtaaagtg c                                                21


<210>  61
<211>  20
<212>  DNA
<213>  artificial

<220>
<223>  PIN1 F primer

<400>  61
ggccgggtgt actacttcaa                                                  20


<210>  62
<211>  20
<212>  DNA
<213>  artificial

<220>
<223>  PIN1 R primer

<400>  62
ttggttcggg tgatcttctc                                                  20


<210>  63
<211>  20
<212>  DNA
<213>  artificial

<220>
<223>  beta-actin F primer

<400>  63
ggcatcctga ccctcaagta                                                  20


<210>  64
<211>  20
<212>  DNA
<213>  artificial

<220>
<223>  beta-actin R primer

<400>  64
cacacggagc tcgttgtaga                                                  20


<210>  65
<211>  21
<212>  DNA
<213>  artificial

<220>
<223>  siRNA anti-PKM2

<400>  65
ctgtgtcaga aaaggacatc c                                                21


<210>  66
<211>  9
<212>  PRT
<213>  artificial

<220>
<223>  Artificial PIN1 inhibitor peptide


<220>
<221>  DISULFID
<222>  (1)..(9)

<400>  66

Cys Arg Tyr Pro Glu Val Glu Ile Cys
1               5


<210>  67
<211>  8
<212>  PRT
<213>  artificial

<220>
<223>  Artificial PIN1 inhibitor peptide


<220>
<221>  MOD_RES
<222>  (1)..(1)
<223>  ACETYLATION

<220>
<221>  MOD_RES
<222>  (2)..(2)
<223>  Lys is Lys(N-biotinoyl)

<220>
<221>  MOD_RES
<222>  (4)..(4)
<223>  Ala is Bth : beta-(3-benzothienyl)alanine

<220>
<221>  MOD_RES
<222>  (5)..(5)
<223>  Thr is D-Thr(PO3H2)

<220>
<221>  MOD_RES
<222>  (6)..(6)
<223>  Xaa is Pip : piperidine-2-carboxylic acid

<220>
<221>  MOD_RES
<222>  (7)..(7)

<223>  Ala is Nal : beta-(2-naphthyl)alanine

<220>
<221>  MOD_RES
<222>  (8)..(8)
<223>  AMIDATION

<400>  67

Lys Ala Ala Ala Thr Xaa Ala Gln
1               5


<210>  68
<211>  5
<212>  PRT
<213>  artificial

<220>
<223>  Artificial PIN1 inhibitor peptide


<220>
<221>  MOD_RES
<222>  (1)..(1)
<223>  ACETYLATION

<220>
<221>  MOD_RES
<222>  (2)..(2)
<223>  Thr is D-Thr(PO3H2)

<220>
<221>  MOD_RES
<222>  (3)..(3)
<223>  Xaa is Pip : piperidine-2-carboxylic acid

<220>
<221>  MOD_RES
<222>  (4)..(4)
<223>  Ala is Nal : beta-(2-naphthyl)alanine

<220>
<221>  MOD_RES
<222>  (5)..(5)
<223>  AMIDATION

<400>  68

Phe Thr Xaa Ala Gln
1               5


<210>  69
<211>  5
<212>  PRT
<213>  artificial

<220>
<223>  Artificial PIN1 inhibitor peptide


<220>

<221>  MOD_RES
<222>  (1)..(1)
<223>  ACETYLATION

<220>
<221>  MOD_RES
<222>  (3)..(3)
<223>  Ser is pSer

<220>
<221>  BINDING
<222>  (3)..(4)
<223>  Pseudopeptidic binding Phy[(Z)CHdC]

<220>
<221>  MOD_RES
<222>  (5)..(5)
<223>  AMIDATION

<400>  69

Phe Phe Ser Pro Arg
1               5

## Description

The present invention relates to the field of lymphoproliferative Theileriosis, and more particularly to peptidyl-prolyl *cis-trans* isomerase 1 (PIN1) inhibitors for their use in the prevention and/or treatment of lymphoproliferative Theileriosis. The invention further encompasses screening, diagnostic and therapeutic methods, as well as kits useful for carrying out said methods.

Parasites of the *Theileria* genus are transmitted by ticks and affects mainly cattle, which significantly limit the development of livestock over large regions of Europe, Africa and Asia.

The parasites *T. annulata, T. parva* and *T.hirci* belonging to the transforming *Theileria* groups are more particularly known for their high pathogenicity, and are responsible for the development of severe lymphoproliferative diseases in cattle (namely, Tropical Theileriosis, East Coast Fever and malignant Theileriosis), which are fatal in most cases. These parasites manipulate the biological signalling pathways of their host to ensure their access to nutrients, their dissemination, transmission and prevent their recognition and destruction by the host immune system. To do so, these parasites follow a particular life cycle. The *Theileria* sporozoites which are contained in the tick saliva are injected into the host three to four days after fixation of the tick to said host. Said sporozoites then rapidly invade the cytoplasm of mononuclear cells such as lymphocytes and macrophages, and develop into trophozoites and multinucleated schizonts by asexual reproduction. This process stimulates proliferation of the host cells, firstly in lymph nodes, allowing further multiplication of the parasite. The schizonts then disseminate through the lymphoid tissues before differentiating into merozoites. In parallel, a proportion of the schizonts develop into merozoites, which enter the erythrocytes and differentiate into piroplasms which are infective to ticks and capable of sexual reproduction. These *Theileria* parasites thus possess a unique capacity to transform parasitized cells of the host, in particular lymphocytes and macrophages.

The mechanism of entry of those parasites in the host cells was nevertheless not fully understood.

*T. annulata, T. parva* and *T. hirci* are the most important *Theileria* species in terms of economic costs, as they are a major cause of death in cattle and sanitary issues. Control measures against Theileriosis linked to those parasites are generally based on the use of acaricids to kill ticks, or theilericides to kill the parasite, but those methods are expensive and not efficient in drug-resistance cases. In particular, unusual cases of resistance to treatment with the theilericide Buparvaquone have been reported in recent years by field veterinarians as well as in the literature (**Mhadhbi** et al., 2010; **Sharifiyazdi** et al., 2012).

There is thus a need for identifying novel drugs and treatments that can efficiently prevent or cure lymphoproliferative Theileriosis, in particular lymphoproliferative Theileriosis resistant to Buparvaquone treatment.

In addition, current diagnosis of lymphoproliferative Theileriosis mainly relies on clinical signs (such as swollen lymph nodes, fever, a gradually increasing respiratory rate, dyspnea and diarrhea occasionally), knowledge of the disease, the examination of Giemsastained blood smears, lymph nodes and tissue biopsies. Theileriosis mediated by *T. parva, T. annulata,* and *T.hirci* are more particularly diagnosed by detection of schizonts in leukocytes, but nevertheless cannot be easily distinguished from each other with such method.

There is thus a need for providing novel diagnostic methods that can, not only rapidly detect the presence of a transforming *Theileria* parasite in a host, but also distinguish between the different subtypes of *Theileria* parasites. It is also critical to diagnose drug resistance so as to accordingly adapt the treatment.

The present invention addresses the above discussed needs in the art.

In particular, the inventors have surprisingly and unexpectedly discovered that transforming *Theileria* parasites secrete a homologue protein of the peptidyl prolyl isomerase (PIN1) into the host cell and modulate at least two signaling pathways (c-Jun/Fbw7 and metabolic signaling pathway), and that prolyl isomerisation is a conserved mechanism used by these parasites to rewire host cell metabolism and induce oncogenic-like signaling.

The inventors have also surprisingly and unexpectedly discovered that mutations of the transforming *Theileria* parasites peptidyl prolyl isomerase (TPIN) gene is responsible for resistance to Buparvaquone treatment. This discovery was particularly surprising, given that up to this day, this resistance was believed to be linked to mutations in the cytochrome b gene of these parasites (**Mhadhbi** et al., 2010; **Sharifiyazdi** et al., 2012).

The inventors have also surprisingly and unexpectedly discovered that buparvaquone affects cancer cell transformation. The present invention is thus also directed to the use of said drug for treating cancer.

### DETAILED DESCRIPTION OF THE INVENTION

Unless stated otherwise, scientific and technical terms used in connection with the present invention shall have the meanings that are commonly understood by those of ordinary skill in the art. Furthermore, unless otherwise required by context, nomenclatures used herein, and techniques of molecular biology, cell culture, and pharmacology are those well-known and commonly used in the art. Such techniques are fully explained in the literature (see Ausubel et al., Current Protocols in Molecular Biology, Eds., John Wiley & Sons, Inc. New York, 2013; Remington: The Science and Practice of Pharmacy, 22nd ed., Mack Publishing Co., Easton, Pa., 2012; and Sambrook et al., Molecular cloning: A laboratory manual 4th edition, Cold Spring Harbor Laboratory Press - Cold Spring Harbor, NY, USA, 2012).

Nevertheless, with respect to the use of different terms throughout the current specification, the following definitions more particularly apply.

In the context of the present invention, by "lymphoproliferative Theileriosis", it is meant a severe tick-borne infection of a host by a transforming *Theileria* parasite. By "transforming *Theileria* parasite", it is more particularly meant a parasitic intracellular protozoan belonging to the *Theileria* genus of the Apicomplexa phylum, which is capable of inducing uncontrollable proliferation of parasitized leukocytes of the host, followed by a lymphodestruction. Examples of transforming *Theileria* parasites according to the present invention include, without limitation, the parasites *T.annulata, T.parva, and T.hirci* (also known as *T.lestoquardi).* Pathogenesis and clinical symptoms of a lymphoproliferative Theileriosis are generally associated with the multiplication of the parasite within the host transformed cells, such as leukocytes. In its acute and subacute forms, a lymphoproliferative Theileriosis is typically characterized by high to irregular intermittent fever, enlarged lymph nodes, edema, dyspnea, anemia, leukopenia and general weakness of the host, which, if left untreated, may lead to the host death. As an illustrative example, a leukocyte count of the host inferior to 1,000 cells/mm³ is fatal in most cases.

By "tropical Theileriosis", also known as "bovine Theileriosis", "Mediterranean Coast Fever" (MCF) or "Egyptian fever", it is meant herein a lymphoproliferative Theileriosis as defined above, mediated by *T.annulata.* Hosts infected by *T.annulata* are preferably bovine, caprine and ovine, and more preferably bovine.

By "East Coast Fever" (ECF), it is meant herein a lymphoproliferative Theileriosis as defined above, mediated by *T.parva.* Hosts infected by *T.parva* are preferably bovine, caprine and ovine, and more preferably bovine.

By "malignant Theileriosis", it is meant herein a lymphoproliferative Theileriosis as defined above, mediated by *T.hirci* (i.e. *T.lestoquardi).* Hosts infected by *T.hirci* are preferably caprine and ovine.

According to the different aspects and embodiments of the invention described herein, a "subject" or "host" preferably refers to a mammal, and most preferably to a bovine, caprine and/or ovine. Said subject may have, be suspected of having, or be at risk of developing lymphoproliferative Theileriosis. A subject is said to be "at risk" of developing lymphoproliferative Theileriosis, when it is preferably surrounded by other subjects suffering from said disease.

As used herein, the terms "preventing" (or "prevention") and "treating" (or "treatment") generally mean obtaining a desired physiological or pharmacological effect depending on the degree of severity of the symptoms, condition or disease of interest, i.e. in the context of the present invention, depending on the degree of severity of the lymphoproliferative Theileriosis. The effect may be prophylactic in terms of a partial or complete prevention of the symptoms, condition or disease ("prevention"), and/or may be therapeutic in terms of a partial or complete cure of the symptoms, condition or disease ("treatment"). The term "prevention" includes the capacity to avoid, or minimize the onset or development of a disease or condition before its onset. The term "treatment" includes the inhibition of the symptoms, condition or disease (i.e. arresting the development thereof), and relief of said symptoms, condition or disease (i.e. regression leading to an improvement). In the context of the invention, the prevention of lymphoproliferative Theileriosis leads to the non-proliferation or weak proliferation of the transforming *Theileria* parasite within the host, while the treatment of lymphoproliferative Theileriosis leads to the death or almost complete death of the transforming *Theileria* parasite within the host.

As used herein, the term "theilericidal drug" or "theilericide" refers to an active agent that prevents the proliferation and/or causes the death of a *Theileria* parasite. In the context of the present invention, a theilericidal drug preferably prevents the proliferation and/or causes the death of a transforming *Theileria* parasite, and may thus be useful for the prevention and/or treatment of lymphoproliferative *Theileria.*

By "protein", "peptide" and "polypeptide" according to the invention, it is meant a sequence of amino acids joined by peptide bonds (-NHCO-) - regardless of length or post-translational modification(s) - which can be naturally occurring or synthetic, and which can play a structural and/or functional role in a cell *in vitro* and/or *in vivo.* This term further encompasses functional variants and functional fragments of a given protein. By "functional variant" or "functional fragment" of a given protein is intended a variant (such as a mutated protein) or a fragment thereof that retains the same biological function as the one of said protein. In the context of the invention, the amino-acid sequence is preferably that of an enzyme and, more preferably, that of PIN1, such as TPIN of a transforming *Theileria* parasite as described below. As well-known in the art, proteins, peptides or polypeptides can be encoded by nucleic acids.

As used herein, the terms "nucleic acid", "nucleic acid sequence", "nucleotide", "nucleotide sequence", which are interchangeable, refer to a precise succession of natural nucleotides (e.g., A, T, G, C and U), or synthetic nucleotides, corresponding to a single-stranded or double-stranded DNA such as cDNA, genomic DNA, ribosomal DNA, and the transcription product of said DNA, such as RNA. The term "nucleic acid" encompasses nucleic acids encoding a protein having a biological function of interest, as well as nucleic acids which can hybridize to a nucleic acid of reference. This term notably encompasses functional variants and functional fragments of a given nucleic acid. By "functional variant" or "functional fragment" of a given nucleic acid is intended a variant (such as a mutated nucleic acid) or a fragment thereof encoding a peptide or polypeptide that retains the same biological function as the one of the protein encoded by said given nucleic acid.

As used herein, the term "complementary" with regard to nucleic acid means that, for example, each nucleotide of a first nucleic acid sequence is paired with the complementary base of a second nucleic acid sequence whose orientation is reversed. Complementary nucleotides are A and T (or A and U) or C and G.

The proteins and/or nucleic acids of the present invention may be prepared by any known method in the art including, but not limited to, any synthetic method, any recombinant method, any *ex vivo* generation method and the like, and any combination thereof. Such techniques are fully explained in the literature as mentioned above.

By "peptidyl-prolyl *cis-trans* isomerase 1", "PIN1", "PIN1 protein", or "PIN1 enzyme" according to the invention, it is meant herein an enzyme belonging to the parvulin family, which catalyzes the *cisltrans* isomerization of phosphorylated Ser/Pro or Thr/Pro peptide bonds preceding prolyl residues (PPlase; E.C. 5.2.1.8) (**Ranganathan** et al., 1997; **Yaffe** et al., 1997; **Lu** et al., 1999). This specific enzymatic activity ("function", "biological activity" or "biological function") displayed by PIN1 is referred herein as a PPlase activity, and can be easily assessed by one skilled practitioner by well-established methods in the art. As illustrative examples, PPlase activity can be measured on induction of the CyclinD1 promoter according to the protocol described by **Wulf** et al. (2001), or via a chymotrypsin-coupled spectrophotometric assay using the Suc-Ala-Glu-Pro-Phe-pNA peptide as a substrate (**Wang** et al., 2004; **Koffron** et al., 1991). This *cisltrans* isomerization is generally mediated thanks to two specific domains of said enzyme: an N-terminal WW domain, which specifically recognizes in its substrate proteins the pSer-Pro and pThr-Pro motifs in which the first amino acid is phosphorylated (p), as well as a C-terminal catalytic domain which catalyzes the *cis*/*trans* isomerization of the peptide bonds of said motifs. More particularly, a "WW domain" refers to an amino-acid domain that consists of a three-stranded anti-parallel beta sheets and that is capable of binding proline-rich or proline-containing ligands. PIN1 enzymes are highly conserved proteins from yeast to human, and can thus be easily identified by one skilled in the art on the basis of sequence homology with corresponding PIN1 amino-acid sequence(s) of species which possess a common evolutionary origin. Examples of such PIN1 enzymes are human PIN1 (hPIN1; Uniprot/Swissprot access reference: Q13526) and bovine PIN1 (bPIN1; Uniprot/Swissprot access reference: Q5BIN5).

By "peptidyl-prolyl *cis-trans* isomerase 1 of a transforming *Theileria* parasite", *"Theileria* PIN", "TPIN", "TPIN protein" or "TPIN enzyme", it is meant an enzyme exhibiting a PPlase activity as described above, said enzyme being expressed by a transforming *Theileria* parasite. A peptidyl-prolyl *cis-trans* isomerase 1 of a transforming *Theileria* parasite typically comprises a C-Terminal catalytic domain as described above, but nevertheless does not possess a WW domain; instead, said enzyme comprises a N-terminal signal peptide which mediates its secretion within the parasitized host cells notably within the host leukocytes. Signal peptides can be easily predicted by the skilled person in the art using, for example, the Signal P3.0 program (http://ww.cbs.dtu.dk/services/SignalIP/; **Bendtsen** et al., 2004; **Emanuelsson** et al., 2007). Said signal peptide may nevertheless be cleaved by a host peptidase, once PIN1 has been secreted within the parasitized host cells.

The term "peptidyl-prolyl *cis-trans* isomerase 1 of *T.annulata",* "TaPIN", "TaPIN protein" or "TaPIN enzyme" refers to the PIN1 enzyme of sequence SEQ ID NO:7 naturally expressed by *T.annulata,* as well as to functional fragments and functional variants (such as mutants) thereof. In the context of the present invention, TaPIN is preferably selected from a peptidyl-prolyl *cis-trans* isomerase comprising at least one sequence selected from the sequences SEQ ID NO:1, SEQ ID NO:7, SEQ ID NO:9, SEQ ID NO:11, and SEQ ID NO:13. Examples of TaPIN functional variants according to the invention include, without limitation, peptidyl-prolyl *cis-trans* isomerase of sequence SEQ ID NO:9, SEQ ID NO:11 or SEQ ID NO:13. Examples of TaPIN functional fragments according to the invention include, without limitation, peptidyl-prolyl *cis-trans* isomerase of sequence SEQ ID NO:1 or SEQ ID NO:9.

TaPIN according to the present invention can contain a signal peptide sequence useful for expression of said enzyme in a host cell, such as the putative native signal peptide sequence of TaPIN of sequence SEQ ID N°3 or SEQ ID N°5 (such as TaPIN of sequence SEQ ID N°7, SEQ ID N°11, or SEQ ID N°13), and/or a protein purification tag, such as a histidine tag, a haemaglutinin (HA) tag, or a FLAG tag, in the N-Terminal extremity and/or C-Terminal extremity of said TaPIN.

TaPIN according to the present invention is preferably encoded by a nucleic acid comprising at least one sequence selected from the sequences SEQ ID N°2, SEQ ID N°8, SEQ ID NO:10, SEQ ID NO:12, and SEQ ID NO:14, and complementary sequences thereof. Examples of TaPIN nucleic acid functional variants according to the invention include, without limitation, nucleic acid of sequence SEQ ID NO:10, SEQ ID NO:12 or SEQ ID NO:14. Examples of TaPIN nucleic acid functional fragments according to the invention include, without limitation, nucleic acid of sequence SEQ ID NO:2 or SEQ ID NO:10.

A nucleic acid encoding TaPIN can contain a nucleotide sequence encoding a signal peptide sequence useful for expression of said enzyme in a host cell, such as the putative native signal peptide sequence of TaPIN of sequence SEQ ID N°4 or SEQ ID N°6 (such as TaPIN of sequence SEQ ID N°8, SEQ ID N°12, or SEQ ID N°14), or a nucleotide sequence encoding a protein purification tag, such as a histidine tag, a haemaglutinin (HA) tag, or a FLAG tag, in the 5' and/or 3' termini of the selected sequence.

The term "peptidyl-prolyl *cis-trans* isomerase 1 of *T.parva",* "TpPIN", "TpPIN protein" or "TpPIN enzyme" refers to the PIN1 enzyme naturally expressed by *T.parva* (preferably of sequence SEQ ID NO:22), as well as to functional fragments and functional variants (such as mutants) thereof. In the context of the present invention, TpPIN is preferably selected from a peptidyl-prolyl *cis-trans* isomerase comprising at least one sequence selected from the sequences SEQ ID NO:15, SEQ ID NO:21, SEQ ID NO:23, SEQ ID NO:25, and SEQ ID NO:27. Examples of putative TpPIN functional variants according to the invention include, without limitation, peptidyl-prolyl *cis-trans* isomerase of sequence SEQ ID NO:23, SEQ ID NO:25, or SEQ ID NO:27. Examples of TpPIN functional fragments according to the invention include, without limitation, peptidyl-prolyl *cis-trans* isomerase of sequence SEQ ID NO:15 or SEQ ID NO:23.

TpPIN according to the invention can contain a signal peptide sequence useful for expression of said enzyme in a host cell, such as the putative native signal peptide sequence of TpPIN of sequence SEQ ID N°17 or SEQ ID N°19 (such as TpPIN of sequence SEQ ID N°21, SEQ ID N°25, or SEQ ID N°27), and/or a protein purification tag, such as a histidine tag, a haemaglutinin (HA) tag, or a FLAG tag, in the N-Terminal extremity and/or C-Terminal extremity of said TpPIN.

TpPIN according to the present invention is preferably encoded by a nucleic acid comprising at least one sequence selected from the sequences SEQ ID N°16, SEQ ID N°22, SEQ ID NO:24, SEQ ID NO:26, and SEQ ID NO:28, and complementary sequences thereof. Examples of TpPIN nucleic acid functional variants according to the invention include, without limitation, nucleic acid of sequence SEQ ID NO:24, SEQ ID NO:26, or SEQ ID NO:28. Examples of TpPIN nucleic acid functional fragments according to the invention include, without limitation, nucleic acid of sequence SEQ ID NO:16 or SEQ ID NO:24.

A nucleic acid encoding TpPIN can contain a nucleotide sequence encoding a signal peptide sequence useful for expression of said enzyme in a host cell, such as the putative native signal peptide sequence of TaPIN of sequence SEQ ID N°18 or SEQ ID N°20 (such as TpPIN of sequence SEQ ID N°22, SEQ ID N°24, or SEQ ID N°28), or a nucleotide sequence encoding a protein purification tag, such as a histidine a histidine tag, a haemaglutinin (HA) tag, or a FLAG tag, in the 5' and/or 3' termini of the selected sequence.

The term "peptidyl-prolyl *cis-trans* isomerase 1 of *T.hirci",* "ThPIN", "ThPIN protein" or "ThPIN enzyme" refers to the PIN1 enzyme naturally expressed by *T.hirci* (i.e. *T.lestoquardi),* as well as to functional fragments and functional variants (such as mutants) thereof. As indicated above, said enzyme may be easily identified by one skilled in the art on the basis of sequence homology with corresponding PIN1 amino-acid sequence(s) which possess a common evolutionary origin, such as *T.annulata* and *T.parva.*

By "peptidyl-prolyl *cis-trans* isomerase 1 inhibitor", it is meant herein an active agent capable of inhibiting the PPlase activity of PIN1, as described above. In the context of the present invention, said inhibitor is preferably specific to PIN1, i.e., it does not inhibit the *cisltrans* isomerase activity displayed by other classes than parvulin, such as Cyclophilins or FKBPs. The inhibition of PPlase activity can be measured by methods well-known in the art, including the CyclinD1-induction assay and the chymotrypsin-coupled spectrophotometric assay described above (**Wulf** et al., 2001; **Wang** et al., 2004; **Koffron** et al., 1991).

By "Buparvaquone resistant", it is referred either to a disease, a subject suffering from said disease, or to a micro-organism mediating said disease which is completely or partially refractory to treatment with Buparvaquone (BW720c). Drug resistance in a micro-organism is frequently mediated by point mutations in key genes whose gene products interact directly or indirectly with the drug. Buparvaquone chemical structure is as follows:

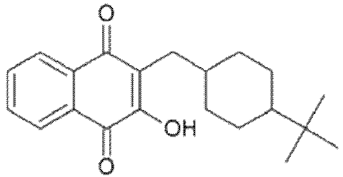

Additional definitions are provided throughout the specification.

The present invention may be understood more readily by reference to the following detailed description, including preferred embodiments of the invention, and examples included herein.

The present inventors have discovered that, upon infection of a host, transforming *Theileria* parasites secrete within the host cells a peptidyl prolyl isomerase 1 (TPIN) homologue to human PIN1 (hPIN1), thereby leading to the development in the host of lymphoproliferative Theileriosis. As illustrated in the following examples, the proliferation of said *Theileria* parasites within the host cells (and thus the uncontrolled proliferation of the parasitized cells) can be efficiently treated by using peptidyl prolyl isomerase 1 inhibitors well-known in the art, without affecting the viability of non-parasitized cells.

The present invention thus proposes to use PIN1 inhibitors as novel theilericidal drugs for preventing and/or treating lymphoproliferative Theileriosis.

So, in a first aspect, the present invention is directed to a peptidyl-prolyl *cis*/*trans* isomerase 1 (PIN1) inhibitor for use in the prevention and/or treatment of lymphoproliferative Theileriosis in a subject in need thereof, with the proviso that said inhibitor is not Buparvaquone or Parvaquone.

More precisely, the invention relates to the use of a peptidyl-prolyl *cisltrans* isomerase 1 (PIN1) inhibitor as described herein, for manufacturing a medicament to prevent and/or treat lymphoproliferative Theileriosis.

In other words, the invention relates to a method for preventing and/or treating lymphoproliferative Theileriosis comprising the step of administering an effective amount of a peptidyl-prolyl *cisltrans* isomerase 1 (PIN1) inhibitor as described herein, to a subject in need thereof.

According to a preferred embodiment, Halofuginone Lactate, and/or tetracyclines such as the oxytetracycline Medamycin or Terramycin are also excluded from the compounds used in this aspect of the present invention, should they be peptidyl-prolyl *cisltrans* isomerase 1 (PIN1) inhibitors.

According to a preferred embodiment, the peptidyl-prolyl *cisltrans* isomerase 1 (PIN1) inhibitor used in the present invention is selected from the group consisting of:
- 5-Hydroxy-1,4-naphthlenedione (Juglone);
- a PIN1 inhibitor of formula (I): 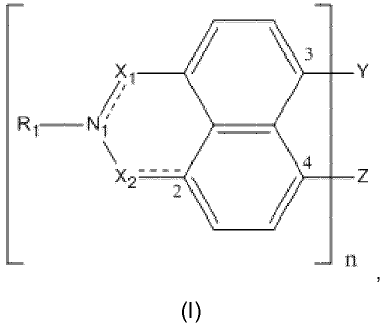 wherein
   - R1 is -CH₃, -CH₂-COOEt , or -(CH₂)₆-NH-CO-CF₃,
   - X₁ and X₂, independently from each other, is C=O and/or CH,
   - the bond between N₁ and X₁ is a single or a double bond,
   - the bond between C₂ and X₂ is a single or a double bond,
   and
   - n is 1 or 2, with the proviso that
      o when n is 2, Y and Z are chemical bonds, or
      o when n is 1, Y and Z are H, or Y and Z form a cycle according to formula (A) 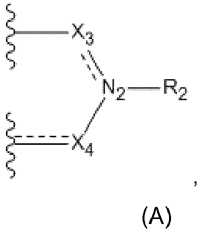
   wherein
   - X₃ is linked to C₃ of formula (I) and X₄ is linked to C₄ of formula (I),
   - R2 is -CH₃, -CH₂-COOEt , -(CH₂)₆-NH-CO-CF₃, or -(CH₂)₅-COOH, and
   - X₃ and X₄, independently from each other, is C=O and/or CH,
   - the bond between N₂ and X₃ is a single or a double bond, and
   - the bond between C₄ of formula (I) and X₄ is a single or a double bond;
- a PIN1 inhibitor of formula (II): 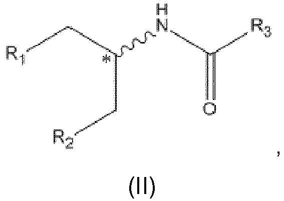 wherein
   - R1 is a mono- or poly-substituted phenyl group, the substituent(s) of R1 being selected from a C₁-C₆ alkyl group or a halogen atom, said halogen atom being preferably F,
   - R2 is a phosphate group,
   - R3 is a C₅-C₁₅ aryl or heteroaryl group comprising at least two cycles, preferably a naphthyl or a benzothiophenyl group, and
   - (*) is R or S, preferably R;
- a PIN1 inhibitor of formula (III): 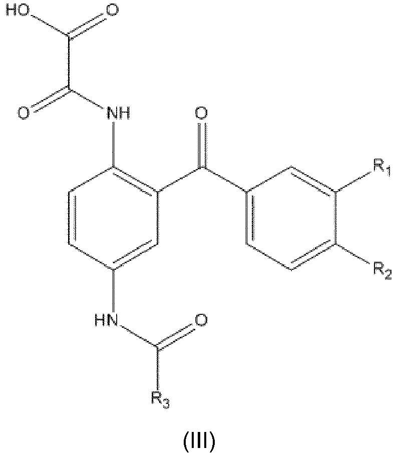 wherein
   - R1 is H, a C₁-C₆ alkyloxy group, or a halogen atom, said alkyloxy group being preferably a methoxy group, and/or said halogen atom being preferably F,
   - R2 is H, or a C₁-C₆ alkyloxy group, said alkyloxy group being preferably a methoxy group, and
   - R3 is a C₅-C₁₅ aryl or heteroaryl group comprising at least two cycles, preferably selected from an indolyl group, a benzofuranyl group, and a benzothiophenyl group optionally substituted by a halogen atom or a nitro group;
- a PIN1 inhibitor of formula (IV): 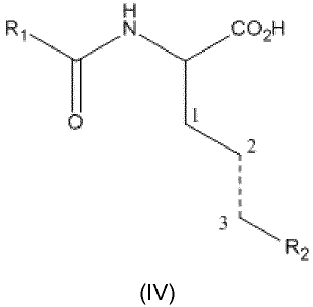 wherein
   - the bond formed between the carbon atoms 2 and 3 is single, double or triple, and when said bond is double, said bond is preferably in the E configuration,
   - R1 is a C₅-C₁₅ aryl or heteroaryl group comprising at least two cycles, preferably a naphthyl or a benzothiophenyl group, and
   - R2 is a cyclohexenyl group, a C₄-C₈ heteroaryl group, preferably a thienyl, or a C₄-C₈ aryl group, preferably a phenyl optionally substituted by a halogen atom or OH;
- 1-Piperidinecarbodithioic acid, anhydrosulfide;
- (3Z)-3-[(3Z)-3-hydroxyiminoindolin-2-ylidene]-5-nitro-indolin-2-one;
- the peptide CRYPEVEIC, wherein the cysteine residues of said peptide are cyclized by a disulfide bond (SEQ ID NO:66);
- the peptide Ac-Lys(N-biotinoyl)-Ala-Ala-Bth-D-Thr(PO₃H₂)-Pip-Nal-Gln-NH₂ (SEQ ID NO:67);
- the peptide Ac-Phe-D-Thr(PO₃H₂)-Pip-Nal-Gln-NH₂ (SEQ ID NO:68);
- the peptide Ac-Phe-Phe-pSer- Ψ [(Z)CHdC]-Pro-Arg-NH₂ (SEQ ID NO:69);
and
a pharmaceutically acceptable salt, and/or solvate thereof.

According to the invention, the expression "alkyl group" refers to a linear or branched saturated aliphatic group with 1 to 6 carbon atoms (C₁-C₆), if it is not specified. Examples of alkyl groups include, without limitation, methyl, ethyl, propyl, butyl, tert-butyl and isopropyl groups.

By "aryl or heteroaryl group", it is meant herein a mono- or polycyclic aromatic group comprising preferably between 4 and 15 carbon atoms. Heteroaryl groups typically comprise at least one heteroatom, such as nitrogen, oxygen, and sulfur - a heteroatom being any atom that is not carbon or hydrogen. Examples of aryl groups covered by the scope of the present invention include, without limitation, phenyl, etc. Examples of heteroaryl groups according to the invention include, without limitation, benzothiophenyl, naphthyl, indolyl, benzofuranyl, and thienyl.

The expression "halogen atom" in the present invention includes fluorine (F), chlorine (Cl), bromine (Br) and iodine (I) atoms.

By "phosphate group", it is meant herein a group of the (-PO₃H₂) type, whether protonated, partially or completely deprotonated, and/or partially or completely neutralized (e.g., with K+, Na+, Li+, NH4+, or the like).

By "alkyloxy group", it is meant herein an alkyl group as defined above linked to an oxygen ("O-alkyl"). Examples of alkyloxy groups covered by the scope of the invention are methoxy, ethoxy groups, etc.

As used herein, the term "pharmaceutically acceptable salt" refers to a salt that is physiologically tolerated (i.e. non-toxic) when used in an appropriate manner in the context of the present invention, particularly when used on mammals. Pharmaceutically acceptable salts include those derived from pharmaceutically acceptable inorganic and organic acids and bases. Examples of suitable acids according to the invention include hydrochloric, hydrobromic, sulfuric, nitric, perchloric, fumaric, maleic, phosphoric, glycolic, lactic, salicylic, succinic, toluene-p-sulfuric, tartaric, acetic, citric, methanesulfonic, formic, benzoic, malonic, naphthalene-2-sulfuric and benzenesulfonic acids. Other acids, such as oxalic acid, while not themselves pharmaceutically acceptable, may be employed in the preparation of salts useful as intermediates in obtaining the compounds and their pharmaceutically acceptable acid addition salts. Salts derived from appropriate bases include alkali metal (e.g., sodium), alkaline earth metal, (e.g., magnesium), ammonium and N-(C1-C4 alkyl)₄⁺ salts.

The term "solvate" according to the invention should be understood as meaning any form of the active agent in accordance with the invention (i.e. PIN1 inhibitor), in which said compound is linked through non-covalent interactions to another molecule (normally a polar solvent), including especially hydrates and alcoholates, like for example, methanolate. Methods of solvation are well-known in the art. In the context of the present invention, hydrates are particularly preferred.

Certain PIN1 inhibitors listed above may exist in stereoisomeric forms (e.g. they may contain one or more asymmetric carbon atoms or may exhibit cis-trans isomerism). In such case, the individual stereoisomers (enantiomers and diastereoisomers) and mixtures thereof are included within the scope of the present invention. Likewise, it is understood that certain PIN1 inhibitors may exist in tautomeric forms other than that shown above and these are also included within the scope of the present invention.

It shall be understood that the PIN1 inhibitors according to the invention, or salts or solvates thereof are preferably in pharmaceutically acceptable or substantially pure form. By pharmaceutically acceptable form, it is meant, *inter alia,* having a pharmaceutically acceptable level of purity, i.e. excluding normal pharmaceutical additives, such as diluents and carriers, and any material considered toxic at normal dosage levels. In the context of the present invention, purity levels for PIN1 inhibitors, salts or solvates thereof are preferably above 98%, more preferably above 99%, and even more preferably above 99.9%. In a preferred embodiment, said purity level is 99.9%.

Preferably, the PIN1 inhibitor of formula (I) of the present invention is selected from the group consisting of:
- benzo[Imn][3,8]phenanthroline-2,7-diacetic acid, 1,3,6,8-tetrahydro-1,3,6,8-tetraoxo-, diethyl ester (i.e. PiB described by Uchida et al., 2003);
- 2,7-Dimethylbenzo[Imn][3,8]phenanthroline-1,3,6,8(2H,7H)-tetrone (i.e. PiA described by Uchida et al., 2003);
- Ethyl (1,3-dioxo-1H-benzo[de]isoquinolin-2(3H)-yl)acetate (i.e. PiF described by Uchida et al., 2003) ; and
- Diethyl 2,2'-(1,3,8,10-tetraoxo-1,3,8,10-tetrahydroisoquinolino[4',5',6':6,5,10] anthrax[2,1,9-def]isoquinoline-2,9-diyl)diacetate (i.e. PiJ described by Uchida et al., 2003).

Preferably, the PIN1 inhibitor of formula (II) of the present invention is selected from the group consisting of:
- (2R)-3-(3-fluorophenyl)-2-(2-naphthoylamino-1-propyl) dihydrogen phosphate;
- (2R)-2-[(1-Benzothiophen-2-ylcarbonyl)amino]-3-(3-fluorophenyl)propyl dihydrogen phosphate;
- (2R)-3-(3-Methylphenyl)-2-(2-naphthoylamino)propyl dihydrogen phosphate;
- (2R)-2-[(1-Benzothiophen-2-ylcarbonyl)amino]-3-(3-methylphenyl)propyl dihydrogen phosphate;
- 3-(2,3-Difluorophenyl)-2-(2-naphthoylamino)propyl dihydrogen phosphate; and
- 2-[(1-Benzothiophen-2-ylcarbonyl)amino]-3-(2,3-difluorophenyl)propyl dihydrogen phosphate.

Preferably, the PIN1 inhibitor of formula (III) of the present invention is selected from the group consisting of:
- 2,3-Dihydro-1H-inden-1-yl(5'-fluoro-2',4-dihydroxy-3',5-dinitro-3-biphenylyl)methanone;
- 4-[(2-{[(3,4-Dichlorophenyl)carbamoyl]amino}-6-nitro-4-quinazolinyl)amino]benzoic acid;
- 2-[2-benzoyl-4-(1H-indole-2-carbonylamino)anilino]-2-oxo-acetic acid;
- 2-[4-(benzofuran-2-carbonylamino)-2-benzoyl-anilino]-2-oxo-acetic acid;
- 2-[4-(benzothiophene-2-carbonylamino)-2-benzoyl-anilino]-2-oxo-acetic acid;
- 2-[4-(benzothiophene-3-carbonylamino)-2-benzoyl-anilino]-2-oxo-acetic acid;
- 2-[2-benzoyl-4-[(3-chlorobenzothiophene-2-carbonyl)amino]anilino]-2-oxo-acetic acid;
- 2-[2-benzoyl-4-[(6-nitrobenzothiophene-2-carbonyl)amino]anilino]-2-oxo-acetic acid;
- 2-[4-(benzothiophene-2-carbonylamino)-2-(3,4-dimethoxybenzoyl)anilino]-2-oxo-acetic acid; 2-[4-(benzothiophene-3-carbonylamino)-2-(3,4-dimethoxybenzoyl)anilino]-2-oxo-acetic acetic acid;
- 2-[4-(benzothiophene-2-carbonylamino)-2-(3-fluoro-4-methoxy-benzoyl)anilino]-2-oxo-acetic acid; and
- 2-[4-(benzothiophene-3-carbonylamino)-2-(3-fluoro-4-methoxy-benzoyl)anilino]-2-oxo-acetic acid.

Preferably, the PIN1 inhibitor of formula (IV) of the present invention is selected from the group consisting of:
- (2R)-2-(2-Naphthoylamino)-5-phenyl-4-pentynoic acid;
- (2R)-5-(3-Fluorophenyl)-2-(2-naphthoylamino)-4-pentynoic acid;
- (2R)-5-(2-Hydroxyphenyl)-2-(2-naphthoylamino)-4-pentynoic acid;
- (2R)-2-(naphthalene-2-carbonylamino)-5-(2-thienyl)pent-4-ynoic acid;
- 5-(3-Fluorophenyl)-N-2-naphthoyl-D-norvaline;
- (2R,4E)-2-(2-Naphthoylamino)-5-phenyl-4-pentenoic acid;
- (E,2R)-5-(3-fluorophenyl)-2-(naphthalene-2-carbonylamino)pent-4-enoic acid; and
- (2R,4E)-2-[(1-Benzothiophen-2-ylcarbonyl)amino]-5-phenyl-4-pentenoic acid.

Table 1 cites, in a non-limitative way, chemical structures of the peptidyl-prolyl *cis*/*trans* isomerase 1 (PIN1) inhibitors according to the invention. Said PIN1 inhibitors can be either commercially purchased or prepared as disclosed in the provided references.

**Table 1. PIN1 inhibitors**

| **PIN1 inhibitor** | **Structure** | **ID Chemspider or CAS number** | **PIN1 inhibition (Ki and/or IC50)** | **References** |
|---|---|---|---|---|
| Juglone, or (also known as 5-Hydroxy-1,4-naphthlenedione, or 5-Hydroxy-1,4-naphthoquinone) | 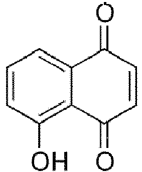 | 481-39-0 (CAS number) | - | Hennig et al., 1998 |
| (2R)-3-(3-fluorophenyl)-2-(2-naphthoylamino-1-propyl) dihydrogen phosphate | 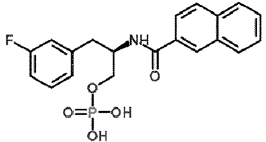 | 9615907 | Ki 0.008 µM | Guo et al., 2009 |
| | | | | Available at Abacipharm |
| (2R)-2-[(1-Benzothiophen-2-ylcarbonyl)amino]-3-(3-fluorophenyl)propyl dihydrogen phosphate | 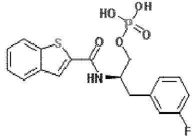 | 9593271 | Ki 0.006 µM | Guo et al., 2009 |
| | | | | Liu et al., 2012 |
| | | | | US-20050250742 |
| (2R)-3-(3-Methylphenyl)-2-(2-naphthoylamino)propyl dihydrogen phosphate | 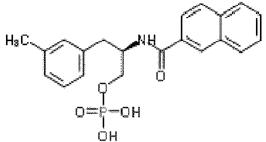 | 9924619 | Ki 0.032 µM | Guo et al., 2009 |
| (2R)-2-[(1-Benzothiophen-2-ylcarbonyl)amino]-3-(3-methylphenyl)propyl dihydrogen phosphate | 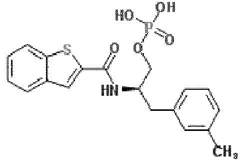 | 9924786 | Ki 0.057 µM | Guo et al., 2009 |
| 3-(2,3-Difluorophenyl)-2-(2-naphthoylamino) propyl dihydrogen phosphate | 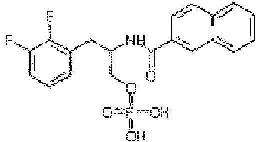 | 9364440 | Ki 0.078 µM | Guo et al., 2009 |
| | | | | US-20050250742 |
| 2-[(1-Benzothiophen-2-ylcarbonyl)amino]-3-(2,3-difluorophenyl) propyl dihydrogen phosphate | 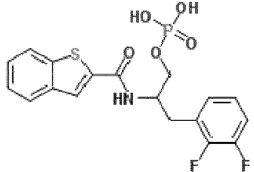 | 9491262 | Ki 0.089 µM | Guo et al., 2009 |
| | | | | WO2004087720 |
| | | | | US-20050250742 |
| 2,3-Dihydro-1H-inden-1-yl(5'-fluoro-2',4-dihydroxy-3',5-dinitro-3-biphenylyl) methanone | 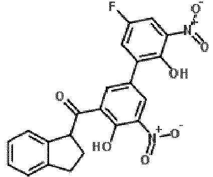 | 17251868 | - | Liu et al., 2012 |
| 4-[(2-{[(3,4-Dichlorophenyl)carbamo yl]aminol-6-nitro-4-quinazolinyl)amino]benz oic acid | 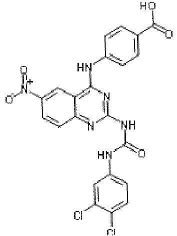 | 26344565 | - | Liu et al., 2012 |
| 2-[2-benzoyl-4-(1H-indole-2-carbonylamino) anilino]-2-oxo-acetic acid | 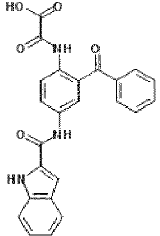 | 28499945 | - | Liu et al., 2012 |
| 2-[4-(benzofuran-2-carbonylamino)-2-benzoyl-anilino]-2-oxo-acetic acid | 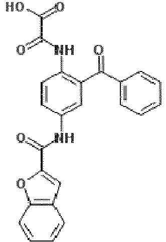 | 28517167 | - | Liu et al., 2012 |
| 2-[4-(benzothiophene-2-carbonylamino)-2-benzoyl-anilino]-2-oxo-acetic acid | 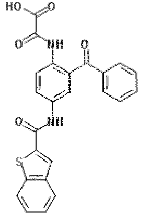 | 28524729 | IC50 10.11 µM | Liu et al., 2012 |
| 2-[4-(benzothiophene-3-carbonylamino)-2-benzoyl-anilino]-2-oxo-acetic acid | 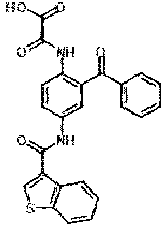 | 28524730 | IC50 11.49 µM | Liu et al., 2012 |
| 2-[2-benzoyl-4-[(3-chlorobenzothiophene-2-carbonyl) amino]anilino]-2-oxo-acetic acid | 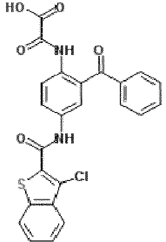 | 28524731 | IC50 8.93 µM | Liu et al., 2012 |
| 2-[2-benzoyl-4-[(6-nitrobenzothiophene-2-carbonyl)amino] anilino]-2-oxo-acetic acid | 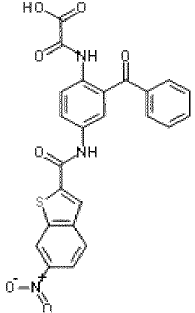 | 28529977 | IC50 5.99 µM | Liu et al., 2012 |
| 2-[4-(benzothiophene-2-carbonylamino)-2-(3,4-dimethoxybenzoyl)anilin o]-2-oxo-acetic acid | 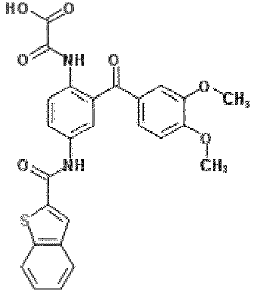 | 28531104 | IC50 54.33 µM | Liu et al., 2012 |
| 2-[4-(benzothiophene-3-carbonylamino)-2-(3,4-dimethoxybenzoyl)anilin o]-2-oxo-acetic acid | 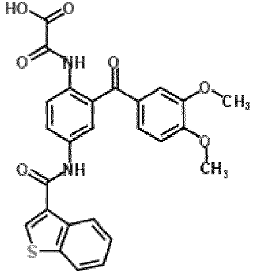 | 28531105 | IC50 53.55 µM | Liu et al., 2012 |
| 2-[4-(benzothiophene-2-carbonylamino)-2-(3-fluoro-4-methoxy-benzoyl)anilino]-2-oxo-acetic acid | 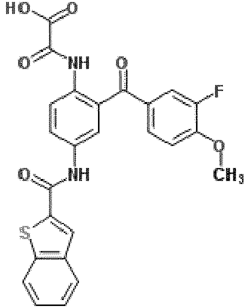 | 28531375 | IC50 10.36 µM | Liu et al., 2012 |
| 2-[4-(benzothiophene-3-carbonylamino)-2-(3-fluoro-4-methoxy-benzoyl)anilino]-2-oxo-acetic acid | 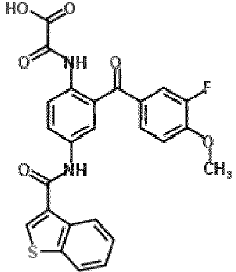 | 28531376 | IC50 14.52 µM | Liu et al., 2012 |
| (2R)-2-(2-Naphthoylamino)-5-phenyl-4-pentynoic acid | 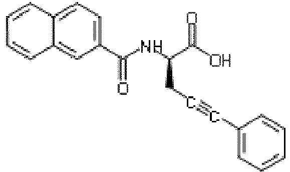 | 24670464 | Ki 12.8 µM | Dong et al., 2010 |
| (2R)-5-(3-Fluorophenyl)-2-(2-naphthoylamino)-4-pentynoic acid | 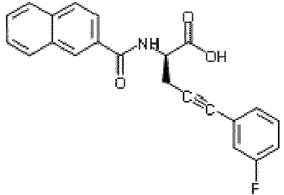 | 24670653 | Ki 1.87 µM | Dong et al., 2010 |
| (2R)-5-(2-Hydroxyphenyl)-2-(2-naphthoylamino)-4-pentynoic acid | 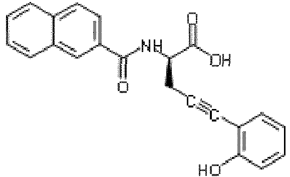 | 24677186 | Ki 4.43 µM | Dong et al., 2010 |
| (2R)-2-(naphthalene-2-carbonylamino)-5-(2-thienyl)pent-4-ynoic acid | 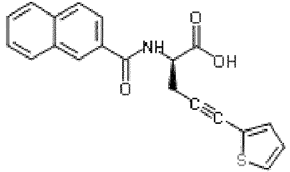 | 24675430 | Ki 7µM | Dong et al., 2010 |
| 5-(3-Fluorophenyl)-N-2-naphthoyl-D-norvaline | 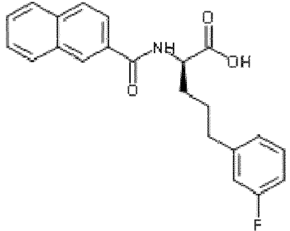 | 24667907 | Ki 18µM | Dong et al., 2010 |
| (2R,4E)-2-(2-Naphthoylamino)-5-phenyl-4-pentenoic acid | 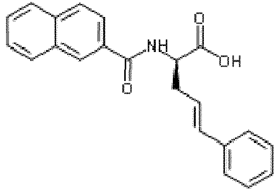 | 24670475 | Ki 1.8µM | Dong et al., 2010 |
| (E,2R)-5-(3-fluorophenyl)-2-(naphthalene-2-carbonylamino)pent-4-enoic acid | 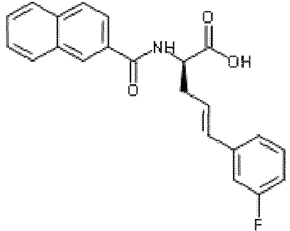 | 24671014 | Ki 0.89 µM | Dong et al., 2010 |
| (2R,4E)-2-[(1-Benzothiophen-2-ylcarbonyl)amino]-5-phenyl-4-pentenoic acid | 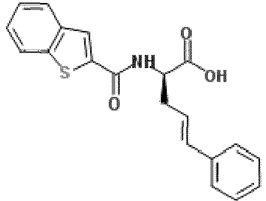 | 24670850 | Ki 3.7 µM | Dong et al., 2010 |
| 1-Piperidinecarbodithioic acid, anhydrosulfide | 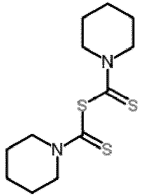 | 62961 | IC50 4.1 µM | Tatara et al., 2009 |
| PiB = benzo[Imn][3,8]phenant hroline-2,7-diacetic acid, 1,3,6,8-tetrahydro-1,3,6,8-tetraoxo-, diethyl ester | 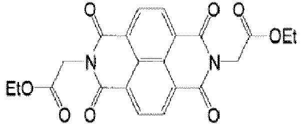 | 559058 | IC50 1.5 µM | Uchida et al., 2003 |
| | | | | WO-2010134975 |
| | | | | Available at : Calbiochem (#529627) |
| | | | | HDH Pharma (#IN1141) |
| 2,7-Dimethylbenzo[Imn][3,8] phenanthroline-1,3,6,8(2H,7H)-tetrone | 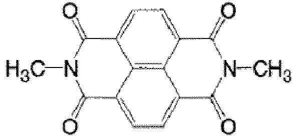 | 2043163 | IC50 2 µM | Uchida et al., 2003 |
| | | | | Available at AKos (#AKX-CD148463) |
| | | | | Molport (#003-824-690) |
| | | | | SynChem (#hs2-a019) |
| Ethyl (1,3-dioxo-1H-benzo[de]isoquinolin-2(3H)-yl)acetate | 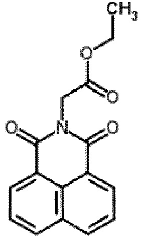 | 3535916 | IC50 5 µM | Uchida et al., 2003 |
| | | | | EP0028923 |
| | | | | Available at : AKos Innovapharm Molport Timtec Zerenex Molecular |
| Diethyl 2,2'-(1,3,8,10-tetraoxo-1,3,8,10-tetrahydroisoquinolino[4 ',5',6':6,5,10]anthra[2,1, 9-def]isoquinoline-2,9-diyl)diacetate | 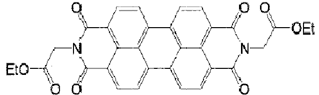 | 8319875 | IC50 1.5 µM | Uchida et al., 2003 |
| (3Z)-3-[(3Z)-3-hydroxyiminoindolin-2-ylidene]-5-nitro-indolin-2-one | 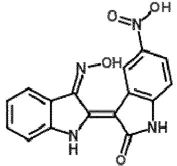 | 24679556 | 0.5 - 2.5 µM | Yoon et al., 2011 |
| cyclic peptide CRYPEVEIC | 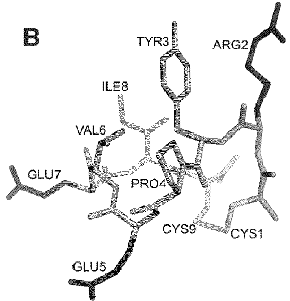 | - | Ki 0.52 µM | Duncan et al., 2011 |
| | SEQ ID NO:66 | | | |
| Ac-Lys(N-biotinoyl)-Ala-Ala-Bth-D-Thr(P03H2)-Pip-Nal-Gln-NH2 | SEQ ID NO:67 | - | Ki 1.2 nM | Wildemann et al.,2006 |
| (Bth=β-(3-benzothienyl) alanine; Pip = β-(4-biphenylyl)alanine) | | | | |
| Ac-Phe-D-Thr(P03H2)-Pip-Nal-Gln-NH2 (Pip = β-(4-biphenylyl) alanine) | SEQ ID NO:68 | - | Ki 18.3 nM | Wildemann et al.,2006 |
| Ac-Phe-Phe-pSer-Ψ[(Z)CHdC]-Pro-Arg-NH2 | SEQ ID NO:69 | - | IC50 1.3 µM | Wang et al., 2004 |
| | | | Kis 1.74 µM | |

According to a more preferred embodiment of the present invention, said peptidyl-prolyl *cisltrans* isomerase 1 (PIN1) inhibitor is selected from the group consisting of:
- 5-Hydroxy-1,4-naphthlenedione (Juglone);
- a PIN1 inhibitor of formula (I) selected from the group consisting of:
   o benzo[Imn][3,8]phenanthroline-2,7-diacetic acid, 1,3,6,8-tetrahydro-1,3,6,8-tetraoxo-, diethyl ester (PiB);
   ○ 2,7-Dimethylbenzo[Imn][3,8]phenanthroline-1,3,6,8(2H,7H)-tetrone;
   ○ Ethyl (1,3-dioxo-1H-benzo[de]isoquinolin-2(3H)-yl)acetate; and
   ○ Diethyl 2,2'-(1,3,8,10-tetraoxo-1,3,8,10-tetrahydroisoquinolino[4',5',6':6,5,10]anthra [2,1,9-def]isoquinoline-2,9-diyl)diacetate;
- a PIN1 inhibitor of formula (II) selected from the group consisting of:
   ○ (2R)-3-(3-fluorophenyl)-2-(2-naphthoylamino-1-propyl) dihydrogen phosphate;
   ○ (2R)-2-[(1-Benzothiophen-2-ylcarbonyl)amino]-3-(3-fluorophenyl)propyl dihydrogen phosphate;
   ○ (2R)-3-(3-Methylphenyl)-2-(2-naphthoylamino)propyl dihydrogen phosphate;
   ○ (2R)-2-[(1-Benzothiophen-2-ylcarbonyl)amino]-3-(3-methylphenyl)propyl dihydrogen phosphate;
   ○ 3-(2,3-Difluorophenyl)-2-(2-naphthoylamino)propyl dihydrogen phosphate; and
   ○ 2-[(1-Benzothiophen-2-ylcarbonyl)amino]-3-(2,3-difluorophenyl)propyl dihydrogen phosphate;
- a PIN1 inhibitor of formula (III) selected from the group consisting of:
   ○ 2-[2-benzoyl-4-(1H-indole-2-carbonylamino)anilino]-2-oxo-acetic acid;
   ○ 2-[4-(benzofuran-2-carbonylamino)-2-benzoyl-anilino]-2-oxo-acetic acid;
   ○ 2-[4-(benzothiophene-2-carbonylamino)-2-benzoyl-anilino]-2-oxo-acetic acid;
   ○ 2-[4-(benzothiophene-3-carbonylamino)-2-benzoyl-anilino]-2-oxo-acetic acid;
   ○ 2-[2-benzoyl-4-[(3-chlorobenzothiophene-2-carbonyl)amino]anilino]-2-oxo-acetic acid;
   ○ 2-[2-benzoyl-4-[(6-nitrobenzothiophene-2-carbonyl)amino]anilino]-2-oxo-acetic acid;
   ○ 2-[4-(benzothiophene-2-carbonylamino)-2-(3,4-dimethoxybenzoyl)anilino]-2-oxo-acetic acid;
   ○ 2-[4-(benzothiophene-3-carbonylamino)-2-(3,4-dimethoxybenzoyl)anilino]-2-oxo-acetic acid;
   ○ 2-[4-(benzothiophene-2-carbonylamino)-2-(3-fluoro-4-methoxy-benzoyl)anilino]-2-oxo-acetic acid; and
   ○ 2-[4-(benzothiophene-3-carbonylamino)-2-(3-fluoro-4-methoxy-benzoyl)anilino]-2-oxo-acetic acid;
- a PIN1 inhibitor of formula (IV) selected from the group consisting of:
   ○ (2R)-2-(2-Naphthoylamino)-5-phenyl-4-pentynoic acid;
   ○ (2R)-5-(3-Fluorophenyl)-2-(2-naphthoylamino)-4-pentynoic acid;
   ○ (2R)-5-(2-Hydroxyphenyl)-2-(2-naphthoylamino)-4-pentynoic acid;
   ○ (2R)-2-(naphthalene-2-carbonylamino)-5-(2-thienyl)pent-4-ynoic acid;
   ○ 5-(3-Fluorophenyl)-N-2-naphthoyl-D-norvaline;
   ○ (2R,4E)-2-(2-Naphthoylamino)-5-phenyl-4-pentenoic acid;
   ○ (E,2R)-5-(3-fluorophenyl)-2-(naphthalene-2-carbonylamino)pent-4-enoic acid; and
   ○ (2R,4E)-2-[(1-Benzothiophen-2-ylcarbonyl)amino]-5-phenyl-4-pentenoic acid;
- the cyclic peptide CRYPEVEIC (SEQ ID NO:66);
- the peptide Ac-Lys(N-biotinoyl)-Ala-Ala-Bth-D-Thr(PO₃H₂)-Pip-Nal-Gln-NH₂ (SEQ ID NO:67);
- the peptide Ac-Phe-D-Thr(PO₃H₂)-Pip-Nal-Gln-NH₂ (SEQ ID NO:68);
- the peptide Ac-Phe-Phe-pSer- Ψ [(Z)CHdC]-Pro-Arg-NH₂ (SEQ ID NO:69);
   and
a pharmaceutically acceptable salt, and/or solvate thereof.

Preferably, said peptidyl-prolyl cis/trans isomerase 1 (PIN1) inhibitor is selected from the group consisting of:
- 5-Hydroxy-1,4-naphthlenedione (Juglone);
- benzo[Imn][3,8]phenanthroline-2,7-diacetic acid, 1,3,6,8-tetrahydro-1,3,6,8-tetraoxo-, diethyl ester (PiB);
   and
a pharmaceutically acceptable salt, and/or solvate thereof.

Still, more preferably, said peptidyl-prolyl cis/trans isomerase 1 (PIN1) inhibitor is selected from the group consisting of:
- 5-Hydroxy-1,4-naphthlenedione (Juglone); and
- benzo[Imn][3,8]phenanthroline-2,7-diacetic acid, 1,3,6,8-tetrahydro-1,3,6,8-tetraoxo-, diethyl ester (PiB).

According to a preferred embodiment, the above PIN1 inhibitors are useful for preventing and/or treating lymphoproliferative Theileriosis mediated by a transforming *Theileria* parasite selected from the group consisting of *Theileria annulata* ("Tropical Theileriosis"), *Theileria parva* ("East Coast Fever") and *Theileria hirci* ("malignant Theileriosis"). More preferably, said lymphoproliferative Theileriosis is mediated by *Theileria annulata* or *Theileria parva.*

According to a further preferred embodiment, the above PIN1 inhibitors are useful for preventing and/or treating lymphoproliferative Theileriosis mediated by a Buparvaquone-resistant transforming *Theileria* parasite. In particular, the inventors have surprisingly discovered that resistance to this theilericidal drug is linked to at least one mutation within the C-Terminal catalytic domain of PIN1 expressed by transforming *Theileria* parasites. Preferably, said resistance is linked to a mutation in amino acid residue 53 of TaPIN, or to a mutation in amino acid residue 54 of TpPIN, and more preferably to a an Alanine to Proline mutation at the amino acid residue 53 of TaPIN, or at the amino acid residue 54 of TpPIN. Examples of Buparvaquone-resistant transforming *Theileria* parasites according to the invention include, without limitation, *Theileria annulata* parasites expressing TaPIN of sequence SEQ ID NO:11 or SEQ ID NO:13, and *Theileria parva* parasites expressing TpPIN of sequence SEQ ID NO:25 or SEQ ID NO:27.

As illustrated in the examples of the present application, Juglone is a particularly preferred PIN1 inhibitor useful for preventing and/or treating a lymphoproliferative Theileriosis mediated by a Buparvaquone-resistant transforming *Theileria* parasite.

It is particularly advantageous to combine the peptidyl-prolyl cis/trans isomerase 1 (PIN1) inhibitor according to the invention with a theilericidal drug conventionally used in the art. Such combination, association or combined preparation can notably improve in a significant manner the efficacy of a prophylactic or therapeutic treatment against lymphoproliferative Theileriosis.

It is thus another aspect of the invention to provide a combination of at least one peptidyl-prolyl *cisltrans* isomerase 1 (PIN1) inhibitor according to the invention and at least one theileceridal drug for simultaneous, separate or sequential use in the prevention and/or treatment of lymphoproliferative *Theileriosis* in a subject in need thereof.

Preferred theilericidal drugs according to the invention are active against at least one stage of *Theileria* parasites life cycle, in particular the sporozoïte or schizont stage, and may as such be administered in combination with a PIN1 inhibitor of the invention during the incubation period of lymphoproliferative Theileriosis, or at the onset of clinical signs of said disease.

In a preferred embodiment, said theilericidal drug is selected from the group consisting of Buparvaquone, Parvaquone, Halofuginone Lactate, and tetracyclines such as the oxytetracycline Medamycin or Terramycin.

It is within the skill of ordinary person in the art to select the appropriate combination of peptidyl-prolyl cis/trans isomerase 1 (PIN1) inhibitor and theilericidal drug among the above active agents, for the purposes of the invention.

In particular, the skilled person in the art will readily understand that Buparvaquone may be combined with a peptidyl-prolyl *cisltrans* isomerase 1 (PIN1) inhibitor according to the invention, if the lymphoproliferative Theileriosis to be prevented or treated is non-resistant to Buparvaquone.

Accordingly, in a preferred embodiment, said theilericidal drug is Buparvaquone. Advantageously, said peptidyl-prolyl *cisltrans* isomerase 1 (PIN1) inhibitor is selected from Juglone and PiB, and said theilericidal drug is Buparvaquone.

Alternatively, Parvaquone, Halofuginone Lactate, and tetracyclines will preferably be combined with a peptidyl-prolyl cis/trans isomerase 1 (PIN1) inhibitor according to the invention, if the lymphoproliferative Theileriosis to be prevented or treated is resistant to Buparvaquone.

Accordingly, in another preferred embodiment, said theilericidal drug is selected from the group consisting of Parvaquone, Halofuginone Lactate, and tetracyclines such as the oxytetracycline Medamycin or Terramycin. Advantageously, said peptidyl-prolyl cis/trans isomerase 1 (PIN1) inhibitor is selected from Juglone and PiB, and said theilericidal drug is selected from the group consisting of Parvaquone, Halofuginone Lactate, and tetracyclines such as the oxytetracycline Medamycin or Terramycin.

In a further aspect, the present invention relates to a pharmaceutical composition comprising at least one peptidyl-prolyl cis/trans isomerase 1 (PIN1) inhibitor of the invention, and at least one pharmaceutically acceptable excipient.

By "pharmaceutically acceptable excipient", it is meant herein a compound of pharmaceutical grade which improves the delivery, stability or bioavailability of an active agent, and can be metabolized by, and is non-toxic to, a subject to whom it is administered. Preferred excipients according to the invention include any of the excipients commonly used in pharmaceutical products, such as, for example, microcrystalline cellulose, lactose, starch, and soybean powder.

According to a preferred embodiment, said pharmaceutical composition further comprises at least one other theilericidal drug. Preferably, said theilericidal drug is selected from the group consisting of Buparvaquone, Parvaquone, Halofuginone Lactate, and tetracyclines such as Medamycin or Terramycin. Indeed, as indicated above, the combined preparation of a peptidyl-prolyl cis/trans isomerase 1 (PIN1) inhibitor according to the invention and of a theilericidal drug can significantly improve the efficacy of a prophylactic or therapeutic treatment against lymphoproliferative Theileriosis.

It is within the skill of ordinary person in the art to select the appropriate combination of peptidyl-prolyl cis/trans isomerase 1 (PIN1) inhibitor and theilericidal drug among the above active agents, for the purposes of the invention.

In particular, the skilled person in the art will readily understand that Buparvaquone may be combined with a peptidyl-prolyl cis/trans isomerase 1 (PIN1) inhibitor according to the invention, if the lymphoproliferative Theileriosis to be prevented or treated is non-resistant to Buparvaquone.

Accordingly, in a preferred embodiment, said theilericidal drug is Buparvaquone. Advantageously, said peptidyl-prolyl cis/*trans* isomerase 1 (PIN1) inhibitor is selected from Juglone and PiB, and said theilericidal drug is Buparvaquone.

Alternatively, Parvaquone, Halofuginone Lactate, and tetracyclines will preferably be combined with a peptidyl-prolyl cis/trans isomerase 1 (PIN1) inhibitor according to the invention, if the lymphoproliferative Theileriosis to be prevented or treated is resistant to Buparvaquone.

Accordingly, in another preferred embodiment, said theilericidal drug is selected from the group consisting of Parvaquone, Halofuginone Lactate, and tetracyclines such as the oxytetracycline Medamycin or Terramycin. Advantageously, said peptidyl-prolyl cis/trans isomerase 1 (PIN1) inhibitor is selected from Juglone and PiB, and said theilericidal drug is selected from the group consisting of Parvaquone, Halofuginone Lactate, and tetracyclines such as the oxytetracycline Medamycin or Terramycin.

The pharmaceutical composition of the invention may preferably be in a form suitable for the purposes of the invention. For example, said composition may be in a form suitable for parenteral, oral or intraruminal administration, such as a liquid suspension, a solid dosage form, or a paste. The term parenteral as used herein includes subcutaneous injection, intravenous, or intramuscular, injection. The compositions can also be administered transdermally by topical administration to skin and mucosal membranes.

In another aspect of the present invention, the peptidyl-prolyl *cis*/*trans* isomerase 1 (PIN1) protein of a transforming *Theileria* parasite may be used for drug screening purposes. In particular, novel drug assays may be provided, which identify therapeutics efficiently interfering with the proliferation of *Theileria* parasites within a host. The identification of novel therapeutics will notably be useful to counteract the resistance to current theilericidal treatments, which has been observed in the recent years. The present invention thus aims to provide a novel screening assay to identify theilericidal drugs that are targeted to lymphoproliferative Theileriosis.

In this aspect, the invention more particularly relates to an *in vitro* screening method for identifying a PIN1 inhibitor or combination of PIN1 inhibitors useful for preventing and/or treating lymphoproliferative Theileriosis, comprising the steps of:
a) contacting a peptidyl-prolyl *cisltrans* isomerase 1 (PIN1) protein of a transforming *Theileria* parasite, with a candidate drug or a combination of candidate drugs;
b) measuring the activity of said protein;
c) calculating a ratio R between the activity of said protein at step b) and that measured in the absence of said drug or combination of drugs;
wherein a ratio R inferior to 1 is indicative that said drug or combination of drugs is a PIN1 inhibitor or combination of PIN1 inhibitors useful for preventing and/or treating lymphoproliferative Theileriosis.

Preferably, said ratio R is inferior to 0.8, and more preferably inferior to 0.6.,

The activity of said peptidyl-prolyl *cisltrans* isomerase 1 (PIN1) protein may be measured by methods well-known in the art, such as on induction of the CyclinD1 promoter according to the protocol described by **Wulf** et al. (2001), or via a chymotrypsin-coupled spectrophotometric assay using the Suc-Ala-Glu-Pro-Phe-pNA peptide as a substrate (**Wang** et al., 2004; **Koffron** et al., 1991).

According to a preferred embodiment, said peptidyl-prolyl *cis*/*trans* isomerase 1 protein is in a purified form. Still, according to another preferred embodiment, said peptidyl-prolyl *cisltrans* isomerase 1 protein is recombinantly expressed in cultured cells, preferably in mammalian cells. Recombinant protein expression, production and purification, and cell culture techniques are well known in the art, and therefore need not to be further detailed (see Ausubel et al., Current Protocols in Molecular Biology, Eds., John Wiley & Sons, Inc. New York, 2013; and Sambrook et al., Molecular cloning: A laboratory manual 4th edition, Cold Spring Harbor Laboratory Press - Cold Spring Harbor, NY, USA, 2012).

According to a preferred embodiment, if said lymphoproliferative Theileriosis is a Tropical Theileriosis (i.e. mediated by *T.annulata),* said PIN1 protein is selected from a peptidyl-prolyl *cisltrans* isomerase comprising at least one sequence selected the group consisting of SEQ ID NO:1, SEQ ID NO:7, SEQ ID NO:9, SEQ ID NO:11, and SEQ ID NO:13. Preferably, if said Tropical Tropical Theileriosis is resistant to Buparvaquone, said PIN1 protein is selected from a peptidyl-prolyl *cis*/*trans* isomerase comprising at least one sequence selected from SEQ ID NO:9, SEQ ID NO:11 and SEQ ID NO:13.

According to another preferred embodiment, if said lymphoproliferative Theileriosis is East Coast Fever (i.e. mediated by *T.parva),* said PIN1 protein is selected from a peptidyl-prolyl *cisltrans* isomerase comprising at least one sequence selected from the group consisting of SEQ ID NO:15, SEQ ID NO:21, SEQ ID NO:23, SEQ ID NO:25, and SEQ ID NO:27. Preferably, if said Tropical East Coast Fever is resistant to Buparvaquone, said PIN1 protein is selected from a peptidyl-prolyl *cis*/*trans* isomerase comprising at least one sequence selected from SEQ ID NO:23, SEQ ID NO:25 and SEQ ID NO:27.

According to another preferred embodiment, if the lymphoproliferative Theileriosis is malignant Theileriosis (i.e. mediated by *T.hirci*)*,* said peptidyl-prolyl *cisltrans* isomerase 1 (PIN1) protein is that expressed by *T.hirci.*

Alternatively, the identification of novel PIN1 inhibitors that are targeted to lymphoproliferative Theileriosis can be performed by carrying out *in silico* screening methods.

In this aspect, the invention more particularly relates to an *in silico* screening method for identifying a candidate PIN1 inhibitor useful for preventing and/or treating lymphoproliferative Theileriosis, comprising the steps of:
a) providing a three-dimensional model of a peptidyl-prolyl *cisltrans* isomerase 1 protein of a transforming *Theileria* parasite (TPIN) as described above, and
b) docking at least one candidate drug to the surface of said structure,
wherein binding of said drug to the active site of TPIN identifies the candidate drug as a candidate PIN1 inhibitor useful for preventing and/or treating lymphoproliferative Theileriosis.

By "three-dimensional model" or "three-dimensional structure" of a given molecule, it is meant herein a structural representation of the atom's spatial relationship to other atoms of said molecule. Such structure can be determined based on atomic coordinates of a molecule of interest, (or of homologous molecules), which are preferably reported in a pdb ("protein data bank") format for macromolecules such as proteins (see http://www wwpdb.org/docs.html). In the context of the present invention, the three-dimensional structure of TPIN can preferably be determined by homology modeling based on the amino-acid sequence of said peptidyl-prolyl *cisltrans* isomerase 1 protein of a transforming *Theileria* parasite (TPIN), and on the atomic coordinates of the human PIN1 (hPIN1) (PDB file Pin1, **Ranganathan** et al.,1997). Methods for performing homology modeling and docking are well-known to the skilled person in the art, and therefore need not be further detailed herein (see e.g. **Lambert** et al., 2012; **Spitzer** et al., 2012; **Thomsen** et al., 2006 and **Sauton** et al., 2008). The binding of a drug to an active site can notably be easily determined *in silico* by one skilled in the art thanks to well-known computational methods (see e.g. **Grove** et al., 2013).

By "active site" of an enzyme, it is meant herein a site within an enzyme that binds the substrate of said enzyme and/or catalyzes a reaction to produce a product. An active site of an enzyme is typically formed by multiple amino acid residues, which may not be adjacent to each other in the primary structure (sequence) of the protein. In the context of the present invention, the active site of TPIN is the PPlase domain as described above. Said PPlase domain can be easily determined by one skilled in the art, based on homology modeling with human PIN1.

In a preferred embodiment, the above method further comprises the step of:
c) optionally, chemically synthesizing said candidate PIN1 inhibitor;
d) contacting said candidate PIN1 inhibitor with said peptidyl-prolyl *cis*/*trans* isomerase 1 (PIN1) protein of a transforming *Theileria* parasite;
e) measuring the activity of said protein; and
f) calculating a ratio R between the activity of said protein at step e) and that measured in the absence of said candidate PIN1 inhibitor;
wherein a ratio R inferior to 1 is indicative that said candidate PIN1 inhibitor is a PIN1 inhibitor useful for preventing and/or treating lymphoproliferative Theileriosis.

Preferably, said ratio R is inferior to 0.8, and more preferably inferior to 0.6.

As indicated above, current diagnosis of lymphoproliferative Theileriosis is limited. In particular, the sole examination of lymph nodes biopsies does not allow the identification of a particular *Theileria* species. The present invention thus proposes a new diagnostic method, which can not only detect the presence of a lymphoproliferative Theileriosis in a host, but also identify resistance to Buparvaquone.

So in another aspect, the present invention relates to an *in vitro* method for diagnosing a lymphoproliferative Theileriosis in a subject, comprising the step of:
a) detecting in a biological sample of said subject the peptidyl-prolyl *cis*/*trans* isomerase 1 of a transforming *Theileria* parasite (TPIN),
wherein the detection of said *Theileria* PIN in said sample is indicative that said subject is suffering from lymphoproliferative Theileriosis.

A "biological sample" according to the invention can be any sample that may be isolated from a subject, including, without limitation, a biological fluid such as blood or a fractional component thereof (serum, plasma, cellular extract), lymph, tumor interstitial fluid, saliva, mucus, urine, as well as a tissue biopsy such as a biopsy of lymph nodes. Preferred biological samples according to the invention are blood samples, fractional component thereof (serum, plasma, cellular extract), and lymph nodes biopsy.

By "detecting" according to the invention, it is meant herein qualitatively analyzing for the presence or absence of a biological marker of interest (e.g. protein or nucleic acid), i.e. in the context of the present invention, for the presence of absence of a peptidyl-prolyl cis/trans isomerase 1 of a transforming *Theileria* parasite (TPIN) as described above, such as a wild-type or a mutated TPIN.

According to a preferred embodiment, said detection may be performed by assessing for the presence or absence of a PPlase activity as described above. To do so, one skilled in the art may for example detect said activity based on the protocols described by **Wulf** et al. (2001), **Wang** et al. (2004) and **Koffron** et al. (1991).

According to another preferred embodiment, said detection may be performed by assessing for the presence or absence of a peptidyl-prolyl *cisltrans* isomerase 1 protein of a transforming *Theileria* parasite, or nucleic acid as described above.

TPIN protein may notably be detected by immunochemistry-based methods, such as Western blot, immunoblot, enzyme-linked immunosorbant assay (ELISA), enzyme-linked immunospot (ELISPOT), radioimmunoassay (RIA), immunohistochemistry and immunoprecipitation. Those methods generally involve contacting a biological sample of interest with one or more reagents that is or are capable of specifically detecting a given protein, such as an antibody. It is within the skill of ordinary person in the art to design suitable reagent(s) to detect a protein in any biological sample. For example, an antibody specifically recognizing a protein may be prepared by any conventional method known in the art by immunizing an animal, such as a mouse, hamster, rabbit or goat, with an immunogenic form of said protein which elicits an antibody response in said animal. Following immunization, antisera can be obtained, and polyclonal antibodies can be isolated from the sera. Monoclonal antibodies specific of a protein can also be produced, by harvesting for example, antibody producing cells from an immunized animal and fused with myeloma cells by standard somatic cell fusion procedures, thereby immortalizing these cells and generating so-called hybridoma cells, according to the procedure originally developed by **Köhler and Milstein** (1975). Other techniques for producing monoclonal antibodies are well known in the art and have notably been described by **Kozbor** et al. (1983), **Roder** et al. (1986) and **Huse** et al. (1986).

Alternatively, TPIN nucleic acid may be detected by any method that is suitable for generating multiple copies of said nucleic acid (i.e. amplifying). The polymerase chain reaction (PCR) is by far the most widely used method to amplify a nucleic acid. Reagents that are capable of specifically detecting a given nucleic acid are hybridizing nucleic acids. Hybridizing nucleic acids can be easily designed by one skilled in the art, based on the nucleic acid sequence encoding a protein of interest. In the context of the present invention, hybridizing nucleic acids will preferably hybridize to the nucleic acid of reference under stringent hybridization conditions. One example of stringent hybridization conditions is where attempted hybridization is carried out at a temperature ranging from about 35°C to about 65°C using a salt solution which is about 0.9 molar. It is within the skill of the person in the art to vary such conditions if need be, by taking into account variables such length of the hybridizing nucleic acid(s), base composition, type of ions present, etc. Examples of nucleic acids of the invention specifically hybridizing to TaPIN include, but are not limited to, the nucleic acids of sequence SEQ ID N°33, SEQ ID N°34, SEQ ID N°35, SEQ ID N°36, SEQ ID N°37, and SEQ ID N°38. Such nucleic acids can be used in a set to amplify the nucleic acid of interest. Examples of a nucleic acid set for amplifying TaPIN include, but is not limited to, the acid nucleic set (SEQ ID N°33, SEQ ID N°34).

It shall be understood that the above detectable reagent(s) that is or are capable of specifically detecting TPIN proteins or nucleic acids are preferably designed by one skilled in the art, so as to not only detect the presence or absence of a transforming *Theileria* parasites, but also to discriminate between different transforming *Theileria* parasites, such as *T.annulata, T.parva* and *T.hirci.*

Preferably, the detection in said sample of TaPIN protein of sequence SEQ ID NO:1, SEQ ID NO:7, SEQ ID NO:9, SEQ ID NO:11, or SEQ ID NO:13, or of a functional fragment thereof, is indicative that said subject is suffering from Tropical Theileriosis.

Still, preferably, the detection in said sample of TaPIN nucleic acid of sequence SEQ ID N°2, SEQ ID N°8, SEQ ID NO:10, SEQ ID NO:12, and SEQ ID NO:14, or of a complementary sequence or a fragment thereof, is indicative that said subject is suffering from Tropical Theileriosis.

Preferably, the detection in said sample of TpPIN protein of sequence SEQ ID NO:15, SEQ ID NO:21, SEQ ID NO:23, SEQ ID NO:25, and SEQ ID NO:27, or of a functional fragment thereof, is indicative that said subject is suffering from East Coast Fever.

Still, preferably, the detection in said sample of TpPIN nucleic acid of sequence SEQ ID N°16, SEQ ID N°22, SEQ ID NO:24, SEQ ID NO:26, and SEQ ID NO:28, or of a complementary sequence or a functional fragment thereof, is indicative that said subject is suffering from East Coast Fever.

Once the *Theileria* PIN presence has been confirmed in a biological sample of a subject, it is possible to assess the presence of a resistance by Buparvaquone.

Thus, according to a preferred embodiment, the *in vitro* diagnostic method further comprises the step of detecting from said biological sample of said subject at least one mutation associated with Buparvaquone resistance in said peptidyl-prolyl *cisltrans* isomerase 1 of a transforming *Theileria* parasite.

In the context of the present invention, by "mutation" of a protein, it is meant herein an amino-acid substitution, deletion or insertion located at one or more amino-acid positions of said protein which does not abolish the biological activity of the protein (i.e. PPlase activity with regard to TPIN), nor its secretion within the cells, if any.

Preferably, a non-conservative mutation in amino-acid residue 53 of TaPIN, or in in amino-acid residue 54 of TpPIN, is a mutation associated with Buparvaquone resistance.

In particular, the substitution of Alanin in the above amino-acid locations of TaPIN and TpPIN with a non-conservative amino-acid, such as Proline, is indicative of a Buparvaquone resistance.

Accordingly, the detection in said sample of a non-conservative mutation in amino-acid residue 53 of TaPIN (such as the substitution A53P) is indicative that said subject is suffering from Tropical Theileriosis resistant to Buparvaquone.

Alternatively, the detection in said sample of a non-conservative mutation in amino-acid residue 54 of TpPIN (such as the substitution A54P) is likely to be indicative that said subject is suffering from East Coast Fever resistant to Buparvaquone.

Conservative and non-conservative amino-acid mutations, in particular conservative and non-conservative amino-acid substitutions, are well-known to the skilled person in the art, and need not to be further detailed herein.

Amino-acid or nucleic acid mutation(s) can be easily detected by any method well-known in the art, which therefore need not to be detailed herein. For example, an amino-acid mutation can be detected, without limitation, by hybridization assays. Alternatively, a nucleic acid mutation can be detected, for example, by sequencing methods. Examples of hybridizing nucleic acids that may be used for sequencing TPIN include, without limitation, the hybridizing nucleic acids of the invention of sequence SEQ ID NO:35, SEQ ID NO:36, SEQ ID NO:37 and SEQ ID NO:38.

Once the TPIN presence has been confirmed in a biological sample of a subject, it is also possible to assess the extent of the *Theileria* parasite proliferation within the host, for example, by quantifying the expression level of *Theileria* PIN protein or nucleic acid.

According to a preferred embodiment, the above diagnostic method further comprises the step of quantifying from said biological sample of said subject the peptidyl-prolyl *cis*/*trans* isomerase 1 of a transforming *Theileria* parasite.

By "quantifying", it is meant herein determining the amount of a biological marker of interest, i.e. in the context of the present invention, determining the amount of a peptidyl-prolyl *cis*/*trans* isomerase 1 (PIN1) of a transforming *Theileria* parasite.

Said quantification may be performed by determining the expression level of peptidyl-prolyl *cis*/*trans* isomerase 1 (TPIN) protein, or nucleic acid.

*Theileria* PIN protein expression level may notably be determined by immunochemistry-based methods as described above, as well as by fluorescence activated cell sorting (FACS), surface plasmon resonance (SPR), Förster resonance energy transfer (FRET), Bioluminescence resonance energy transfer (BRET), chemiluminescence, fluorescent polarization, phosphorescence, mass spectrometry such as liquid chromatography mass spectrometry (LC-MS) or liquid chromatography/ mass spectrometry/ mass spectrometry (LC-MS-MS), matrix-assisted laser desorption/ionization time-of-flight (MALDI-TOF), surface-enhanced laser desorption/ionization time-of-flight (SELDI-TOF), and magnetic resonance imaging (MRI), and any combination thereof. All these techniques are well known in the art and need not be further detailed here.

Alternatively, *Theileria* PIN nucleic acid expression level may be determined by quantitative PCR (q-PCR) or by FISH (fluorescence in situ hybridization). Examples of quantitative PCR tools are commercially available and include, but are not limited to, Taqman® MGB probes (Applied Biosystems), SYBR® green (Applied Biosystems), Molecular beacons, and Scorpion™ probes (Sigma-Aldrich). The hybridizing nucleic acids of the invention as described above may be used in combination with said tools.

In another aspect of the present invention, the peptidyl-prolyl *cis*/*trans* isomerase 1 (PIN1) protein of a transforming *Theileria* parasite infecting a host may be used to determine if said host will respond or not to a theilericidal therapy, such as a therapy performed with a drug identified according to the screening method above. Associating a host's response to treatment with the detection and quantification of *Theileria* PIN can notably elucidate new opportunities for treatment in non-responding hosts or indicate one treatment over other treatment choices.

Therefore, the invention further provides an *in vitro* method for determining a PIN1 inhibitor-responding or non-responding phenotype in a subject suffering from lymphoproliferative Theileriosis comprising the steps of:
a) detecting in a biological sample of said subject the peptidyl-prolyl *cis*/*trans* isomerase 1 of a transforming *Theileria* parasite; and
b) determining the PIN1 inhibitor-responding or non-responding phenotype in said subject.

According to the present invention, a "PIN1 inhibitor-responding phenotype" refers to a response state of a subject to the administration of a PIN1 inhibitor. A "response state" means that said subject responds to the treatment, i.e. that the treatment is efficacious in said subject. A responding phenotype is thus characterized by an improvement in clinical symptoms such as fever, or an improvement of the infection, such as the regression or disappearance of proliferation of a transforming *Theileria* parasite within the subject.

By contrast, a "PIN1 inhibitor-non responding phenotype" refers to the absence in said subject of a state response, meaning that said subject is refractory to the treatment (i.e. resistant to said treatment).

All the above embodiments concerning the detection, and the quantification of a peptidyl-prolyl *cis*/*trans* isomerase 1 (*Theileria* PIN) apply *mutatis mutandis* to the present method.

In a further aspect of the present invention, the peptidyl-prolyl *cis*/*trans* isomerase 1 protein of a transforming *Theileria* parasite infecting a host can be used to design or adapt a treatment against lymphoproliferative Theileriosis, such as a treatment performed with a PIN1 inhibitor of the invention or with a drug identified according to the screening method above. In particular, a treatment against lymphoproliferative Theileriosis may be designed or adapted once a subject has been diagnosed as having said disease, or a resistance to Buparvaquone.

Accordingly, the present invention provides a method for designing or adapting a treatment regimen for a subject suffering from lymphoproliferative Theileriosis, comprising the steps of:
a) determining from a biological sample of said subject a PIN1 inhibitor-responding or non-responding phenotype according to the method described above; and
b) designing or adapting a treatment regimen based upon said responding or non-responding phenotype.

The term "treatment regimen" refers herein to a treatment plan that specifies the type of treatment (i.e. type of agent or combination of agents and its mode of administration), dosage, schedule and/or duration of a treatment provided to a subject in need thereof (e.g. a subject having lymphoproliferative Theileriosis). A dosage, schedule and/or duration of treatment can vary, depending on the stage of lymphoproliferative Theileriosis (e.g. incubation period or at the onset of clinical signs) and the selected type of treatment. In this regard, in addition to the PIN1 inhibitors of the invention described above and to the PIN1 inhibitors that can be identified according to the screening method of the invention, therapeutic agents that may be used in the lymphoproliferative Theileriosis treatment regimen according to the invention include, without limitation, Buparvaquone, Parvaquone, Halofuginone Lactate, and tetracyclines such as the oxytetracycline Medamycin or Terramycin and any combination thereof.

In the above method, the treatment regimen that is designed or adapted and optionally administered to the subject depends on the responding or non-responding phenotype. In particular, a treatment regimen may be selected for the first time, continued, adjusted or stopped based upon said phenotype. For example, a treatment regimen may be adjusted by increasing the dose to be administered, or stopped and switched to an alternative treatment regimen, if the subject is non-responding. Still, alternatively, a treatment regimen may be selected for the first time or continued if a subject is responding. One skilled in the art would nevertheless easily design or adjust the type of treatment with the dosage, schedule and duration of treatment, depending upon the phenotype of the subject.

For example, if a subject is non-responding to a particular PIN1 inhibitor treatment, one skilled in the art may select an alternative PIN1 inhibitor of the invention, combined if necessary with at least one alternative theilericidal drug selected from Parvaquone, Halofuginone Lactate, and tetracyclines (e.g. oxytetracycline Medamycin or Terramycin). Preferably, in such case, said PIN1 inhibitor is Juglone.

In another aspect, the invention relates to a method for treating lymphoproliferative Theileriosis in a subject in need thereof, comprising the step of administering to said subject a PIN1 inhibitor as described above.

The term "administering" as used herein means that a therapeutic agent or combination of therapeutic agents of interest, such as the ones described above, is delivered or dispensed to a subject orally, intraruminally, transdermally or parenterally such as by subcutaneous injection, intravenous injection, intramuscular injection or intraperitoneal injection.

According to a preferred embodiment, the above method further comprises a preliminary step of determining from a biological sample of said subject the PIN1 inhibitor-responding or non-responding phenotype of said subject according to the method described above.

In another aspect, the present invention provides kits that can be employed in the methods described herein. In this regard, the invention relates to a kit for use in any method described above, comprising:
a) at least one reagent capable of specifically detecting a peptidyl-prolyl *cis*/*trans* isomerase 1 of a transforming *Theileria* parasite (TPIN); and
b) instructions for using said kit in said method.

As used herein, the term "instructions" refers to a publication, a recording, a diagram, or any other medium of expression which can be used to communicate how to perform a method of the invention. Said instructions can, for example, be affixed to a container which contains said kit. Preferably, the instructions for using said kit include at least one reference expression level, as defined above.

The term "reagent capable of specifically detecting" a biological marker of interest designates a reagent or a set of reagents which specifically recognizes a given biomarker and allows for the detection, and preferably the quantification, of said marker. These reagents can be, for example, antibodies or hybridizing nucleic acid which specifically recognize a biological marker of interest. In the context of the present invention, such reagent is said to be "specific" for its target (i.e. biological marker) or "recognizes specifically" its target if it 1) exhibits a threshold level of binding activity, and/or 2) does not significantly cross-react with target molecules known to be related to said target. The binding affinity of such reagent can be easily determined by one skilled in the art, for example, by Scatchard analysis. Cross-reactivity of a reagent can as well be easily determined by one skilled in the art, and thus need not to be further detailed herein. Examples of reagents capable of specifically detecting PIN1, such as antibodies or hybridizing nucleic acids, are as described above.

Preferably, said reagent capable of specifically detecting TPIN is selected from the group consisting of the hybridizing nucleic acids of sequence SEQ ID NO:33, SEQ ID NO:34, SEQ ID NO:35, SEQ ID NO:36, SEQ ID NO:37 and SEQ ID NO:38, and complementary sequences thereof, more preferably of the hybridizing nucleic acids of sequence SEQ ID N°33 and SEQ ID N°34.

In a preferred embodiment, said reagent is capable of specifically detecting TaPIN.

In another preferred embodiment, said reagent is capable of specifically detecting TpPIN.

Still, in another preferred embodiment, said reagent is capable of specifically detecting ThPIN.

Yet, in another preferred embodiment, said kit comprises:
a) at least one reagent capable of specifically detecting TaPIN;
b) at least one reagent capable of specifically detecting TpPIN;
c) at least one reagent capable of specifically detecting ThPIN; and
d) instructions for using said kit in said method.

In another aspect, the present invention relates to buparvaquone, for use in the treatment of cancer.

Similarly, the present invention also relates to the use of buparvaquone for manufacturing a drug intended for the treatment of cancer.

The present invention further relates to a method for treating cancer in a subject in need thereof, comprising administering an efficient amount of buparvaquone to said subject.

PIN1 is known to be overexpressed in numerous cancers, at least in some patients. In particular, overexpression in human breast cancer cell lines and cancer tissues and its critical role in the transformation of mammary epithelial cells by activating multiple oncogenic pathways has been well established, PIN1 inhibition being able to suppress transformed phenotypes and inhibit tumor cell growth (**Ryo** et al., 2002). Pin1 expression is also known as an excellent independent prognostic marker in prostate cancer (**Ayala** et al., 2003), and RNAi to PIN1 has been shown to suppress tumorigenicity in prostate cancer (**Ryo** et al., 2005). Bao and colleagues tested the expression of PIN1 in normal and tumor human tissues and cell lines, and found that while Pin1 is usually expressed at very low levels in most normal tissues, it is strikingly overexpressed in many different human cancers, including prostate, lung, ovary, cervical, brain tumors, and melanoma (**Bao** et al., 2004). PIN1 inhibitors have thus been proposed as new anticancer agents (**Ryo** et al., 2003). PIN1 has further been associated to increased VEGF-mediated angiogenesis (**Kim** et al., 2009).

In specific embodiments, the cancer treated using buparvaquone is selected from the group consisting of: breast cancer, skin cancer, bone cancer, prostate cancer, liver cancer, lung cancer, brain cancer, cancer of the larynx, gallbladder, esophagus, pancreas, rectum, parathyroid, thyroid, adrenal, neural tissue, head and neck, colon, stomach, bronchi, kidney. More particularly, buparvaquone may be used in the treatment of liver cancer (in particular hepatocellular carcinoma (HCC)), breast cancer, prostate cancer.

In other embodiments, the cancer treated using buparvaquone is selected from liquid cancers, i.e. cancers of blood cells, including T or B lymphocytes, NK cells, macrophages, dendritic cells, neutrophils, eosinophils, granulocytes, mastocytes. Such liquid cancers thus include T cell, B cell or myeloid leukemia (including acute myeloid leukemia and adult T cell leukemia), T cell or B cell lymphoma, and myeloma. In particular, PIN1 inhibition has been shown to increase the response of acute myeloid leukemia (AML) cells to retinoic acid (**Gianni** et al., 2009). PIN1 overexpression has also been associated to transformation leading to adult T-cell leukemia (ATL) (**Peloponese** et al., 2009).

In a specific embodiment, the subject in need thereof has been shown to overexpress PIN1 (e.g., PIN1 protein or nucleic acid), or to present an elevation in the phosphorylation level of c-Jun or an elevation in the expression of cyclin D1, since PIN1 has been shown to induce an increase in the phosphorylation level of c-Jun and in the expression of cyclin D1 (**Ryo** et al., 2003). Such aberrant levels of PIN1, elevation in the phosphorylation level of c-Jun or elevation in the expression of cyclin D1 have preferably been determined in a tumor sample. By "overexpress", it is meant that the expression level of PIN1 in said subject is significantly higher than the expression level of PIN1 is normal tissue. The comparison with normal tissue may be made based on a control sample, which may come from the same or another subject. Similarly, elevation in the phosphorylation level of c-Jun or in the expression of cyclin D1 may be detected by comparison with a control sample taken from normal tissue of the same or another subject.

Buparvaquone may be comprised in a pharmaceutical composition, with at least one pharmaceutically acceptable excipient. By "pharmaceutically acceptable excipient", it is meant herein a compound of pharmaceutical grade which improves the delivery, stability or bioavailability of an active agent, and can be metabolized by, and is non-toxic to, a subject to whom it is administered. Preferred excipients according to the invention include any of the excipients commonly used in pharmaceutical products, such as, for example, microcrystalline cellulose, lactose, starch, and soybean powder.

The pharmaceutical composition comprising buparvaquone may preferably be in a form suitable for the purposes of the invention. For example, said composition may be in a form suitable for parenteral or oral administration, such as a liquid suspension, a solid dosage form, or a paste. The term parenteral as used herein includes subcutaneous, intravenous, intramuscular, and intraperitoneal injection. The compositions can also be administered transdermally by topical administration to skin and mucosal membranes.

In the context of using buparvaquone for the treatment of cancer, the subject is preferably a mammal, even more preferably a human subject.

The present invention will be better understood in the light of the following detailed description of experiments, including examples. Nevertheless, the skilled artisan will appreciate that this detailed description is not limitative and that various modifications, substitutions, omissions, and changes may be made without departing from the scope of the invention.

### BRIEF DESCRIPTION OF THE DRAWINGS

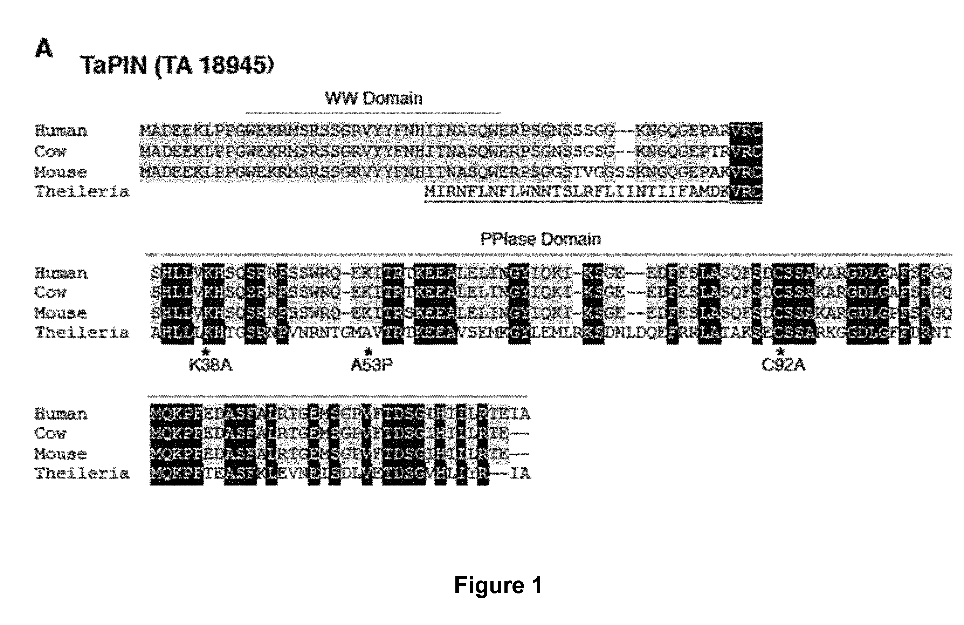

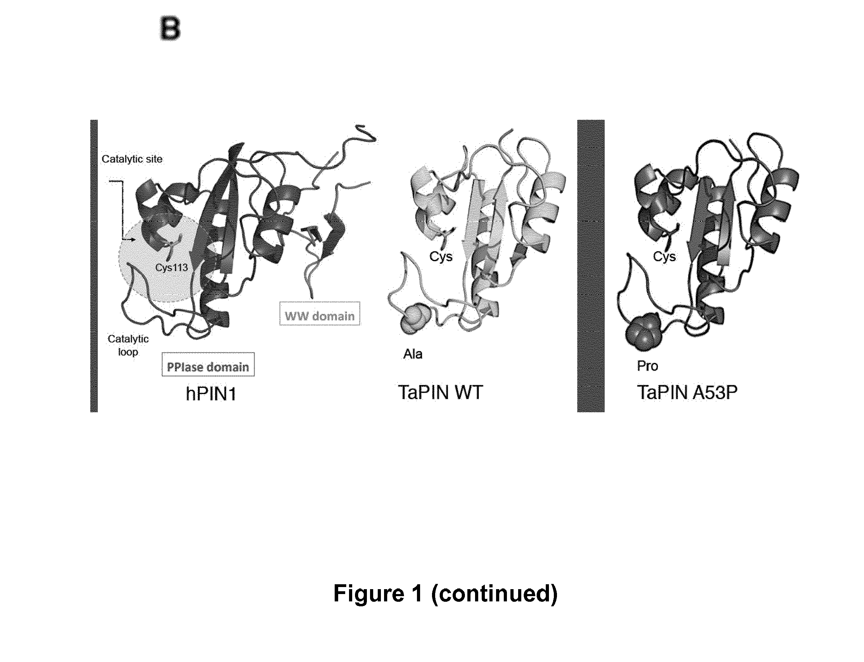

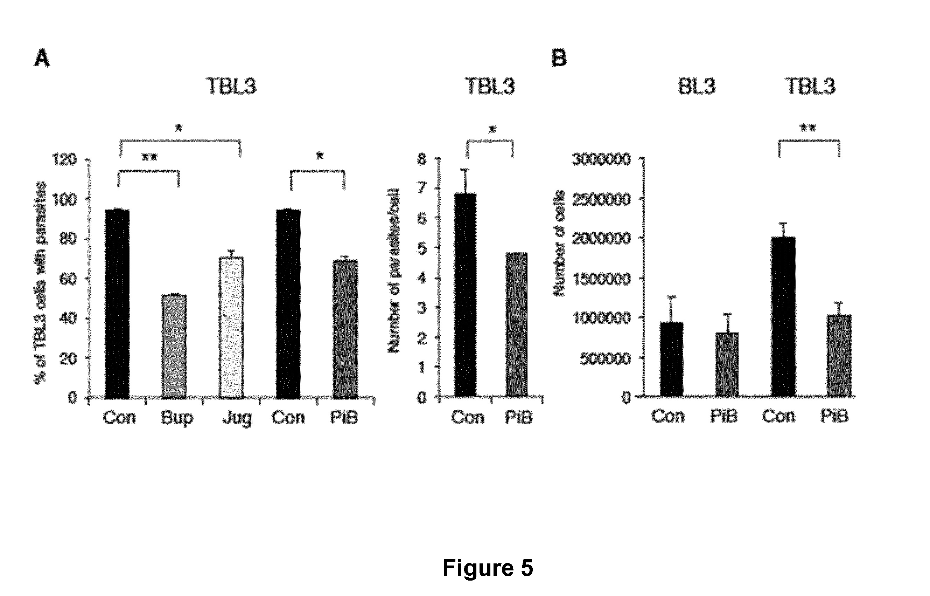

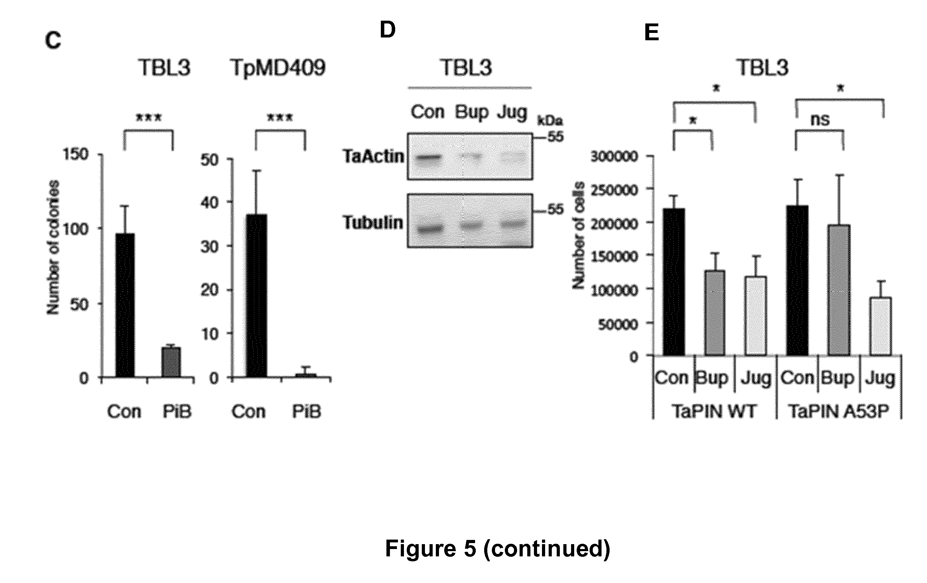

**Figure 1****. *Theileria* possess a gene coding for conserved PPlase PIN1.**
   A. Sequence alignment of PIN1 genes in *Homo sapiens (Human)*, *Bos taurus (Cow)*, *Mus musculus (Mouse)* and *Theileria annulata (Theileria)* shows that *T. annulata* PIN (TaPIN) has an *in silico* predicted signal peptide and a well conserved PPlase catalytic domain. Stars indicate TaPIN residues that were mutated in subsequent experiments.
   B. 3D structure of the experimentally determined hPIN1 structure and of the TaPIN WT and mutant models (ribbon diagram). The 3D structures are well-conserved among these molecules with some differences in the catalytic loops. The TaPIN enzyme also lacks the WW domain present in hPIN1.

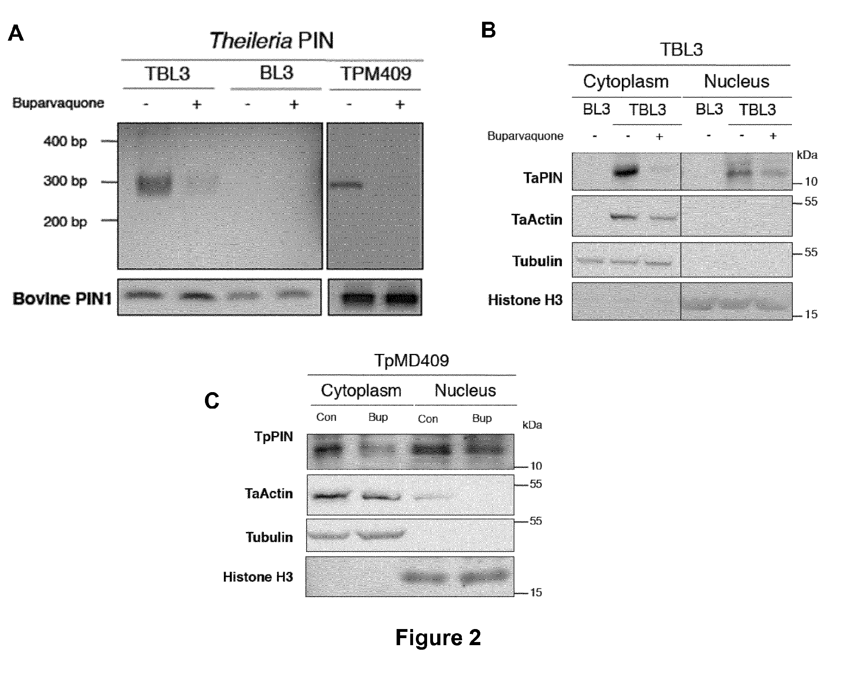

**Figure 2****. *Theileria* express and secretes a conserved PIN1 found in cytoplasmic and nuclear host fraction.**
   A. *Theileria*-infected cells express TaPIN transcripts. Quantitative PCR analysis of the expression level of *Theileria* PIN using RNA from *T. annulata*-infected TBL3 cells, BL3 parental non-infected cells or *T. parva*-infected TpMD409 cells, with/without Buparvaquone treatment (Bup) (this image is representative of 3 independent experiments).
   B. The *Theileria* PIN protein is secreted into the host cells and is located in the cytoplasm and the nucleus of infected cells. Nuclear and cytoplasmic proteins were extracted from non-infected BL3 and parasite-infected TBL3 and TpMD409 cells. The parasite protein TaPIN and TaActin (*T.annulata* actin) were detected using specific antibodies. TaActin is found only in the cytoplasmic fraction of the host cells, whereas TaPIN is mainly present in the host nuclear fraction. Bovine Histone H3 (nuclear) and Tubulin (cytoplasmic) are loading controls (this image is representative of 3 independent experiments).
   C. TpPIN protein was detected in the nuclear and cytoplasmic fractions of *T. parva*-infected TpM409 B cells and decreased upon Buparvaquone treatment. Antibodies recognizing Apicomplexa actin, bovine tubulin or bovine histone H3 were used as controls (these results a representative of three independent experiments).

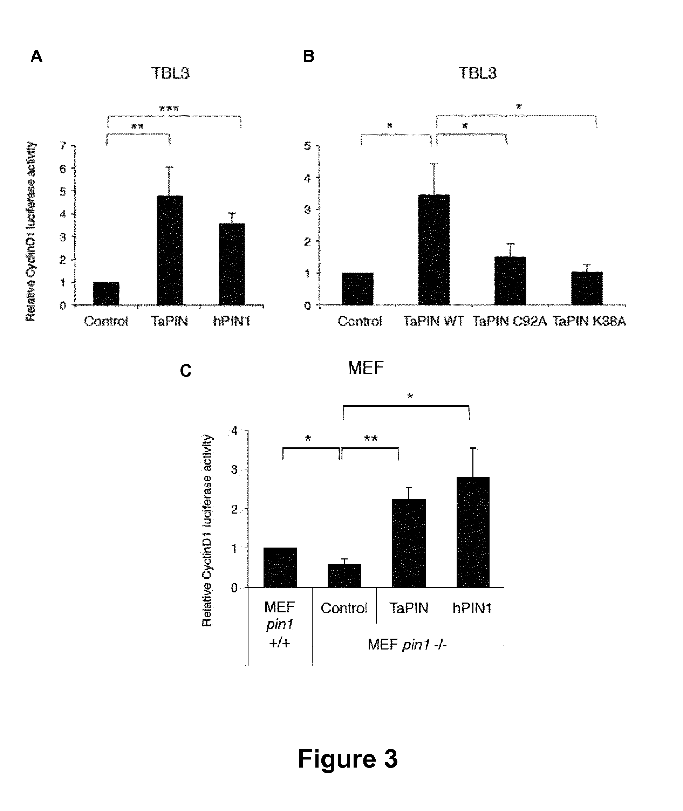

**Figure 3****: TaPIN functionally replaces hPIN1.**
   A. TaPIN and hPIN1 increase *CyclinD1* promoter activity as measured using a *CyclinD1-Luciferase* reporter (a well-known hPIN1 target) in *Theileria*-infected TBL3 cells. Transfection efficiency was normalized with co-transfection of a Renilla-encoding plasmid and results are presented relative to an empty-plasmid control (average ± sd, n=3).
   B. C92A and K38A TaPIN mutants were deficient in activating the *CyclinD1* promoter in parasite infected cells (TBL3). Transfection efficiency was normalized with co-transfection of a Renillaencoding plasmid and results are presented relative to an empty-plasmid control (average ± sd, n=3). *P<0.05, **p<0.01, ***p<0.001
   C. Decreased of *CyclinD1-Luciferase* promoter activity in PIN1-/- MEFs compared to PIN1+/+ MEFs, was rescued by transfection of TaPIN or hPIN1. Transfection efficiency was normalized with cotransfection of a Renilla-encoding plasmid (average ± sd, n=3).

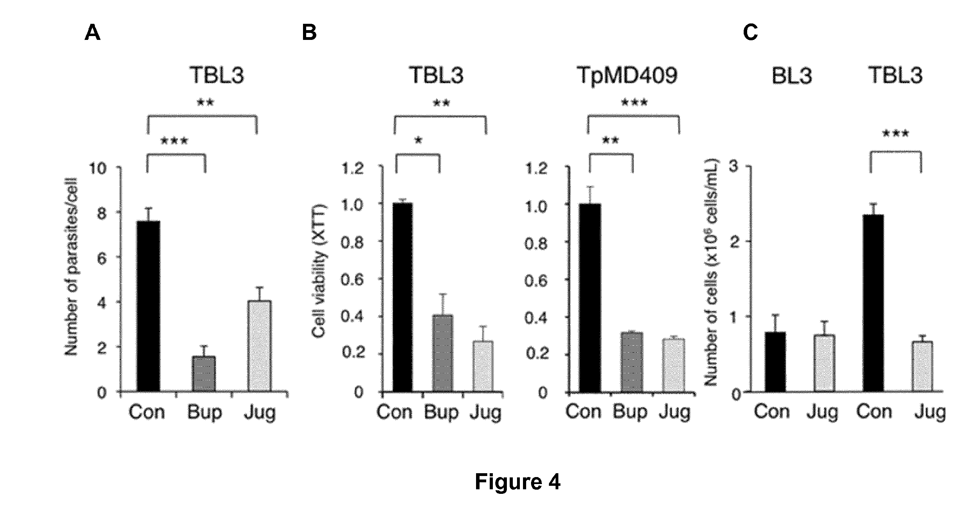

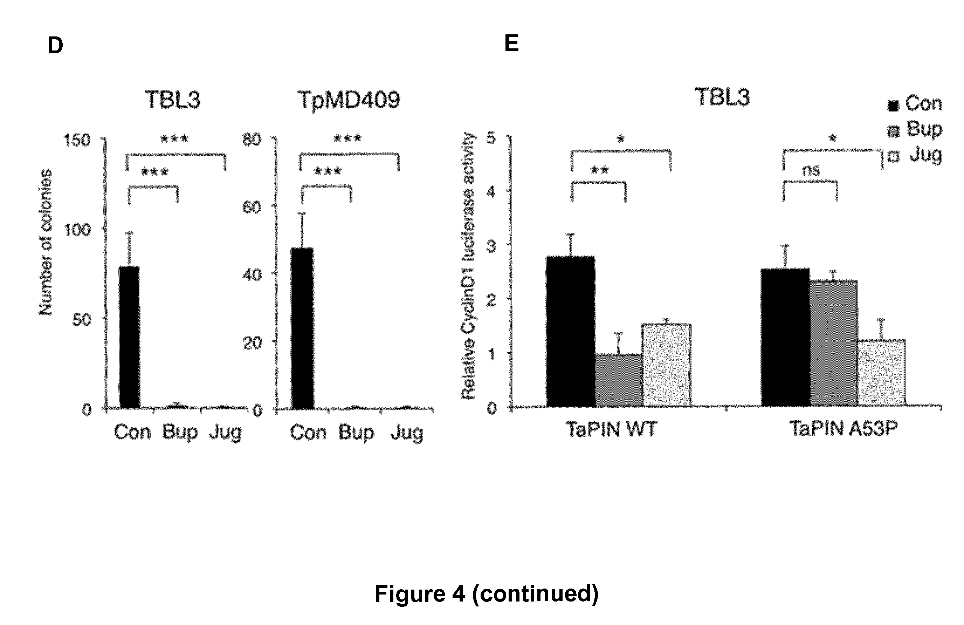

**Figure 4****. Buparvaquone and Juglone target TaPIN.**
   A. Buparvaquone and Juglone eliminate the *Theileria* parasites in TBL3-infected cells. *Theileria* infected cells were treated by Buparvaquone and Juglone for 72h and parasites were counted following DAPI staining (average ± sd, n=3).
   B. Buparvaquone and Juglone decreased the viability of parasite-infected cells (*Theileria annulata* - TBL3 and *Theileria parva* - TpMD409). Cells viability upon Buparvaquone or Juglone treatment (72h) was assessed using the XTT assay (average ± sd, n=3).
   C. Juglone treatment affects TBL3 cell proliferation. Parasite-infected TBL3 or non-infected BL3 cells were grown in the presence or absence of Juglone. Live cells were counted following trypan blue exclusion. Juglone had no effect on the proliferation of non-parasitized BL3 cells (average ± sd, n=3).
   D. Parasite-infected TBL3 cells and TpMD409 formed colonies when grown in soft agar. Treatment with Buparvaquone or Juglone for 72h reversed the transformed phenotype. The number of colonies per plate, and their appearance under the microscope 10 days after plating, are shown. (average ± sd, n=3).
   E. *CyclinD1* promoter activation by TaPIN WT was reversed upon Buparvaquone or Juglone treatments in parasite-infected cells (TBL3). Buparvaquone had no significant effect on *CyclinD1* promoter activation induced by the TaPIN Mutant A53P. The transfection efficiency was normalized with co-transfection of a Renilla-encoding plasmid (average ± sd, n=3). *P<0.05, **p<0.01, ***p<0.001
**Figure 5****. PIN1 inhibitors eliminate *Theileria* parasites in infected cells.**
   A. Buparvaquone, Juglone and PiB eliminate parasites in TBL3 cells. Infected cells were treated with Buparvaquone, Juglone or PiB for 72h and parasites were detected following DAPI staining (average ± sd, n=3).
   B. PiB affects TBL3 cell proliferation. Parasite-infected TBL3 or non-infected BL3 cells were grown in the presence or absence of PiB and cell numbers were monitored by counting live cells following trypan blue exclusion. PiB had no effect on the proliferation of non-parasitized BL3 cells (average ± sd, n=3).
   C. The colony forming potential of TBL3 and TpMD409 cells was reduced after PiB treatment. *Theileria* infected cells were plated in soft agar with or without the addition of PiB. Colony numbers are the average of three independent experiments.
   D. Buparvaquone and Juglone treatment decreased parasite load in infected TBL3 cells as measured by western blot analysis of parasite actin (TaActin) after 72h treatment. Bovine Tubulin levels control for host cell proteins. (Representative of 3 independent experiments).
   E. Overexpression of the TaPIN Mutant A53P rescued the Buparvaquone effect on TBL3 cell proliferation. Parasite-infected TBL3 cells transiently expressing TaPIN WT or TaPIN Mutant A53P were grown in the presence or absence of Buparvaquone or Juglone. Cell numbers were monitored by counting live cells following trypan blue exclusion (average ± sd, n=3). *P<0.05, **p<0.01.

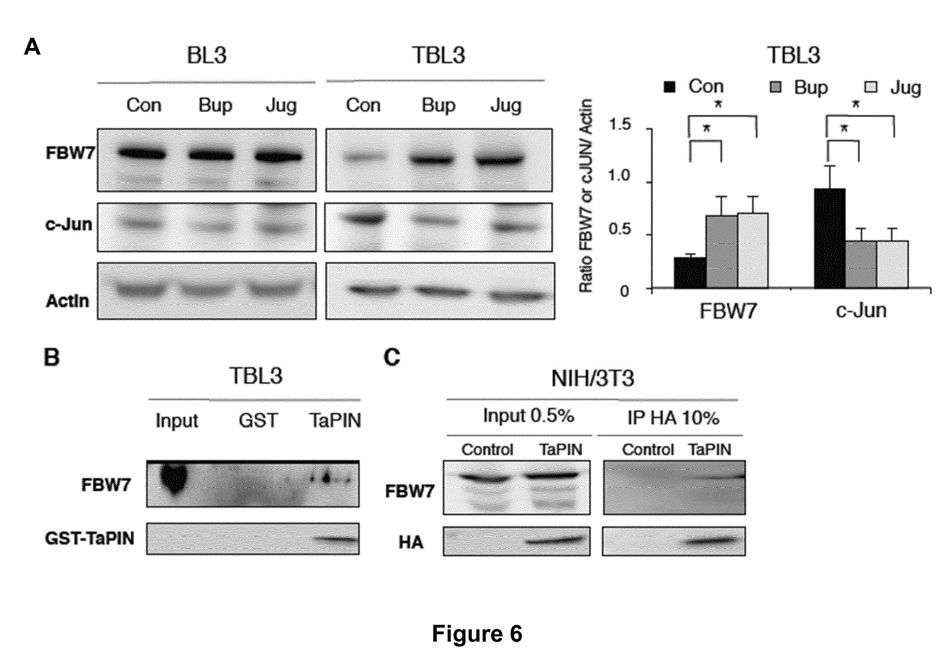

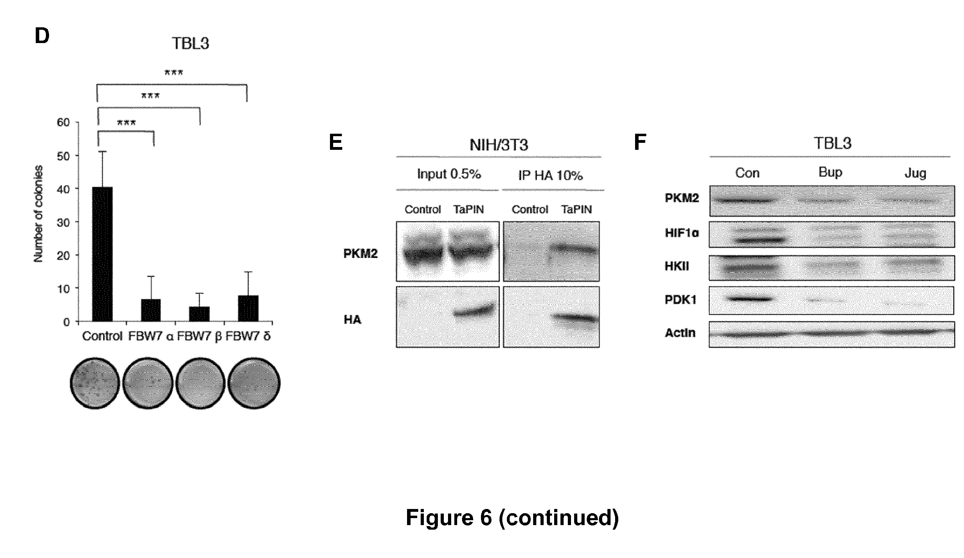

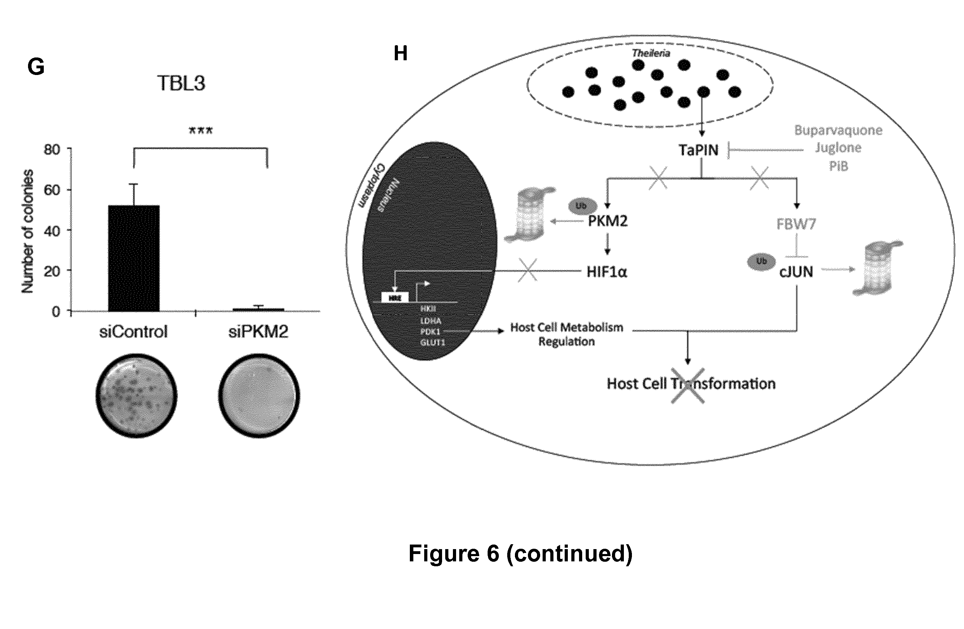

**Figure 6****. TaPIN induces host cell transformation through the regulation of Fbw7.**
   A. Inhibition of TaPIN in TBL3 cells induced FWB7 protein and decreased c-Jun protein expression. Relative quantification (right panel) indicates the FBW7/Actin and c-Jun/Actin ratios calculated using Image J software (average ± sd, n=3).
   B. TaPIN interacts with endogenous bovine FBW7. Parasite-infected TBL3 cell lysates were incubated for 4h with GST-TaPIN or GST-coupled beads, followed by immunoblot analysis using an FBW7 antibody (representative result, n=3).
   C. TaPIN interacts with endogenous mouse FBW7. Protein extract from NIH/3T3 cells stably expressing HA-Flag-TaPIN or HA-Flag-Control were used for HA immunoprecipitation, followed by immunoblot analysis using FBW7 and HA antibodies (representative of 3 independent experiments).
   D. The transformed phenotype of TBL3 cells was markedly reduced by the ectopic expression of the human FBW7 isoforms. The photographs are representative of three independent experiments and the average number of colonies per plate are indicated (average ± sd, n=3). *P<0.05, **p<0.01, ***p<0.001.
   E. TaPIN interacts with mammalian PKM2 protein. Protein extract from NIH/3T3 cells stably expressing HA-Flag-TaPIN or HA-Flag-Control were used for HA immunoprecipitation, followed by immunoblot analysis using PKM2 and HA antibodies (representative result of 3 independent experiments).
   F. Inhibition of TaPIN by Buparvaquone or Juglone in TBL3 cells reduced PKM2 and HIF1α protein expression, as well as two HIF1α target genes, Hexokinase II and PDK1. Actin was a loading control (results are representative of 3 independent experiments).
   G. Inhibition of PKM2 reversed the transformed phenotype of parasite-infected TBL3 cells. The photographs are representative of three independent experiments and the averages "number of colonies per plate" are indicated (average ± sd, n=3).
   H. *T. annulata* parasites secrete a conserved oncoprotein, TaPIN, into the host cell compartment to rewire host glucose metabolism and drive cell transformation. TaPIN interacts with the host ubiquitin ligase FBW7, leading to auto-ubiquitination, thereby inducing the accumulation of c- JUN, a target of FBW7. In parallel, TaPIN interacts with host Pyruvate Kinase, PKM2, and enhances its stability. PKM2 is a co-activator of HIF1α. Consequently, by stabilizing PKM2, TaPIN up-regulates glycolytic enzymes expression and host cell metabolism leading to a "Warburg- like" effect. Inhibition of TaPIN by Buparvaquone, Juglone or other known hPIN1 inhibitors, causes increased FBW7 levels and degradation of c-Jun. Inhibition of TaPIN also induces proteasomal degradation of PKM2, reducing HIF1α transcriptional activity and contributing to the death of both parasites and the host cell. *P<0.05, **p<0.01, ***p<0.001.
**Figure 7****. TaPIN is well conserved between *Theileria annulata* and *Theileria parva.*** Sequence alignment of PIN1 genes in *T. annulata* (TaPIN) and *T. parva* (TpPIN). The annotated *T. annulata* intron is indicated in grey and the annotated Methionine codon (ATG) in *T. parva* is indicated on the sequence. TaPIN and TpPIN are well conserved including in the intronic sequence.
**Figure 8****. Buparvaquone-resistant parasites are mutated in the TaPIN PPlase domain.**
   A. Sequence alignment of the TaPIN gene in *T.annulata* WT or Buparvaquone-resistant genomes. The annotated *T.annulata* intron is indicated in grey. Buparvaquone-resistant parasites exhibit three nucleotide mutations in the exonic region encoding the signal peptide sequence, as well as three nucleotide mutations in the exonic region encoding the PPlase domain.
   B. Sequence alignment of the TaPIN protein in *T.annulata* WT and Buparvaquone-resistant strains. Buparvaquone-resistant parasites exhibit one mutation in the PPlase domain (Ala53Pro), as well as two mutations in the signal peptide (Thr22Ile and Ala26Leu).

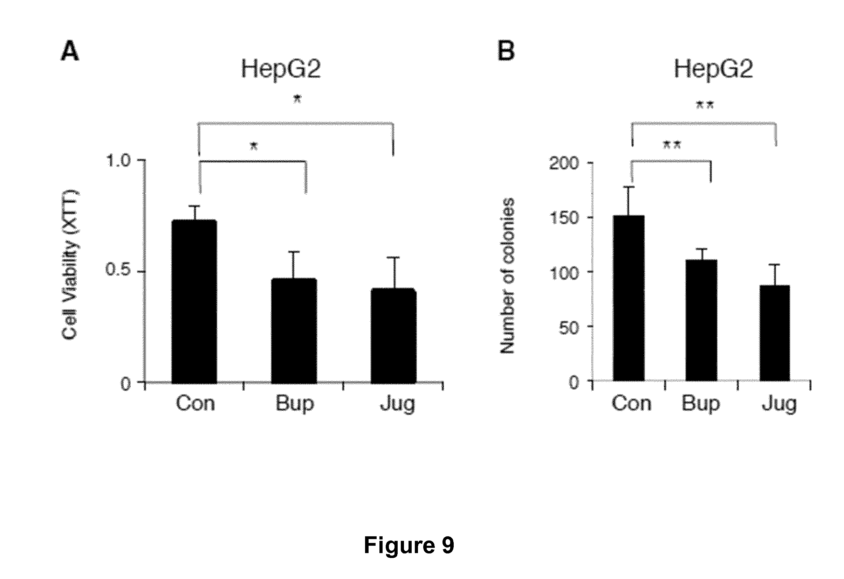

**Figure 9****. Inhibition of PIN1 by Buparvaquone affects cancer cell transformation.**
   A. Buparvaquone and Juglone reduce the viability of HepG2 cells. Cell viability after Buparvaquone or Juglone treatment for 72h was assessed using the XTT assay (average ± sd, n=3).
   B. The soft agar colony-forming potential of HepG2 cells is decreased after Buparvaquone or Juglone treatment. The number of colonies per plate is shown (average ± sd, n=3). *P<0.05, **p<0.01, ***p<0.001.

### EXAMPLES

### EXAMPLE 1 - PIN1 inhibitors for treating lymphoproliferative Theileriosis

### 1. MATERIAL AND METHODS

### 1.1. Cell lines and culture conditions

All infected bovine cell lines used in this study were previously described: TBL3 cells were derived from *in vitro* infection of the spontaneous bovine-B lymphosarcoma cell line, BL3, with Hissar stock of *T.annulata.* The TpMD409 lymphocyte cell line are infected with *T. parva.* The culture conditions of these cell lines were previously described by Moreau et al., 1999. Cells were cultured in RPMI 1640 (Gibco-BRL, Paisley, UK), supplemented with 10% heat-inactivated Fetal calf serum, 4mM L-Glutamine, 25mM HEPES, 10µM β-mercaptoethanol and 100µg/ml penicillin/streptomycin in a humidified 5%CO₂ atmosphere at 37°C. HepG2, NIH/3T3 and MEF cells lines were cultured in DMEM (Gibco-BRL, Paisley, UK), supplemented with 10% heat-inactivated Fetal calf serum, 4mM L-Glutamine, 25 mM HEPES, 10µM β-mercaptoethanol and 100µg/ml penicillin/streptomycin in a humidified 5% CO₂ atmosphere at 37°C. Cell numbers, as judged by Trypan Blue exclusion test, were determined by counting cells using a Countess automated cell counter (Invitrogen, CA, USA). The anti-parasite drug Buparvaquone (BW720c) was used at 200ng/ml for 72 hours. BW720c has no effect on growth of uninfected cells.

Cells were treated with either Juglone at 5 µM resuspended in Ethanol (Sigma, St Louis, MO, Ref.: H47003), or PiB at 1 µM resuspended in DMSO (Sigma, Ref.: B7688), or Dimethyloxalylglycine DMOG at 0.5 mM resuspended in DMSO (Tebu-bio, MO, USA).

### 1.2. Cloning of wild-type TaPIN and control genes

The parasites genes coding for TaPIN WT (TA18945), and TaCyclophilin (TA19600), and the gene coding for human PIN1 (AAC50492.1),were independently cloned between the restriction sites *Xho*I and *Not*I of the pREV-HA-FLAG-RIL2 plasmid, using the specific oligonucleotides described in Table 2 below.

**Table 2: cloning primers**

| **Target** | **Forward primer (5' to 3') (SEQ ID NO)** | **Reverse primer (5' to 3') (SEQ ID NO)** |
|---|---|---|
| TaCyclophylin | CCGCTCGAGTTCTACAATCAACCCAAGCAT (SEQ ID NO:29) | AAGGAAAAAAGCGGCCGCTCACAATAATTCTCCACAGTCC (SEQ ID NO:30) |
| human PIN1 (hPIN1) | CCGCTCGAGGCGGACGAGGAGAAGCTG (SEQ ID NO:31) | AAGGAAAAAAGCGGCCGCTCACTCAGTGCGGAGGATGA (SEQ ID NO:32) |
| TaPIN wt | CCGCTCGAGGCCCACTTGCTACTAAAG (SEQ ID NO:33) | ATAAGAATGCGGCCGCTTATGCGATTCTATATATAAGATG (SEQ ID NO:34) |

### 1.3. Parasite genomic DNA extraction and sequencing of Buparvaquone-resistant infected cells

Buparvaquone-resistant infected cells were cultured in RPMI 1640 and parasite DNA was extracted using the kit Promega (Wizard Genomic DNA Purification Kit, Ref.: A1125) following the manufacturer's instructions.

To sequence the TaPIN gene expressed in these resistant cells, a PCR was performed with the oligonucleotides of sequence SEQ ID NO:35 and SEQ ID NO:36 of Table 2 using High Fidelity Platinium Taq Polymerase (Invitrogen, Ref: 11304), followed by sequencing using the same oligonucleotides as well as the primers of sequence SEQ ID NO:37 and SEQ ID NO:38 indicated in Table 3 below.

**Table 3: sequencing primers for TaPIN**

| **Target** | **Sequencing primer (5' to 3') (SEQ ID NO)** |
|---|---|
| TaPIN | CCGCTCGAGGCCCACTTGCTACTAAAG (SEQ ID NO:35) |
| TaPIN | ATAAGAATGCGGCCGCTTATGCGATTCTATATATAAGATG (SEQ ID NO:36) |
| TaPIN | GTCTGTCAAATAGGTAGAAATC (SEQ ID NO:37) |
| TaPIN | GAGAGGAAGTTGAATCAAACAT (SEQ ID NO:38) |

### 1.4. Cloning of mutated TaPIN

After sequencing the TaPIN gene expressed in Buparvaquone-resistant infected cells, the Ala53Pro point mutation identified in said gene was introduced into the pRev-HA-FLAG-TaPIN WT-RIL2 plasmid using the set of primers of sequence SEQ ID NO:41 and SEQ ID NO:42 following a 3-step PCR protocol.

The point mutations Lys38Ala and Cys92Ala were similarly introduced, independently from each other, into the pRev-HA-FLAG-TaPIN WT-RIL2 plasmid, using the set of primers SEQ ID NO:39 and SEQ ID NO:40, and the set of primers SEQ ID NO:43 and SEQ ID NO:44, respectively. These residues were not detected as being mutated in Buparvaquone-resistant infected cells, but were previously identified as critical for the catalytic activity of human PIN1 (Zheng et al., 2011).

**Table 4: cloning primers for mutated TaPIN**

| **Target** | **Forward primer (5' to 3') (SEQ ID NO)** | **Reverse primer (5' to 3') (SEQ ID NO)** |
|---|---|---|
| TaPIN K38A | GCCCACTTGCTACTAGCGCACACTGGATCTAGG (SEQ ID NO:39) | CCTAGATCCAGTGTGCGTAGTAGCAAGTGGGC (SEQ ID NO:40) |
| TaPIN A53P | GGAATACTGGAATGCCAGTAACAAGAAC (SEQ ID NO:41) | GTTCTTGTTACTGGCATTCCAGTATTCC (SEQ ID NO:42) |
| TaPIN C92A | GCAACTGCCAAATCTGAGGCTTCAAGCGCAAGAAAAGG (SEQ ID NO:43) | CCTTTTCTTGCGCTTGAAGCCTCAGATTTGGCAGTTGC (SEQ ID NO:44) |

### 1.5. RNA extraction and Reverse Transcription-qPCR

Total cellular RNAs were extracted using an RNeasy Plus Mini Kit (Qiagen, Hilden, Germany) and parasite RNAs were extracted using the classical Trizol Protocol. cDNA synthesis was performed with the Reverse Transcriptase Superscript III (Invitrogen, CA, USA).

Quantitative PCR amplification was performed in the ABI 7500 machine (Applied Biosystems, CA, USA) using the Sybr Green reagent (Applied Biosystems, CA, USA) using the primers listed in Table 5 below. The detection of a single product was verified by dissociation curve analysis. Relative quantities of mRNA were analyzed using the delta Ct method. The β*actin* qPCR was used for normalization.

**Table 5: qPCR primers**

| **Target** | **Forward primer (5' to 3') (SEQ ID NO)** | **Reverse primer (5' to 3') (SEQ ID NO)** |
|---|---|---|
| cJUN | ACGTTTTGAGGCGAGACTGT (SEQ ID NO:57) | TCTGTTTCCCTCTCGCAACT (SEQ ID NO:58) |
| FBW7 | AGCTGGAGTGGACCAGAGAAATTG (SEQ ID NO:59) | GAATGAGAGCACGTAAAGTGC (SEQ ID NO:60) |
| PIN1 | GGCCGGGTGTACTACTTCAA (SEQ ID NO:61) | TTG GTTCGGG TG ATCTTCTC (SEQ ID NO:62) |
| β-actin | GGCATCCTGACCCTCAAGTA (SEQ ID NO:63) | CACACGGAGCTCGTTGTAGA (SEQ ID NO:64) |

### 1.6. Nucleus/Cytoplasmic protein extraction

Cells were lysed in the following buffer: 5mM Tris HCL pH7.5, 40mM KCL, 2mM MgCL2, 0.5mM EDTA, 0.05mM Spermidine and Spermine, 0.1% NP-40, H2O. Lysates were incubated for 5min on ice and centrifuged for 10min at 5000rpm. The supernatant constitutes the Cytoplasmic fraction and the pellet constitutes the nuclear fraction.

### 1.7. Immunoblot analysis and immunostaining

Total proteins were extracted with Laemmli lysis, sonicated: 15 secs ON / 30 secs OFF for 5 min, resolved on 10.5% acrylamide/bis-acrylamide SDS-PAGE gels and transferred to nitrocellulose membranes (Thermo Fisher Scientific, MA, USA) in transfer buffer. Protein transfer was assessed by Ponceau-red staining. Membranes were blocked in Tris-buffered saline pH7.4 containing 0.1% Tween-20 and 5% milk for 1 h at room temperature. Incubations with primary antibodies were carried out at 4°C overnight using antibody dilutions as manufacturer recommendations in Trisbuffered saline pH7.4, 0.05% Tween-20 and 5% milk. Following 1 h incubation with an anti-rabbit or anti-mouse peroxidase-conjugated antibody (Sigma, St Louis, MO, USA) at room temperature, proteins were detected by chemiluminescence (Thermo Fisher Scientific, MA, USA) following the manufacturer's instructions. The following antibodies were used: Rabbit anti-TaPIN (Home-made antibody, Proteogenix, Peptide used: NPVNRNTGMAVTR-Cys), rabbit anti-PIN1 (Cell Signaling, Ref: 3722), rabbit anti-TaActin (kindly provided by Jake Baum, Australia), rabbit anti-c-Jun (Santa Cruz, CA, USA Ref: sc1694), mouse anti-αTubulin (Sigma, Ref: T9026), , rabbit anti-HIF1α (Abcam, Cambridge, UK; Ref: ab75918), rabbit anti-Hexokinase II (Cell Signaling, Ref: C64G5), rabbit anti-PDK1 (Cell signaling, Ref: C47H1), mouse anti-HA (Roche, Ref: 11583816001), rabbit anti-FBW7 (Bethyl Laboratories, Ref: A301-721A), rabbit anti-Histone H3 (Abcam, Ref: ab1791), rabbit anti-PKM2 (Abcam, Ref: ab38237), mouse anti-Actin (Sigma, Ref: A1978) and monoclonal anti-FLAG M2-Peroxidase (Sigma, Ref: A8592). c-Jun, FBW7 or PKM2/Actin ratios were calculated after Western blot signal quantification with the Plot Lanes Analysis tool of Image J software (National Institutes of Health, Bethesda, MD, USA).

### 1.8. Parasites Quantification

After indicated treatments, parasite-infected cells were plated on slides using CytoSpin centrifugation at 2000rpm for 10min. Cells were fixed in PBS 3.7% Formaldehyde for 15 min at room temperature. Slides were mounted and coverslipped with ProLong Gold Antifade Reagent with DAPI (Invitrogen, CA, USA. Ref: P-36931). Images of immunofluoresence staining were photographed with a fluorescent microscope (Leica Inverted 6000) and the number of parasites per cells was counted. Staining was repeated for three independent biological replicates.

### 1.9. Spreading Assay

Mouse Embryonic Fibroblast (MEF) cells transfected by the indicated constructs (pREV-HA-FLAG-RIL2, pREV-HA-FLAG-RIL2-TaPIN, pREV-HA-FLAG-RIL2-TaCyclophilin or pREV-HA-FLAG-RIL2-hPIN1 were plated on slides and then fixed in PBS 3.7% Formaldehyde for 15min at room temperature. Slides were rinsed in PBS and permeabilized with PBS 0.2% Triton X-100 for 5min, and then blocked for 30min with PBS 1%SVF and 1%BSA to prevent nonspecific staining. The slides were incubated with mouse anti-HA (1:1000, Roche, Ref: 11583816001) in PBS 1%SVF and 1%BSA at room temperature for 40min. After washing in PBS 0.2% Tween, the slides were incubated with Cy2 AffinyPure anti-mouse IgG (1:5000, Jackson Immunology, Ref: 715-225-150) for 30min. After three washes in PBS 0.2% Tween, slides were incubated for 15min with Phalloidin-TRITC (Life Technologies, USA. Ref: R415). Slides were subsequently washed in PBS 0.2% Tween, mounted on slides and covered with ProLong Gold Antifade Reagent with DAPI (Invitrogen, USA. Ref.: P-36931). Images of immunofluoresence staining were photographed with a fluorescent microscope (Leica Inverted 6000). Staining was repeated for three independent biological replicates.

### 1.10. Luciferase Assay

Bovine cells were transfected with the 5xHRE (Staller et al., 2003) or CyclinD1- (Wulf et al., 2001) Luciferase reporters, using electroporation (Neon kit, Invitrogen, CA, USA). Mouse cells were transfected using Lipofectamine 2000 (Invitrogen, CA, USA). Transfections efficiencies were normalized to Renilla activity by co-transfection of a pRL-TK *Renilla* reporter plasmid (Promega, Ref.: E6241). Luciferase assays were performed 36h post-transfection using the Dual-Luciferase Reporter Assay System (Promega, Ref: E1980) in a microplate luminometer. Relative luminescence was represented as the ratio Firefly/Renilla luminescence, compared with the corresponding empty vector control. The 5xHRE promoter-Luciferase construct was obtained from P Staller (Biotech Research & Innovation Centre, Copenhagen, Sweden).

### 1.11. Soft Agar Colony Forming Assay

A two-layer soft agar culture system was used. A total of 20,000 cells (for bovine cells) or 40,000 cells (for human and mouse cells) were plated in a volume of 1.5 ml (0.7% SeaKem ME Agarose: Lonza, Basel, Switzerland, Ref.: 50011) + 2x DMEM 20% Fetal calf Serum over 1.5-ml base layer (1% SeaKem ME Agarose + 2x DMEM 20% Fetal calf Serum) in 6-well plates. Cultures were incubated in humidified 37°C incubators with an atmosphere of 5%CO2 in air, and control plates were monitored for growth using a microscope. At the time of maximum colony formation (10 days in culture), final colony numbers were counted manually after fixation a staining with 0.005% Crystal Violet (Sigma, Ref.: C3886).

### 1.12. Colony Forming Assay

A total of 1,500 cells (for human and mouse cells) were plated in 6-well plates. Cultures were incubated in humidified 37°C incubators with an atmosphere of 5%CO2 in air, and control plates were monitored for growth using a microscope. At the time of maximum colony formation (10 days in culture), final colony numbers were counted manually after fixation and staining with 0.5% Crystal Violet (Sigma, Ref.: C3886).

### 1.13. GST pull down

Plasmid constructs, as described in sections 1.2 and 1.4 above, were expressed in *E. coli* strain BL21-DE3 and purified using glutathione sepharose beads. Concentration of purified protein was estimated by Coomassie staining. Beads coated with 1µg of GST fusion proteins were incubated with 250µL of cell lysate (see Immunoprecipitation - HA) in 50mM Tris pH7.6, 150mM NaCl, 0.1 % Triton, for 2h at 4°C. Beads were washed 5 times with 50mM Tris pH7.6, 300mM NaCl, 0.5 % Triton 100. Proteins were revealed by Western Blot analysis using specific antibodies.

### 1.14. Protein complex immune-purification

Retroviral transduction strategy was used to establish NIH/3T3 cell lines expressing double-tagged proteins (Robin et al., 2007; Ouararhni et al., 2006). Polyclonal NIH/3T3 cell lines stably expressing Flag-HA-tagged hPIN1 or TaPIN were established. All the proteins were tagged with double-HA (Haemagglutinin) and double-FLAG epitopes at the N-terminus. A control cell line transduced with the empty pREV vector was established. Double-affinity purification of Flag-HA-hPIN1 from NIH/3T3 were carried out, using either nuclear soluble or cytoplasmic fractions. Both fractions were then subjected to a two-step immunopurification with Flag and HA antibodies as described previously (Fritsch et al., 2010).

### 1.15. Immunoprecipitation - HA

NIH/3T3 cells stably expressing TaPIN were lysed in the following buffer: 20mM Tris HCL pH8, 150mM NaCl, 0.6% NP-40 and 2mM EDTA. Protein complexes were affinity-purified on anti-HA antibody-conjugated agarose (Sigma, Ref.: A2095) and eluted with the HA peptide (Ansynth, The Netherlands). After 5 washes, immunopurified complexes were resolved on 4-12% SDS-PAGE bis-Tris acrylamide gradient gel in MOPS buffer (Invitrogen, Ref.: NP 0322 BOX, NP0001-02, respectively).

### 1.16. Viability assays

1×10⁴ cells were plated in 96-well plates in triplicate and Buparvaquone, Juglone or PiB was added. Cell viability was measured after 48h using the Cell proliferation Kit II-XTT (Roche) and the GloMax- Multi Detection System (Promega).

### 1.17. siRNA silencing

Bovine cells were transfected with siRNA (Table 6) using electroporation (Neon kit, Invitrogen, CA, USA).

**Table 6. siRNA**

| **Target** | **siRNA sequence (5' to 3') (SEQ ID NO)** |
|---|---|
| PKM2 | CTGTGTCAGAAAAGGACATCC (SEQ ID NO:64) |

### 1.18. Data and statistical analysis

**The SPSS 19.0** program (SPSS Inc. Chicago, IL, USA) was used for statistics. The results presented in all the figures represent the mean ± SEM of at least three independent experiments. Statistical analysis was performed using the paired-samples t-test to analyse the significant difference between the control and treatment groups. p values of <0.05 were considered statistically significant and are indicated with asterisks *,p<0.05; **,p<0.01; ***,p<0.001.

### 1.19. Structures analysis

Homology models of TaPIN WT and Mutant A53P were built with the online server EsyPred (**Lambert** et al., 2002)). The experimental structure of human Pin1 (PDB file Pin1, **Ranganathan** et al., 1997) co-crystallized with a dipeptide Ala-Pro (resolution 1.35 A) was used as a template.

The server PCE described by (**Miteva** et al., 2005) was used to check the protonation state of the protein titratable groups. The most likely binding pocket areas for TaPIN WT were predicted with FTMap and investigation of side chain flexibility (if any) in the area of the predicted binding cavities was carried out with the server FTFlex (FTFlex: accounting for binding site flexibility to improve fragment-based identification of druggable hot spots, **Grove** et al., 2013).

The 2D structures of Juglone and Buparvaquone were obtained from PubChem and the 3D structures were generated with our package DG-AMMOS (**Lagorce** et al., 2009). Three docking tools, Surflex (**Spitzer** et al.; 2012), Molegro Virtual Docker (**Thomsen** et al., 2006) and MS-DOCK (**Sauton** et al., 2008) were used to search for possible poses of these compounds in hPIN1, for TaPIN WT and Mutant TaPIN A53P. Calibration of docking tools was performed on the thiol-stress sensing regulator co-crystallized with a quinone molecule, which in this structure is covalently attached to a Cys residue (PDB file 4HQM) (**Ji** et al, 2013). Visualization and figures were prepared with PyMol (DeLano, W.L. The PyMOL Molecular Graphics System (2002); DeLano Scientific (San Carlos, CA, USA).

### 2. RESULTS & DISCUSSION

### 2.1. PIN1: a key protein mediating Theileria infection

The intracellular parasite *Theileria* is the only Apicomplexa species which can transform its mammalian host cells. *Theileria* infection of bovine leukocytes (lymphocytes or macrophages) induces proliferative and invasive phenotypes which mirror human lymphoma pathologies. *Theileria* likely manipulates host cellular pathways and several signaling mechanisms have been implicated, including c-Jun N-terminal Kinase (JNK) and host nuclear factors c-Myc, HIFlalpha and AP-1 (**Chaussepied** et al., 1998; **Dessauge** et al., 2005; **Medjkane** et al., 2013). A remarkable feature of this model is the curability of the transformed phenotypes by treatment with the theilericidal drug Buparvaquone (BW720c), which kills the parasite (**Chaussepied** et al., 1996).

Key questions addressed herein are how does the parasite enter the host cells, what is the molecular mechanism of this parasite driving an oncogenic-like phenotype, and how does Buparvaquone cure this process?

The inventors surprisingly identified in *Theileria* a predicted peptidyl prolyl isomerase (PPlase) (TA18945) homologous to the human PIN1 enzyme as a promising candidate for parasite-encoded proto-oncogenes.

Human PIN1 is known to specifically catalyze the *cis*/*trans* isomerization of peptidyl-prolyl peptide bonds in phosphorylated Ser/Thr-Pro motifs leading to conformation changes that affect the substrates' stability, localization or activity. PIN1 activity is notably known to regulate key proteins involved in cell proliferation, pluripotency, cell survival and stress responses and mammalian PIN1 contributes to tumorigenesis.

Sequence alignment showed herein that this peptidyl prolyl isomerase (named herein TaPIN) has a highly conserved PPlase domain (**Figure 1**), but lacks a WW domain which is usually important for substrate recognition and binding present in mammalian PIN1.

Instead of a WW domain, this enzyme exhibits in N-terminal a signal peptide sequence which most certainly contributes to its secretion within the host cells (**Figure 2B**).

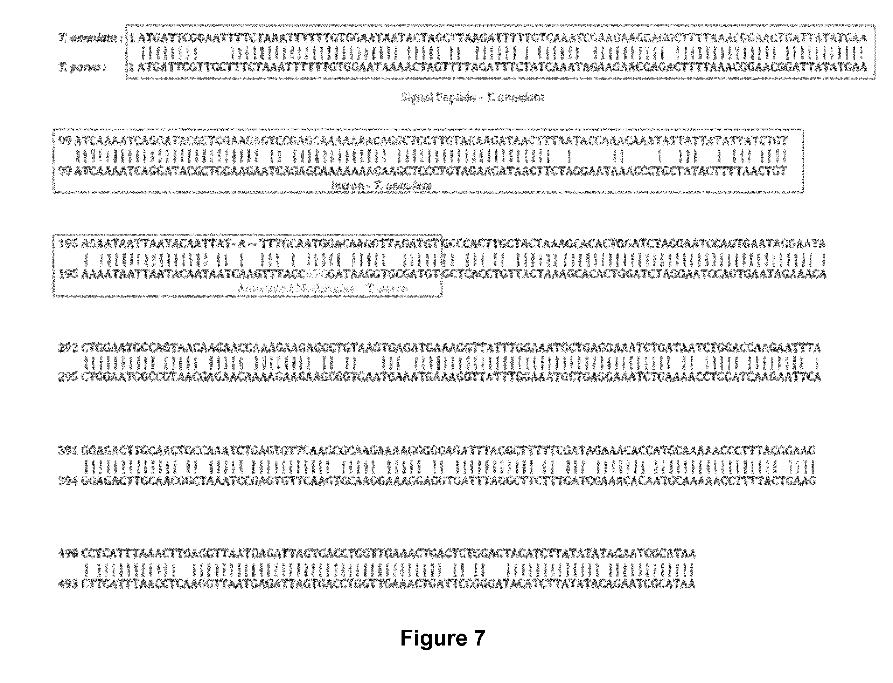

The inventors showed here that TaPIN transcripts were expressed in *T.annulata*-infected cells and lost upon treatment with Buparvaquone (**Figure 2A**). The *T.parva* genome contains a gene (TP01_0114) encoding a conserved TpPIN1 predicted protein (**Figure 7**) and the inventors observed also transcripts for this gene in *T.parva*-infected T cells which were reduced by Buparvaquone treatment (**Figure 2A**). The level of RNA transcripts encoding the host bovine bPIN1 protein was not affected either by *Theileria* infection or by a Buparvaquone treatment (**Figure 2A**). In addition, immunoblot analysis, carried out with a TaPIN antibody developed by the inventors, demonstrated that *Theileria annulata* and *Theileria parva* secrete TaPIN or TpPIN respectively both in the cytoplasm and the host nucleus of infected cells (**Figure 2B**).

By contrast, the PIN1 signal peptide appeared not to be conserved in the related *T.orientalis* genome, and this species is known not to transform infected host cells.

These combined results demonstrate that infectious *Theileria* species secrete a protein, related to PIN1, in the infected cells.

### 2.2. TaPIN, functional similarity with PIN1

The PPlase activity of *T. annulata* PIN protein was assessed on induction of the mammalian CyclinD1 promoter, an established readout for PIN1 activity (**Wulf** et al., 2001), and showed that TaPIN capacity to induce the cyclinD1-Luciferase reporter was equivalent to the one of hPIN1 in bovine B cells (**Figure 3A**), as well as in mouse fibroblasts (**Figure 3C**). In addition, the C92 and K38 amino-acid residues were mutated in TaPIN, based on the fact that these two key Cysteine and Lysine residues had previously been identified as critical for hPIN1 catalytic activity (**Zheng** et al., 2011). The introduction of those mutations in TaPIN confirmed the critical role of those residues, as their mutations led to a loss of the PPlase activity as measured by cyclinD1-Luciferase induction (**Figure 3B**).

Finally, PIN1 -/- MEF cells exhibited a spreading phenotype which was rescued by overexpressing hPIN1 as well as TaPIN, but not with TaCyclophilin, another predicted Theileria-secreted PPlase.

These combined results demonstrate that *Theileria* secretes a functionally active PPlase into the host cell, and that TaPIN could contribute to host cell proliferation.

### 2.3. PIN1 inhibitors as novel drugs for preventing or treating Theileriosis

Based on the above discovery, the inventors hypothesized that Buparvaquone (2-((4-tert-butylcyclohexyl)methyl)-3-hydroxy-1,4-naphthoquinone also known as BW-720c), which is the standard drug for treating Theileriosis, acts as a PIN1 inhibitor, and that other well-characterized PIN1 inhibitors, such as Juglone (5-hydroxy-1,4-naphthoquinone), may be used as alternative theilericidal drugs, particularly in Buparvaquone resistant subjects.

As a first step to verify this hypothesis, homology models of TaPIN protein were built based on the publicly available hPIN1 backbone, which revealed a similar structure with high conservation of the catalytic pocket (**Figure 1B**) (**Ranganathan** et al., 1997; **Yaffe** et al., 1997; **Lu** et al., 1999). One region of the catalytic loop was more positively charged in PIN1 compared to TaPIN, protruding into the solvent and reducing accessibility of the binding pocket. The Juglone and Buparvaquone molecules were then both docked into the active site of both TaPIN1 and hPIN1 models (data not shown). The TaPIN model was investigated with the binding pocket and hot-spot detection algorithm Ftmap, using the server FTFlex: the key hot spot regions predicted the catalytic site area as the most likely region for interaction with small chemical probes (data not shown). It further appeared that this predicted region precisely matched the area where the small Ala-Pro dipeptide co-crystallizes in the active site of hPIN1, supporting the quality of the homology model.

Based on this modeling analysis, which strongly implied that Buparvaquone targets TaPIN, and that Juglone might functionally replace Buparvaquone to block parasite transformation, the inventors, as a next step, treated Theileria-infected cells with Buparvaquone or Juglone. This experiment showed that both molecules reduced parasite load, host cell viability, cell proliferation and cell transformation (**Figure 4A, 4B, 4C** **and** **4D**).

Further experiments with another anti-PIN1 drug, namely PiB (diethyl-1,3,6,8-tetrahydro-1,3,6,8 tetraoxo benzo[Imn] phenanthroline-2,7-diacetate) showed that this drug also reduced parasite load, host cell viability, cell proliferation and cell transformation (Figure **5A-C**).

Those results confirmed that TaPIN1 is critical for *Theileria* transformation, and strongly showed that well-characterized PIN1 inhibitors can be used as alternative theilericidal drugs.

### 2.4. Juglone as a novel drug for preventing or treating Theileriosis resistant to Buparvaquone

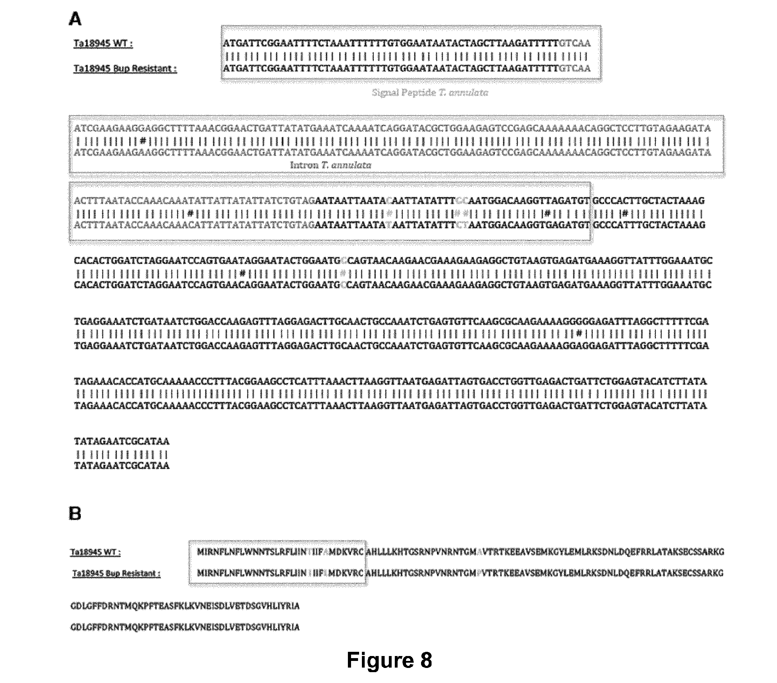

The emergence of Buparvaquone-resistant Theileria strains is an emerging clinical concern (**Mhadhbi** et al., 2010). Genomic DNA analysis from these drug resistant isolates showed the presence of mutations in the TaPIN gene, in particular a point mutation in the catalytic loop leading to an Alanine 53>Proline substitution (**Figure 8**). Structural modeling suggested that this mutation affects the nearby catalytic region and interaction with ligands. Docking of the small Juglone molecule showed that this drug could locate the electrophilic molecule to react with the thiolate group of Cys 92 or 113 in hPIN1, TaPIN or the TaPIN_A53P mutant. As Juglone is relatively small, it was thus not expected that the Ala>Pro mutation would impede this interaction (data not shown). By contrast, the Buparvaquone compound, is much larger (MW = 326) than Juglone (MW = 176) and contains a hydrophobic moiety. Two potential poses of the interaction of those drugs with hPIN1 or TapIN were proposed by the inventors (data not shown), but one pose appeared the most likely. Indeed, in this orientation (pose 1) *WT* TaPIN1 would differ from the A53P TapIN1 mutant, as possible direct or indirect steric hindrance might affect Buparvaquone accessibility.

The effect of both drugs was then assessed on *WT* or mutant TaPIN in the CyclinD1-Luciferase reporter assay and the TBL3 cell proliferation assay (**Figures 4E** **and** **5E**). Both Buparvaquone and Juglone inhibited *WT* TaPIN effects, but the mutant TaPIN A53P was resistant to Buparvaquone inhibition.

The emergence of Buparvaquone-resistant *Theileria* strains, with mutations in the TaPIN1 gene, suggests that alternative anti-PIN1 compounds, such as Juglone, might be effective compounds to treat drug-resistant Theileriosis.

### 2.5. TaPIN mechanism of action in Theileria

To investigate the mechanisms by which TaPIN affects host signaling pathways, the inventors searched for relevant substrates targeted by TaPIN. The FBW7 (F-box and WD repeat domain containing 7) protein is a substrate of hPIN1 and has anti-tumor function in human cancers. The FBW7 (F-box and WD repeat domain containing 7) protein has anti-tumor function in human cancers, exerting its function by promoting the degradation of various oncoproteins required for cellular proliferation, such as c-Jun ***(*Nateri** et al., 2004; **Wei** et al., 2005). As c-Jun is induced (and essential) during *Theileria*-induced transformation (**Chaussepied** et al., 1998) and as FBW7 is a substrate of hPIN1 (**Min** et al., 2012), it was examined whether TaPIN1 targets the bovine FBW7 protein.

Bovine FBW7 levels were shown to be reduced in parasitized TBL3 cells compared with parental uninfected BL3 cells, correlating with elevated c-Jun levels (Figure 6A). Treatment with Buparvaquone, Juglone or PiB induced FBW7 protein and reduced c-Jun levels while it had no effect on the mRNA expression (**Figure 6A**). The results of **Figure 6B** and C showed that TaPIN protein interacted with endogenous FBW7 in TBL3 cells or in mouse fibroblasts.

Furthermore, it was demonstrated that TaPIN1 could rescue c-Jun protein levels in PIN1-/- knockout MEFs (mouse embryonic fibroblasts) by reducing FBW7 levels.

The ubiquitination of endogenous c-Jun protein was elevated by Buparvaquone or Juglone treatment of TBL3 cells. Conversely, pharmacological PIN1 inhibition decreased FBW7 (auto)-ubiquitination in these cells.

The present inventors then demonstrated that the c-Jun protein degradation was FBW7-dependent, as the c-Jun ubiquitination induced by Buparvaquone or Juglone in TBL3 cells was abolished by siRNA targeting endogenous FBW7. Besides, the effect of Buparvaquone, but not of Juglone, on c-Jun ubiquitination was rescued by overexpressing the Buparvaquone-resistant TaPIN A53P mutant. Finally, the inventors demonstrated the functional importance of FBW7 degradation by *Theileria,* as transfection with FBW7 isoforms blocked soft-agar proliferation of parasitized TBL3 cells (**Figure 6D**).

To identify additional novel PIN1 targets, a proteomic screen for PIN1 interacting partners was performed in the cytoplasmic or nuclear compartments of mouse fibroblasts. This experiment identified Pyruvate Kinase Isoform M2 (PKM2) as a potential PIN1 substrate (**Yang** et al., 2012). As PKM2 is known to be specifically associated with tumorigenesis (**Christofk** et al., 2008) and *Theileria* transformation is known to cause rewiring of the host cell metabolism (**Medjkane** et al., 2013), the inventors investigated whether TaPIN functions might involve PKM2 pathways. This experiment showed that TaPIN also interacts with endogenous PKM2 protein in mouse fibroblasts or in parasitized TBL3 cells (**Figures 6E** **and data not shown**). PKM2 was recently characterized as a cofactor for HIF1alpha (Hypoxia Induced Factor 1 alpha), a transcription factor critical for the Warburg effect and for the transcription of glycolytic enzymes in cancer cells (Luo et al., 2011). It was found here that PKM2 protein levels were reduced upon Buparvaquone/Juglone treatment of TBL3 cells, which correlated with reduced expression of HIF1a and of the glycolytic enzymes HK2 and PDK1 (**Figure 6F**). The effects of Buparvaquone/Juglone were mimicked by silencing bovine PKM2 using siRNA in Theileria-infected cells (**Figure 6G**). Furthermore, TaPIN1 transfection rescued the stabilization of endogenous PKM2 in PIN1-/- knockout MEFs. The functional importance of the TaPIN-PKM2 interaction was supported by the observation that Buparvaquone/Juglone/PiB treatment led to reduced expression of the transcripts encoding glycolytic enzymes, as well as reduced HIF1alpha transcriptional activity using a HRE-Luciferase reporter. The effects of Buparvaquone/Juglone were mimicked by silencing bovine PKM2 using siRNA and rescued by stabilizing HIF1a with DMOG in Theileria infected cells. Overexpression of the TaPIN A53P mutant reversed the Buparvaquone-induced decrease in glycolytic enzyme expression. The functional relevance was further shown by the reduced glucose uptake and consumption upon TaPIN inhibition in TBL3 cells. Based on the above findings, the following model for *Theileria-*induced transformation is herein proposed by the inventors (**Figure 6H**): the parasite encoded TaPIN PPlase is secreted into the host cells, where it interacts with FBW7 leading to its auto-degradation and subsequent c-Jun stabilization. c-Jun was reported as being critical for *Theileria* transformation and host cell proliferation, but it was unknown how the parasite initiated this effect. Subsequent activation of a feedback loop involving c-Jun control of the miR-155 oncomiR, may create a subsequent epigenetic switch which then maintains transformation and proliferation (**Marsolier** et al., 2013). The TaPIN protein also interacts with, and stabilizes, bovine PKM2, leading to the activation of HIF1alpha target genes which link the parasite to host metabolic rewiring and to a Warburg effect that is also critical for parasite-induced transformation. Treatment with anti-PIN1 compounds targets these pathways leading to decreased c-Jun and PKM2/HIF1alpha signaling and parasite death, reversing the transformed phenotypes.

### 3. CONCLUSION

The present discovery highlights TPIN as a key element of the host-parasite dynamic that contributes to tumor-like phenotypes in Theileriosis-infected animals. The emergence of Buparvaquone-resistant *Theileria* strains, with mutations in the TaPIN gene, suggests that anti-PIN1 compounds might be effective clinical reagents to treat drug-resistant Theileriosis. Finally, the evolution of a PIN1 homologue with an acquired signal peptide only in *Theileria,* provides fascinating insight into how this Apicomplexa species has hijacked oncogenic pathways to drive host cell transformation.

### EXAMPLE 2 - Buparvaquone for treating cancer

Based on the finding that buparvaquone is a PIN1 inhibitor, the inventors tested the ability of buparvaquone to reduce the viability of cancer cells, using hepatocellular carcinoma (HCC) cell line HepG2 in a cell viability XTT assay.

Results are shown in **Figure 9A** and clearly show that buparvaquone inhibits growth of HCC cell line HepG2.

The inventors further tested the ability of buparvaquone to inhibit the soft agar colony-forming potential of HepG2 cells, and found that buparvaquone is indeed able to significantly decrease the ability of HepG2 cells to form soft agar colonies (**Figure 9B**).

### REFERENCES

Ayala G., Wang D.G., Wulf G., Frolov A., Li R., Sowadski J., Wheeler T.M., Lu K.P., and Bao L. (2003). Cancer Res.; 63:6244-6251.
Bao L., Kimzey A., Sauter G., Sowadski J.M., Lu K.P., and Wang D.G. (2004). Am J Pathol.; 164(5):1727-37.
Bendtsen J.D., Nielsen H., von Heijne G., and Brunak S. (2004). J. Mol. Biol.; 340(4): 783-95.
Chaussepied M., and Langsley G. (1996). Res. Immunol.; 147: 127-138.
Chaussepied M., Lallemand D., Moreau M.F., Adamson R., Hall R., and Langsley G. (1998). Mol. Biochem. Parasitol.; 94: 215-226.
Christofk H.R., Vander Heiden M.G., Harris M.H., Ramanathan A., Gerszten R.E., Wei R., Fleming M.D., Schreiber S.L., Cantley L.C. (2008).The M2 splice isoform of pyruvate kinase is important for cancer metabolism and tumour growth. Nature; 452: 230-233.
Dessauge F., Hilaly S., Baumgartner M., Blumen B., Werling D., and Langsley G. (2005). Oncogene; 24:1075-1083.
Dong L., Marakovits J., Hou X., Guo C., Greasley S., Dagostino E., Ferre R., Johnson M.C., Kraynov E., Thomson J., Pathak V., and Murray B.W. (2010). Bioorg Med Chem Lett.; 20(7):2210-4.
Duncan K.E., Dempsey B.R., Killip L.E., Adams J., Bailey M.L., Lajoie G.A., Litchfield D.W., Brandl C.J., Shaw G.S., and Shilton B.H. (2011). J Med Chem.; 54(11):3854-65.
Emanuelsson 0., Brunak S., von Heijne G., and Nielsen H. (2007). Nat. Protoc. 2(4): 953-71.
Gianni' M., Boldetti A., Guarnaccia V., Rambaldi A., Parrella E., Raska I. Jr, Rochette-Egly C., Del Sal G., Rustighi A., Terao M., and Garattini E. (2009). Cancer Res.; 69(3):1016-26.
Guo C., Hou X., Dong L., Dagostino E., Greasley S., Ferre R., Marakovits J., Johnson M.C., Matthews D., Mroczkowski B., Parge H., Vanarsdale T., Popoff I., Piraino J., Margosiak S., Thomson J., Los G., Murray B.W. (2009). Bioorg Med Chem Lett.; 19(19):5613-6.
Grove L.E., Hall D.R., Beglov D., Vajda S., Kozakov D. (2013). Bioinformatics ;29(9):1218-9. Hennig L., Christner C., Kipping M., Schelbert B., Rücknagel K.P., Grabley S., Küllertz G., and Fischer G. (1998). Biochemistry (Mosc.); 37:5953-5960 (1998).
Huse W.D., Sastry L., Iverson S.A., Kang A.S., Alting-Mees M., Burton D.R., Benkovic S.J., and Lerner R.A. (1989). Science; 246:1275-1281.
Ji Q., Zhang L., Jones M.B., Sun F., Deng X., Liang H., Cho H., Brugarolas P., Gao Y.N., Peterson S.N., Lan L., Bae T., and He C. (2013). Proc Natl Acad Sci USA;110(13):5010-5. Kim M.R., Choi H.S., Yang J.W., Park B.C., Kim J.A., and Kang K.W. (2009). Mol Cancer Ther ; 8(8):2163-71.
Kofron J.L., Kuzmic P., Kishore V., Colonbonilla E., and Rich D.H (1991). Biochemistry; 1(30):6127-6134.
Köhler G. and Milstein C. (1975). Nature ;256 (5517):495-7.
Kozbor D., Roder J.C. (1983). Immunology Today vol. 4: p. 72-79.
Lagorce D., Pencheva T., Villoutreix B.O., and Miteva M.A. (2009). BMC Chem Biol.; 13; 9:6.
Lambert C., Léonard N., De Bolle X., and Depiereux E. (2002). Bioinformatics; 18(9):1250-6.
Liu C., Jin J., Chen L., Zhou J., Chen X., Fu D., Song H., and Xu B. (2012). Bioorg Med Chem.; 20(9):2992-9.
Lu P.J., Zhou X.Z., Shen M., and Lu K.P. (1999). Science; 28:1325-1328.
Luo W., Hu H., Chang R., Zhong J., Knabel M., O'Meally R., Cole .RN., Pandey A., and Semenza G.L. (2011). Cell; 145:732-744.
Miteva M.A., Tufféry P., and Villoutreix (2005). B.O.Nucleic Acids Res; vol. 33(Web Server issue):W372-5.
Marsolier J., Pineau S., Medjkane S., Perichon M., Yin Q., Flemington E., Weitzman M.D., Weitzman J.B. (2013). Plos Pathog.; 9(4): e1003222.
Medjkane S., Perichon M., Marsolier J., Dairou J., and Weitzman J.B. (2013). Oncogene doi:10.1038/onc.2013.134.
Mhadhbi M., Naouach A., Boumiza A., Chaabani M.F., BenAbderazzak S., and Darghouth M.A. (2010). Vet. Parasitol.; 169: 241-247.
Min S.H., Lau A.W., Lee T.H., Inuzuka H., Wei S., Huang P., Shaik S., Lee D.Y., Finn G., Balastik M., Chen C.H., Luo M., Tron A.E., Decaprio J.A., Zhou X.Z., Wei W., and Lu K.P. (2012). Negative Regulation of the Stability and Tumor Suppressor Function of Fbw7 by the Pin1 Prolyl Isomerase, Mol. Cell.; 46: 771-783.
Nateri A.S., Riera-Sans L., Costa C.D., and Behrens A. (2004). Science; 303:1374-1378.
Peloponese J.M. Jr, Yasunaga J., Kinjo T., Watashi K., and Jeang K.T. (2009). J Virol.; 83(7):3238-48.
Ranganathan R., Lu K.P., Hunter T., and Noel J.P. (1997). Cell; 89: 875-886.
Roder J.C., Cole S.P., and Kozbor D. (1986). Methods Enzymol.; 121:140-167.
Ryo A., Liou Y.C., Wulf G., Nakamura M., Lee S.W., and Lu K.P. (2002). Mol. Cell. Biol.; 22: 5281-5295.
Ryo A., Uemura H., Ishiguro H., Saitoh T., Yamaguchi A., Perrem K., Kubota Y., Lu K.P., and Aoki I. (2005). Clin Cancer Res.; 11 (20):7523-31.
Sauton N., Lagorce D., Villoutreix B.O., and Miteva M.A. (2008). BMC Bioinformatics; 9:184.
Sharifiyazdi H, Namazi F., Oryan A., Shahriari R., and Razavi M. (2012). Vet Parasitol.; 187(3-4):431-5.
Spitzer R., and Jain A.N. (2012). J Comput Aided Mol Des; 26(6):687-99.
Tatara Y., Lin Y.C., Bamba Y., Mori T., and Uchida T. (2009). Biochem Biophys Res Commun.; 384(3):394-8.
Thomsen R., and Christensen M.H. (2006). J. Med. Chem.; 49(11):3315-3321.
Uchida T., Takamiya M., Takahashi M., Miyashita H., Ikeda H., Terada T., Matsuo Y., Shirouzu M., Yokoyama S., Fujimori F., and Hunter T. (2003). Chem. Biol.; 10, 15-24.
Uchida T.; 2012/02/25. Bangladesh JSPS Alumni Association Symposium on "Science for Society", Dhaka. A plenary talk: Peptidyl Prolyl cis/trans Isomerase Pin1; a target of drug for Cancer, Infectious disease, Alzheimer's disease, Diabetes and so on.
Wang X.J., Xu B., Mullins A.B., Neiler F.K., and Etzkorn F.A. (2004). J Am Chem Soc.; 126(47):15533-42.
Wei W., Jin J., Schlisio S, Harper J.W., and Kaelin W.G. (2005). Cancer Cell; 8: 25- 33.
Wildemann D., Erdmann F., Alvarez B.H., Stoller G., Zhou X.Z., Fanghänel J., Schutkowski M., Lu K.P., and Fischer G.(2006). J Med Chem.; 49(7):2147-50. Erratum in: J Med Chem. (2006); 49(11):3430.
Wulf G.M., Ryo A., Wulf G.G., Lee S.W., Niu T., Petkova V., and Lu K.P. (2001). Embo J.; 20: 3459-3472.
Yaffe M.B., Schutkowski M., Shen M., Zhou X.Z., Stukenberg P.T., Rahfeld J.U., Xu J., Kuang J., Kirschner M.W., Fischer G., Cantley L.C., and Lu K.P. (1997). Science; 278: 1957-1960.
Yang W., Zheng Y., Xia Y., Ji H., Chen X., Guo F., Lyssiotis C.A., Aldape K., Cantley L.C., and Lu Z. (2012). Nat. Cell Biol.; 14: 1295-1304.
Yoon H.E., Kim S.A., Choi H.S., Ahn M.Y., Yoon J.H., and Ahn S.G. (2011). Cancer Lett.; 316(1):97-104.

## Claims

1. Peptidyl-prolyl *cis*/*trans* isomerase 1 (PIN1) inhibitor for use in the prevention and/or treatment of lymphoproliferative Theileriosis in a subject in need thereof, with the proviso that said inhibitor is not Buparvaquone or Parvaquone.

2. Peptidyl-prolyl *cis*/*trans* isomerase 1 (PIN1) inhibitor for use according to claim 1, wherein said inhibitor being selected from the group consisting of:
• 5-Hydroxy-1,4-naphthlenedione;
• a PIN1 inhibitor of formula (I): 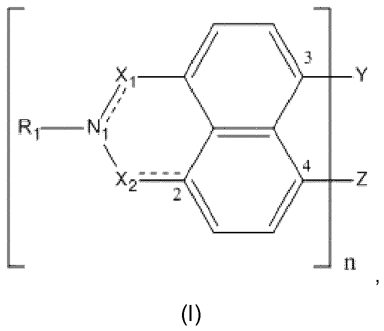 wherein
- R1 is -CH₃, -CH₂-COOEt, or -(CH₂)₆-NH-CO-CF₃,
- X₁ and X₂, independently from each other, is C=O and/or CH,
- the bond between N₁ and X₁ is a single or a double bond,
- the bond between C₂ and X₂ is a single or a double bond, and
- n is 1 or 2, with the proviso that
o when n is 2, Y and Z are chemical bonds, or
o when n is 1, Y and Z are H, or Y and Z form a cycle according to formula (A) 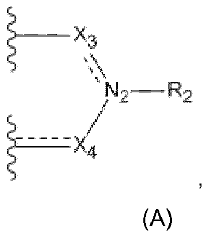
wherein
- X₃ is linked to C₃ of formula (I) and X₄ is linked to C₄ of formula (I),
- R2 is -CH₃, -CH₂-COOEt , -(CH₂)₆-NH-CO-CF₃, or -(CH₂)₅-COOH, and
- X₃ and X₄, independently from each other, is C=O and/or CH,
- the bond between N₂ and X₃ is a single or a double bond, and
- the bond between C₄ of formula (I) and X₄ is a single or a double bond;
• a PIN1 inhibitor of formula (II): 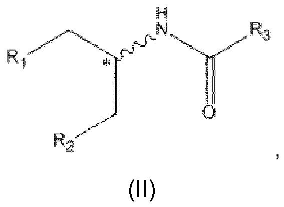 wherein
- R1 is a mono- or poly-substituted phenyl group, the substituent(s) of R1 being selected from a C₁-C₆ alkyl group or a halogen atom, said halogen atom being preferably F,
- R2 is a phosphate group,
- R3 is a C₅-C₁₅ aryl or heteroaryl group comprising at least two cycles, preferably a naphthyl or a benzothiophenyl group, and
- (*) is R or S, preferably R;
• a PIN1 inhibitor of formula (III): 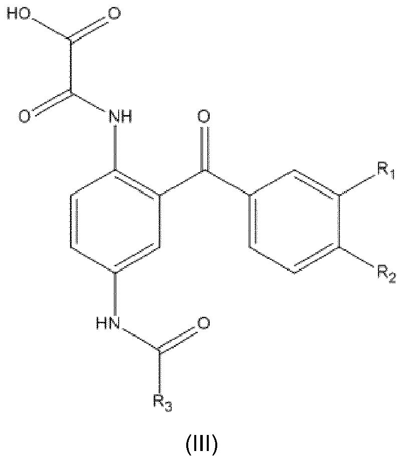 wherein
- R1 is H, a C₁-C₆ alkyloxy group, or a halogen atom, said alkyloxy group being preferably a methoxy group, and/or said halogen atom being preferably F,
- R2 is H, or a C₁-C₆ alkyloxy group, said alkyloxy group being preferably a methoxy group, and
- R3 is a C₅-C₁₅ aryl or heteroaryl group comprising at least two cycles, preferably selected from an indolyl group, a benzofuranyl group, and a benzothiophenyl group optionally substituted by a halogen atom or a nitro group;
• a PIN1 inhibitor of formula (IV): 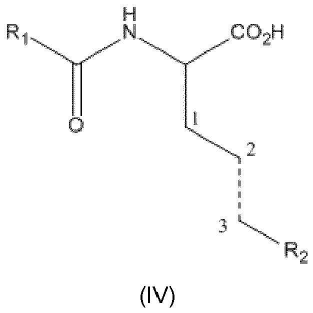 wherein
- the bond formed between the carbon atoms 2 and 3 is single, double or triple, and when said bond is double, said bond is preferably in the E configuration,
- R1 is a C₅-C₁₅ aryl or heteroaryl group comprising at least two cycles, preferably a naphthyl or a benzothiophenyl group, and
- R2 is a cyclohexenyl group, a C₄-C₈ heteroaryl group, preferably a thienyl, or a C₄_ C₈ aryl group, preferably a phenyl optionally substituted by a halogen atom or OH;
• 1-Piperidinecarbodithioic acid, anhydrosulfide;
• (3Z)-3-[(3Z)-3-hydroxyiminoindolin-2-ylidene]-5-nitro-indolin-2-one;
• the peptide CRYPEVEIC, wherein the cysteine residues of said peptide are cyclized by a disulfide bond (SEQ ID NO:66);
• the peptide Ac-Lys(N-biotinoyl)-Ala-Ala-Bth-D-Thr(PO₃H₂)-Pip-Nal-Gln-NH₂ (SEQ ID NO:67);
• the peptide Ac-Phe-D-Thr(PO₃H₂)-Pip-Nal-Gln-NH₂ (SEQ ID NO:68);
• the peptide Ac-Phe-Phe-pSer- Ψ [(Z)CHdC]-Pro-Arg-NH₂ (SEQ ID NO:69);
and
a pharmaceutically acceptable salt, and/or solvate thereof.

3. Peptidyl-prolyl *cis*/*trans* isomerase 1 (PIN1) inhibitor for use according to claim 2, wherein said PIN1 inhibitor is selected from the group consisting of:
• 5-Hydroxy-1,4-naphthlenedione;
• diethyl-1 ,3,6,8-tetrahydro-1 ,3,6,8tetraoxobenzo[lmn] phenanthroline-2,7-diacetate;
and
a pharmaceutically acceptable salt, and/or solvate thereof.

4. Peptidyl-prolyl *cis*/*trans* isomerase 1 (PIN1) inhibitor for use according to any one of claims 1 to 3, wherein said lymphoproliferative Theileriosis is mediated by a transforming *Theileria* parasite selected from the group consisting of *Theileria annulata*, *Theileria parva* and *Theileria hirci* , more preferably *Theileria annulata* and *Theileria parva.*

5. Peptidyl-prolyl *cis*/*trans* isomerase 1 (PIN1) inhibitor for use according to any one of claims 1 to 4, wherein said lymphoproliferative Theileriosis is mediated by a Buparvaquone-resistant transforming *Theileria* parasite.

6. Combination of at least one peptidyl-prolyl *cis*/*trans* isomerase 1 (PIN1) inhibitor as defined in any one of claims 1 to 3 and of at least one other theilericidal drug for simultaneous, separate or sequential use in the prevention and/or treatment of lymphoproliferative Theileriosis in a subject in need thereof.

7. Combination according to claim 6, wherein said theilericidal drug is selected from the group consisting of Buparvaquone, Parvaquone, Halofuginone Lactate, and tetracyclines such as Medamycin or Terramycin.

8. Pharmaceutical composition comprising at least one peptidyl-prolyl *cis*/*trans* isomerase 1 (PIN1) inhibitor as defined in any one of claims 1 to 3, and at least one pharmaceutically acceptable excipient.

9. Pharmaceutical composition according to claim 8, further comprising at least one theilericidal drug, said drug being preferably selected from the group consisting of Buparvaquone, Parvaquone, Halofuginone Lactate, and tetracyclines such as Medamycin or Terramycin.

10. An *in vitro* screening method for identifying a PIN1 inhibitor or combination of PIN1 inhibitors useful for preventing and/or treating lymphoproliferative Theileriosis, comprising the steps of:
a) contacting a peptidyl-prolyl *cis*/*trans* isomerase 1 (PIN1) protein of a transforming *Theileria* parasite, with a candidate drug or a combination of candidate drugs;
b) measuring the activity of said protein;
c) calculating a ratio R between the activity of said protein at step b) and that measured in the absence of said drug or combination of drugs;
wherein a ratio R inferior to 1 is indicative that said drug or combination of drugs is a PIN1 inhibitor or combination of PIN1 inhibitors useful for preventing and/or treating lymphoproliferative Theileriosis.

11. An *in vitro* method for diagnosing a lymphoproliferative Theileriosis in a subject, comprising the step of:
a) detecting in a biological sample of said subject the peptidyl-prolyl *cis*/*trans* isomerase 1 (PIN1) of a transforming *Theileria* parasite,
wherein the detection of said PIN1 in said sample is indicative that said subject is suffering from lymphoproliferative *Theileriosis.*

12. The method of claim 11, further comprising the step of detecting from said biological sample at least one mutation associated with Buparvaquone resistance in said peptidyl-prolyl *cis*/*trans* isomerase 1 of a transforming *Theileria* parasite.

13. An *in vitro* method for determining a PIN1 inhibitor-responding or non-responding phenotype in a subject suffering from lymphoproliferative Theileriosis comprising the steps of:
a) detecting in a biological sample of said subject the peptidyl-prolyl cis/trans isomerase 1 (PIN1) of a transforming *Theileria* parasite according to the method of claim 11 or 12; and
b) determining the PIN1 inhibitor-responding or non-responding phenotype in said subject.

14. A method for designing or adapting a treatment regimen for a subject suffering from lymphoproliferative Theileriosis, comprising the steps of:
a) determining from a biological sample of said subject a PIN1 inhibitor-responding or non-responding phenotype according to the method of claim 13; and
b) designing or adapting a treatment regimen based upon said responding or non-responding phenotype.

15. A kit for use in a method according to any one of claims 10 to 14, comprising:
a) at least one reagent capable of specifically detecting PIN1 of a transforming *Theileria* parasite; and
b) instructions for using said kit in said method.
